# EUROPEAN PATENT SPECIFICATION

(11) **EP 3 600 372 B1**
(45) Date of publication and mention of the grant of the patent: **13.05.2026**
(21) Application number: 18774902.3
(22) Date of filing: 07.03.2018
(51) Int. Cl.: A61K 38/00, A61K 38/19, C07K 14/52, G01N 33/68

(54) **SYNTHEKINE COMPOSITIONS AND METHODS OF USE**
SYNTHEKINZUSAMMENSETZUNGEN UND VERFAHREN ZUR VERWENDUNG
COMPOSITIONS DE SYNTHÉKINE ET PROCÉDÉS D'UTILISATION

(30) Priority: 31.03.2017 US 201762479993 P
(43) Date of publication of application: 05.02.2020
(73) Proprietor: The Board of Trustees of the Leland Stanford Junior University, Stanford, CA 94305-2038 (US)
(72) Inventor: GONZALEZ, Ignacio Moraga, Palo Alto, California 94306 (US); GARCIA, Kenan Christopher, Menlo Park, California 94025 (US)
(74) Representative: Mewburn Ellis LLP
(86) International application number: PCT/US2018/021301
(87) International publication number: WO 2018/182935

(56) References cited:
- WO-A1-2015/044386
- WO-A2-2006/036878
- US-A1- 2016 244 499
- V. KERMER ET AL: "Combining antibody-directed presentation of IL-15 and 4-1BBL in a trifunctional fusion protein for cancer immunotherapy.", MOLECULAR CANCER THERAPEUTICS, vol. 13, no. 1, 6 November 2013 (2013-11-06), US, pages 112 - 121, XP055200723, ISSN: 1535-7163, DOI: 10.1158/1535-7163.MCT-13-0282
- STEPHEN D GILLIES ET AL: "Bi-functional cytokine fusion proteins for gene therapy and antibody-targeted treatment of cancer", vol. 51, no. 8, 1 October 2002 (2002-10-01), pages 449 - 460, XP002685580, ISSN: 0340-7004, Retrieved from the Internet <URL:http://download.springer.com/static/pdf/274/art%253A10.1007%252Fs00262-002-0302-6.pdf?auth66=1352214660_7b6ef750a190d34f6bb16311f087c884&ext=.pdf> [retrieved on 20020712], DOI: 10.1007/S00262-002-0302-6
- ELIZABETH ORTIZ-S�NCHEZ ET AL: "Antibody-cytokine fusion proteins: applications in cancer therapy", HHS PUBLIC ACCESS AUTHOR MANUSCRIPT, vol. 8, no. 5, 13 April 2008 (2008-04-13), pages 1 - 42, XP055535112, DOI: 10.1517/14712598.8.5.609
- MARCOS R. DIFALCO ET AL: "The improved survival of hematopoietic cells cultured with a fusion protein of insulin-like growth factor II (IGF-II) and interleukin 3 (IL-3) is associated with increases in Bcl-x L and phosphatidylinositol-3 kinase activity", JOURNAL OF LEUKOCYTE BIOLOGY, vol. 73, no. 2, 1 February 2003 (2003-02-01), GB, pages 297 - 305, XP055751350, ISSN: 0741-5400, DOI: 10.1189/jlb.0802396
- E. A. ROSSI ET AL: "A Bispecific Antibody-IFN 2b Immunocytokine Targeting CD20 and HLA-DR Is Highly Toxic to Human Lymphoma and Multiple Myeloma Cells", CANCER RESEARCH, vol. 70, no. 19, 28 September 2010 (2010-09-28), US, pages 7600 - 7609, XP055685155, ISSN: 0008-5472, DOI: 10.1158/0008-5472.CAN-10-2126
- SCHLEHUBER S ET AL: "DUOCALINS: ENGINEERED LIGAND-BINDING PROTEINS WITH DUAL SPECIFICITY DERIVED FROM THE LIPOCALIN FOLD", BIOLOGICAL CHEMISTRY, WALTER DE GRUYTER GMBH & CO, BERLIN, DE, vol. 382, no. 9, 1 September 2000 (2000-09-01), pages 1335 - 1342, XP001078830, ISSN: 1431-6730, DOI: 10.1515/BC.2001.166
- CLAIRE GORBY ET AL: "Mapping Determinants of Cytokine Signaling via Protein Engineering", FRONTIERS IN IMMUNOLOGY, vol. 9, 1 January 2018 (2018-01-01), pages 2143, XP055748524, DOI: 10.3389/fimmu.2018.02143
- ROLAND KONTERMANN: "Dual targeting strategies with bispecific antibodies", MABS, vol. 4, no. 2, 1 March 2012 (2012-03-01), US, pages 182 - 197, XP055566203, ISSN: 1942-0862, DOI: 10.4161/mabs.4.2.19000
- BRAD J STISH ET AL: "A Bispecific Recombinant Cytotoxin (DTEGF13) Targeting Human Interleukin-13 and Epidermal Growth Factor Receptors in a Mouse Xenograft Model of Prostate Cancer", CLINICAL CANCER RESEARCH, AMERICAN ASSOCIATION FOR CANCER RESEARCH, US, vol. 13, no. 21, 1 November 2007 (2007-11-01), pages 6486 - 6493, XP008138313, ISSN: 1078-0432, DOI: 10.1158/1078-0432.CCR-07-0938
- LIN SU ET AL: "Cloning and Expression of Human Stem Cell Factor Fused with Thrombopoietin Mimetic Peptide in Escherichia coli", BIOTECHNOLOGY LETTERS, SPRINGER NETHERLANDS, DORDRECHT, vol. 28, no. 12, 31 May 2006 (2006-05-31), pages 857 - 862, XP019391505, ISSN: 1573-6776, DOI: 10.1007/S10529-005-5530-3
- MORAGA ET AL.: "Synthekines are surrogate cytokine and growth factor agonists that compel signaling through non- natural receptor dimers", ELIFE, vol. 6, 12 May 2017 (2017-05-12), pages 22882, XP055542997
- MORAGA ET AL.: "Tuning cytokine receptor signaling by re-orienting dimer geometry with surrogate ligands", CELL, vol. 160, 26 February 2015 (2015-02-26), pages 1196 - 1208, XP029203787
- NG ET AL.: "Concise review: engineering the fusion of cytokines for the modulation of immune cellular responses in cancer and autoimmune disorders", STEM CELLS TRANSL MED, vol. 4, 12 November 2014 (2014-11-12), pages 66 - 73, XP055542998
- SPANGLER ET AL.: "Insights into cytokine-receptor interactions from cytokine engineering", ANNU REV IMMUNO L, vol. 33, 10 December 2014 (2014-12-10), pages 139 - 167, XP055294970

## Description

### Cross Reference

This application claims benefit of U.S. Provisional Patent Application No. 62/479,993, filed March 31, 2017.

### BACKGROUND

The manipulation of cells, particularly immune cells, to differentiate, develop specialized functions and expand in numbers is of great clinical interest. Many protein factors that affect these activities are known in the art, including in particular cytokines and chemokines. However, these signaling molecules also have pleiotropic effects on cells not targeted for manipulation, and thus methods of selectively activating signaling in a targeted cell population are desirable.

Cytokines, chemokines, growth factor agonists and the like activate JAK/STAT; RTK linked; or death domain (TNF super family) receptors by multimerization, e.g. generating homo or hetero-dimers, or higher order oligomers to elicit signaling through intracellular trans-phosphorylation. The identity of the specific receptor chains within a multimer (e.g. dimer or trimer) determines the signaling and functional response. In the case of cytokines, they act as bi-specific ligands to specify which receptors are included in the dimers by forming specific contacts with each of the two receptor extracellular domains, thus acting to bridge or cross-link the dimeric signaling complex. Cytokine receptor dimerization leads to the activation of an intracellular JAK/STAT signaling pathway, comprised of four Janus Kinases (JAK1-3, TYK2) and seven signal transducer and activator of transcription (STAT1- 6) proteins.

While the ligands are specific for the extracellular domains of their receptors, the JAK/TYK/STAT signaling modules are found in many combinations in endogenous receptor signaling complexes, and thus are capable of extensive cross-talk. Ligands for RTK receptors (such as EGF, VEGF, etc.) also compel signaling through receptor dimerization, although the molecular mechanisms can be quite distinct from cytokines. In both cases: JAK/STAT cytokines and RTK ligands, their role is to induce a positioning of their specific receptor subunits into dimers such that the intracellular kinases domains are in an orientation and proximity to enable trans-phosphorylation of both the kinases and the receptor intracellular domains. The sequence requirements (i.e. substrate specificity) of these tyrosine kinases can be rather degenerate, raising the possibility that these enzymes can be redirected by alternative receptor dimerizing ligands to phosphorylate receptor substrates other than those they are normally presented with in nature.

Given that the ligands determine the composition of the receptor dimers, and the intracellular kinase degeneracy of JAK/TYK and RTK enzymes, the number of cytokine and growth factor receptor dimer pairings that occur in nature only represents a small proportion of the total number of signaling-competent receptor pairings theoretically allowed by the system. For example, the human genome encodes for approximately forty different JAK/STAT cytokine receptors. In principle, approximately 1600 unique homo- and hetero-dimeric cytokine receptor pairs could be generated with the potential to signal through different JAK/TYK/STAT combinations. However, the human genome encodes for less than fifty different cytokine ligands, limiting the scope of cytokine receptor dimers to those that can be assembled by the natural ligands. A similar argument can be made for the RTK family of receptors and ligands. Furthermore, given that is has been shown that Death receptors are capable of signaling as dimers or trimers, this concept can also be extended to this family.

The ability to selectively activate signaling pathways of interest is of great interest. The present invention provides compositions and methods for this purpose.

### SUMMARY

The invention is defined by the appended claims.

In a first aspect, the invention provides a synthetic ligand comprising: at least a first and a second binding domain, wherein each binding domain of the synthetic ligand specifically binds to the extracellular domain of a cytokine receptor polypeptide subunit expressed on the surface of a cell, wherein: the first binding domain specifically binds to the extracellular domain of a first cytokine receptor subunit, wherein the first cytokine receptor subunit has an intracellular domain comprising at least one JAK/STAT binding sequence; and the second binding domain specifically binds to the extracellular domain of a second cytokine receptor subunit, wherein the second cytokine receptor subunit has an intracellular domain comprising at least one JAK/STAT binding sequence, wherein the first and second cytokine receptor subunits do not naturally multimerize in response to contacting the cell with a naturally occurring cytokine ligand; wherein contacting the cell expressing the first and second cytokine receptor subunits with the synthetic ligand results in multimerization of the cytokine receptor subunits and activation of a JAK/STAT-mediated signal in the cell; and wherein each binding domain is an antibody derived binding protein.

In a second aspect, the invention provides a synthetic ligand as disclosed herein for use in a method of treatment. In some embodiments, the invention provides a synthetic ligand disclosed herein for use in a method of treating an inflammatory disease, an autoimmune disease, or cancer.

The invention further provides a pharmaceutical formulation comprising a synthetic ligand as disclosed herein as well as an in vitro method for selective activation of a non-native combination of cytokine subunits in a cell.

Further disclosed herein are engineered synthetic signaling molecules, termed "synthekines". Synthekines are genetically engineered, bi-specific ligands of cell surface receptors, where the synthekine specifically binds at high affinity to the extracellular domain(s) of at least one and frequently two different cell surface receptor polypeptides. The cell surface receptors are characterized by activation of signaling upon multimerization. The generation of a receptor multimer by binding to a synthekine may result in intracellular trans-phosphorylation of the receptor. Synthekines include, without limitation, small organic molecules and polypeptides.

Synthekines may also be tri-specific ligands of cell surface receptors, or more, where the synthekine specifically binds at high affinity to the extracellular domain(s) of at least three different cell surface receptor polypeptides. The cell surface receptors are characterized by activation of signaling upon multimerization. The generation of a receptor multimer by binding to a synthekine may result in intracellular trans-phosphorylation of the receptor. Synthekines include, without limitation, small organic molecules and polypeptides.

The cell surface receptor polypeptide may be one or more of (i) a cytokine receptor that activates the JAK/STAT pathway in the cell; (ii) a receptor tyrosine kinase; or (iii) a TNFR superfamily member. Each of the multimeric receptor polypeptides may be naturally expressed in a targeted single cell. A target cell may be engineered to express the one or more of multimeric receptor polypeptides.

A synthekine may specifically bind to two or more different cytokine receptors that, when activated by multimerization, trans-phosphorylate and signal through JAK/STAT, and other pathways, including but not limited to ERK, AKT, and other signaling messengers. The cytokine receptors may be selected from, but not limited to βc, γc, IL-3Rα, βIL-3R, GM-CSFRα, IL-5Rα, CNTFα, CRLF1, LIFRα, gp130, IL-6Rα, IL-11Rα, OSMRβ, IL-2Rα, IL-2Rβ, IL-2Rγ, IL-4Rα, IL-7Rα, IL-9Rα, IL-13Rα, IL-15Rα, IL-21Rα, IFNAR2, IL-23R, EpoR, IL-12Rβ, IFNAR1, IFNAR2, G-CSFR, c-MPLR. A synthekine may bind to two of such receptors, and activates JAK/STAT signaling. A synthekine may bind to three of such receptors, and activates JAK/STAT signaling. Generally a synthekine activates pathways distinct from those of a native cytokine that activates the receptor(s).

A synthekine may bind to two or more different receptor tyrosine kinase proteins that are activated by trans-phosphorylation when the proteins are multimerized. The RTK receptors may be selected from but not limited to EGFR, ErbB2, ErbB3, ErbB4, InsR, IGF1R, InsRR, PDGFRα, PDGFRβ, CSF1R/Fms, cKit, Flt-3/Flk2, VEGFR1, VEGFR2, VEGFR3, FGFR1, FGFR2, FGFR3, FGFR4, PTK7/CCK4, TrkA, TrkB, TrkC, Ror1, Ror2, MuSK, Met, Ron, Axl, Mer, Tyro3, Tie1, Tie2, EphA1-8, EphA10, EphB1-4, EphB6, Ret, Ryk, DDR1, DDR2, Ros, LMR1, LMR2, LMR3, ALK, LTK, SuRTK106/STYK1, Activin-R, BMP-R, TGF-beta-R, Noggin-R. Specifically, a synthekine may bind to two of such receptors, and activates signaling. Generally a synthekine activates pathways distinct from those of a native ligand that activates the receptor(s).

A synthekine may bind to two or more different TNFR superfamily polypeptides that are activated when the proteins are multimerized. The receptors may be selected from TNFR1 (TNFRSF1A), TNFR2 (TNFRSF1B; TNFRSF2), 41-BB (TNFRSF9); AITR (TNFRSF18); BCMA (TNFRSF17), CD27 (TNFRSF7), CD30 (TNFRSF8), CD40 (TNFRSF5), Death Receptor 1 (TNFRSF10C), Death Receptor-3 (TNFRSF25), Death Receptor 4 (TNFRSF10A), Death Receptor 5 (TNFRSF10B), Death Receptor -6 (TNFRSF21), Decoy Receptor-3 (TNFRSF6B), Decoy Receptor 2 (TNFRSF10D), EDAR, Fas (TNFRSF6), HVEM (TNFRSF14), LTβ-R (TNFRSF3), OX40 (TNFRSF4), RANK (TNFRSF11A), TACI (TNFRSF13B), Troy (TNFRSF19), XEDAR (TNFRSF27), Osteoprotegerin (TNFRSF11B), TWEAK receptor (TNFRSF12A), BAFF Receptor (TNFRSF13C), NGF receptor (TNFRSF16). A synthekine may bind to two or more of such receptors, and activates signaling. Generally a synthekine activates pathways distinct from those of a native ligand that activates the receptor(s).

In some cases, a synthekine may bind to two or more receptors of mixed classes, e.g. a JAK/STAT receptor combined with a TNFRSF and/or RTK receptor; an RTK receptor combined with a TNFRSF receptor, and the like.

In some aspects of the disclosure, the synthekine is a polypeptide, which can comprise separate or contiguous binding domains or elements that bind to each of the receptor extracellular domain (ECD) polypeptides. A polypeptide synthekine may be a single chain, dimer, or higher order multimer. The binding domain/element for each receptor may be directly joined, or may be separated by a linker, e.g. a polypeptide linker, or a non-peptidic linker, *etc.* The synthekine may not activate a native receptor configuration. For example, a synthekine binding domain may bind one chain of a native receptor, but be disabled from binding the second chain of a native receptor. Such binding domains include, without limitation, dominant negative mutants of cytokines.

In some aspects of the disclosure, the receptor binding domains may be selected from any domain that binds the desired receptor extracellular domain at high affinity, e.g. a Kd of not more than about 1 x 10⁻⁷ M, not more than about 1 x 10⁻⁸ M, not more than about 1 x 10⁻⁹ M, or not more than about 1 x 10⁻¹⁰ M. Suitable binding domains include, without limitation, de novo designed binding proteins, antibody derived binding proteins, e.g. scFv, Fab, etc. and other portions of antibodies that specifically bind to one or more receptor ECD sequences; nanobody derived binding domains; knottin-based engineered scaffolds; norrin and engineered binding fragments derived therefrom, naturally occurring binding domains, and the like. Naturally occurring binding domains, such as cytokines, growth factors and the like are generally engineered to prevent activity from activation of the native receptor. A binding domain may be affinity selected to enhance binding to a desired ECD; and/or mutagenized to prevent binding to an undesired ECD.

A synthekine polypeptide can be fused, linked, or alternatively co-administered with an agent to enhance receptor activation. A synthekine can be fused, linked or alternatively co-administered with a cytokine, chemokine, or growth factor of interest.

The binding domains may be contiguous within one globular domain, or separated by a linker, e.g. a polypeptide linker, or a non-peptidic linker, *etc.* The length of the linker, and therefore the spacing between the binding domains can be used to modulate the signal strength, and can be selected depending on the desired use of the synthekine. The enforced distance between binding domains can vary, but in certain embodiments may be less than about 100 angstroms, less than about 90 angstroms, less than about 80 angstroms, less than about 70 angstroms, less than about 60 angstroms, or less than about 50 angstroms.

The linker may be a rigid linker, or a flexible linker. Where the linker is a peptide linker, it may be from about 1, 2, 3, 4, 5, 6, 7, 8, 9, 10, 11, 12, 13, 14, 15, 16, 17, 18, 19, 20 21, 22, 23, 24, 25, 26, 27, 28, 29, 30 or more amino acids in length, and is of sufficient length and amino acid composition to enforce the distance between binding domains. The linker may comprise or consist of one or more glycine and/or serine residues.

A synthekine can be multimerized, e.g. through an Fc domain, by concatenation, coiled coils, polypeptide zippers, biotin/avidin or streptavidin multimerization, and the like. The synthekine can also be joined to a moiety such as PEG, Fc, etc. as known in the art to enhance stability in vivo.

Compositions of interest include, without limitation, an effective dose of a synthekine in a pharmaceutically acceptable excipient. Compositions may comprise additional agents, e.g. adjuvants and the like. Synthekines may be produced synthetically; by various suitable recombinant methods, and the like, as known in the art.

In some aspects of the disclosure, a method is provided for activating, increasing or enhancing selected JAK/STAT, DD, and/or RTK signaling in a cell. In such methods, a cell expressing cognate receptor polypeptides for a synthekine of interest is contacted with a concentration of a synthekine that is effective to increase signaling, e.g. to increase signaling by 25%, 50%, 75%, 90%, 95%, or more, relative to the signaling in the absence of the synthekine. Such signaling activation may induce JAK/STAT or RTK signaling pathways and include, without limitation, modulation of immune responses, growth factor responses, induction of death domain responses, and the like. In some methods, the receptor-expressing cell is contacted *in vitro.* In some cases, the receptor-expressing cell is contacted *in vivo.*

A method is for treating or preventing a disease or disorder in a subject in need thereof is also disclosed herein, the method comprising providing to the subject an effective amount of a synthekine. The subject may have an immune disease or dysfunction.

### BRIEF DESCRIPTION OF THE DRAWINGS.

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures.

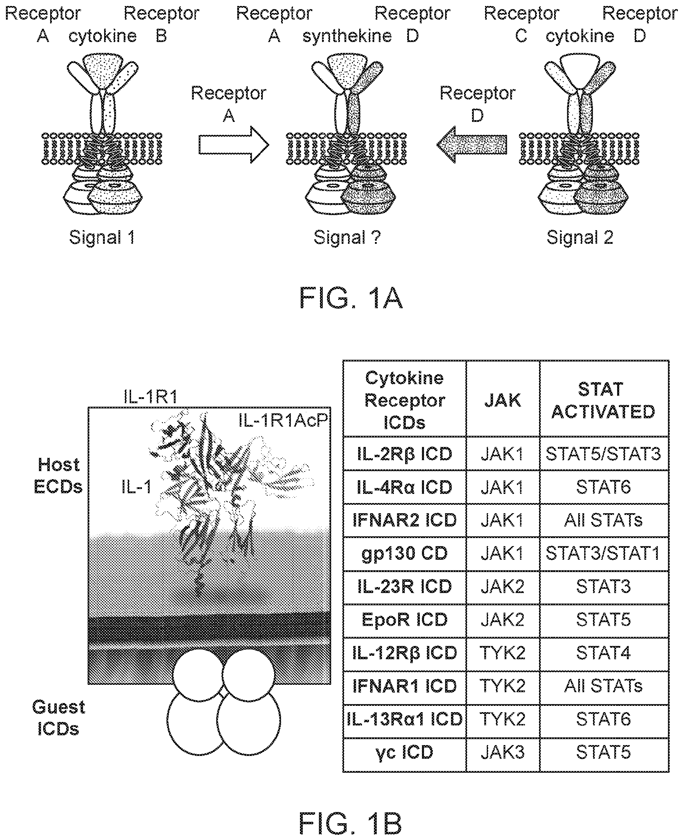

FIG. 1A-1B: Dimerization of non-natural receptor pairs by engineered synthekine ligands. FIG. 1A Schematic detailing the dimerization of new cytokine receptor pairs by synthekines. A hypothetical synthekine recruits receptors A and D to form a new ternary complex distinct from that formed by cytokines X and Y. FIG. 1B Schematic representation of the IL-1-mediated complexation of IL-1R1 and IL-1R1AcP chimeric receptors. The intracellular domains of the cytokine receptors indicated in the right table were grafted onto the IL-1R1 or IL-1R1AcP extracellular domains. JAKs and STATs activated by each receptor are indicated in the table.

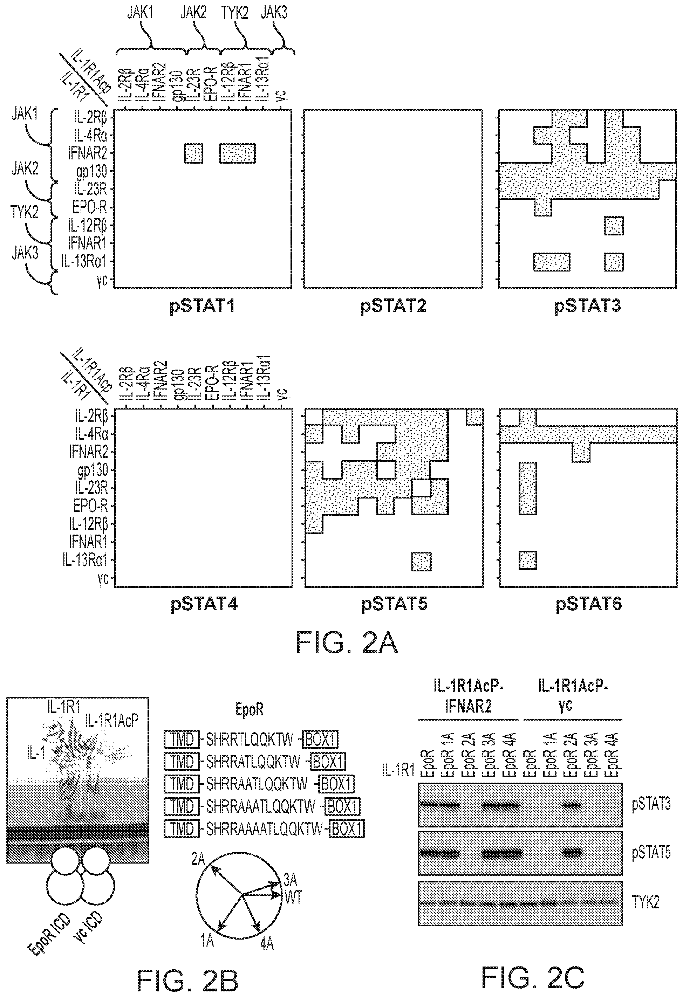

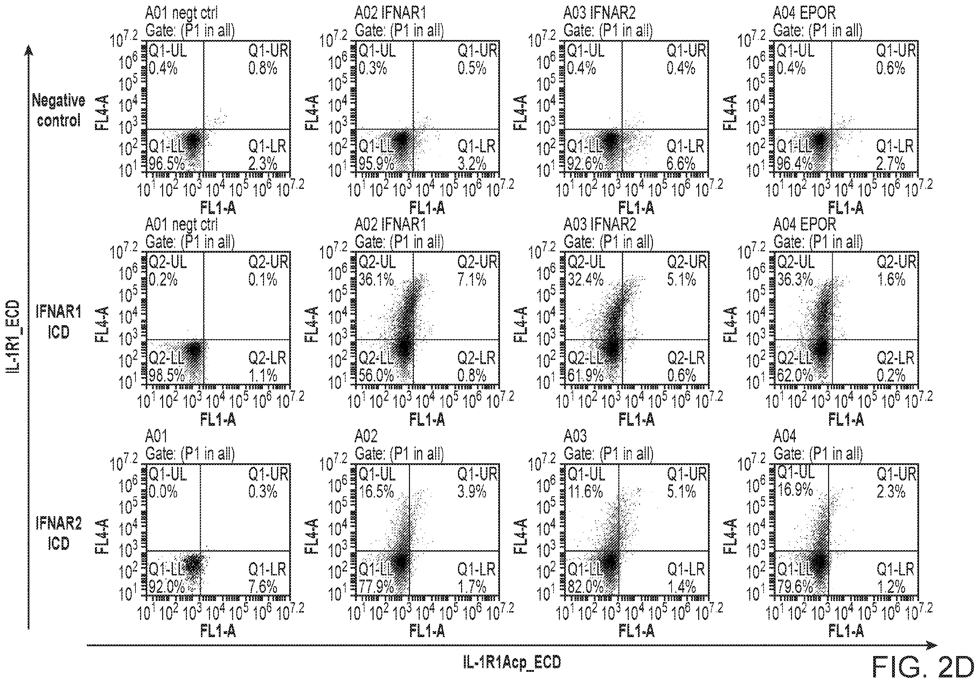

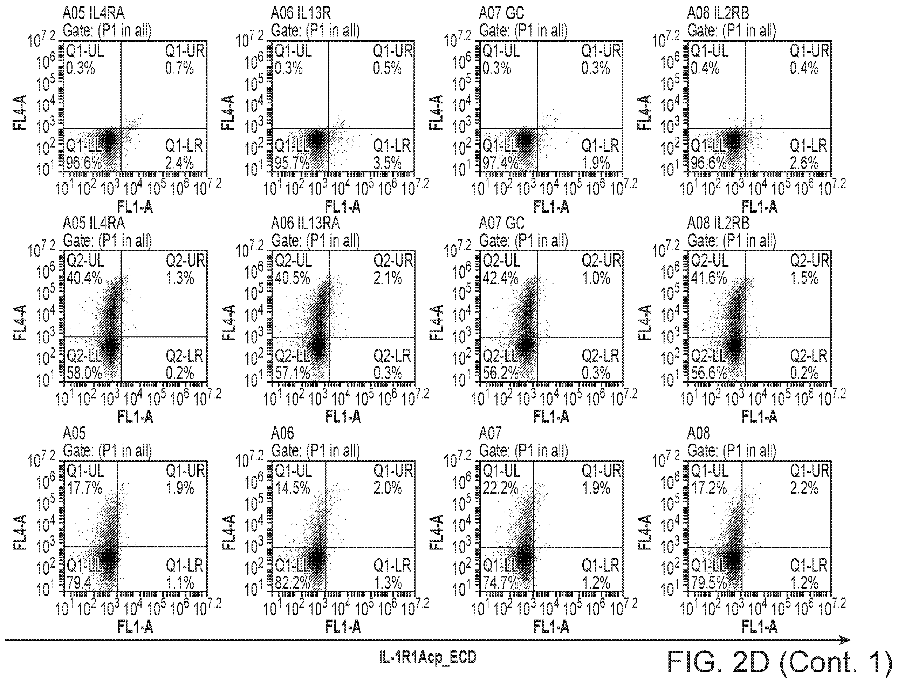

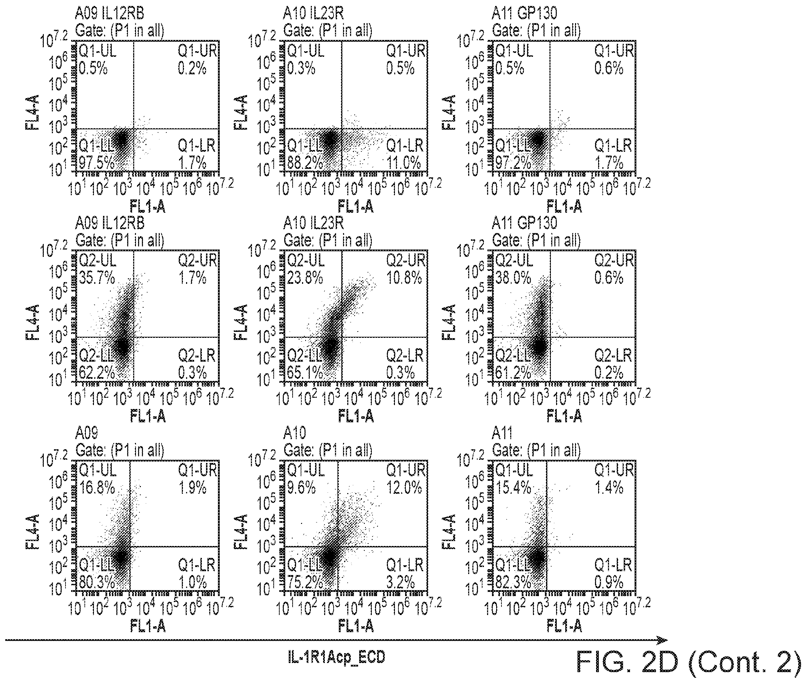

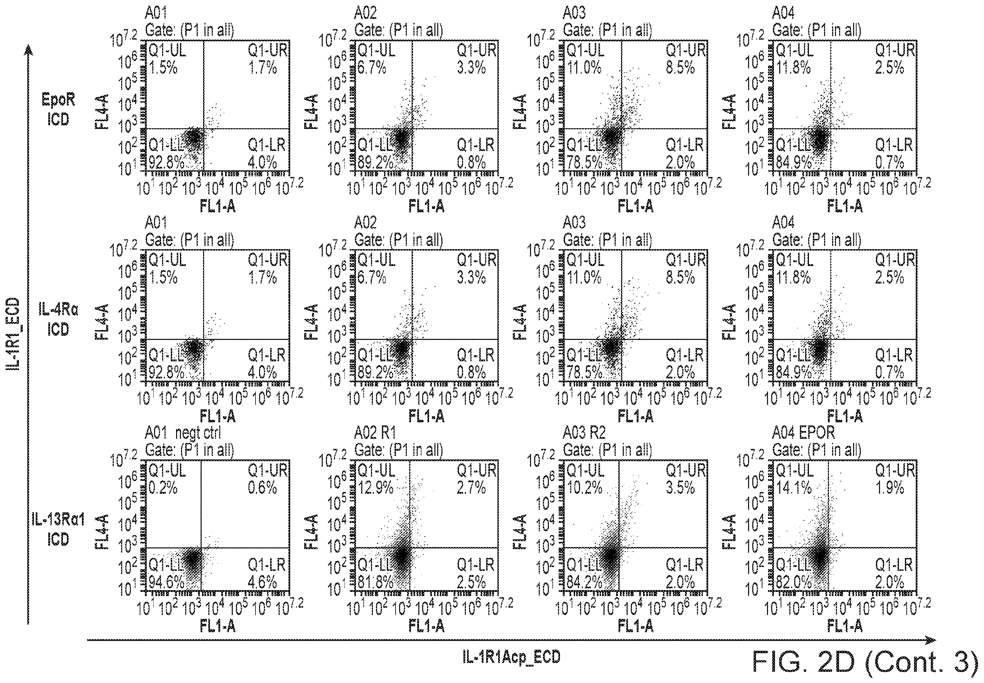

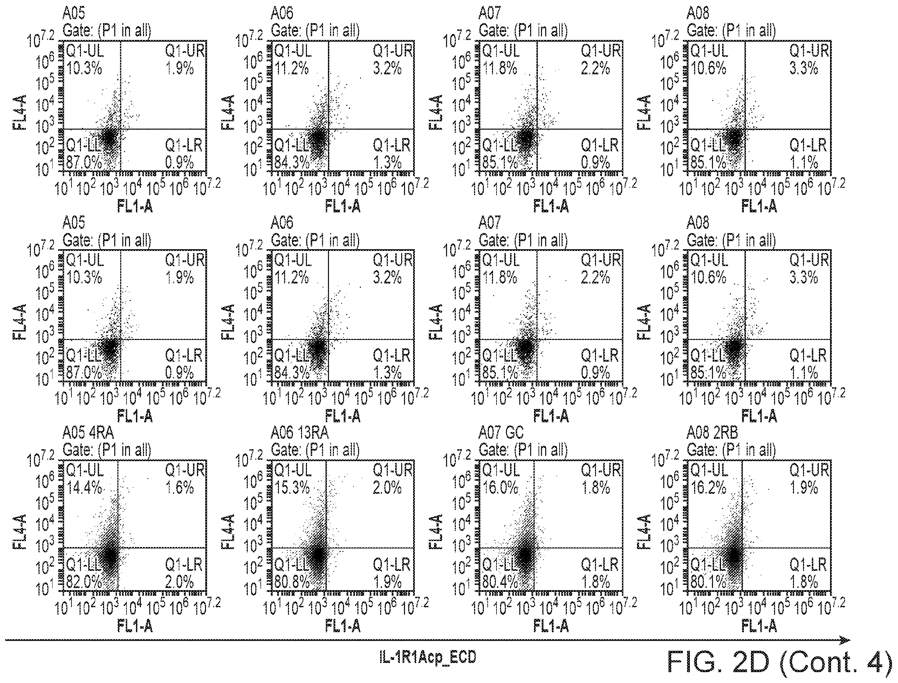

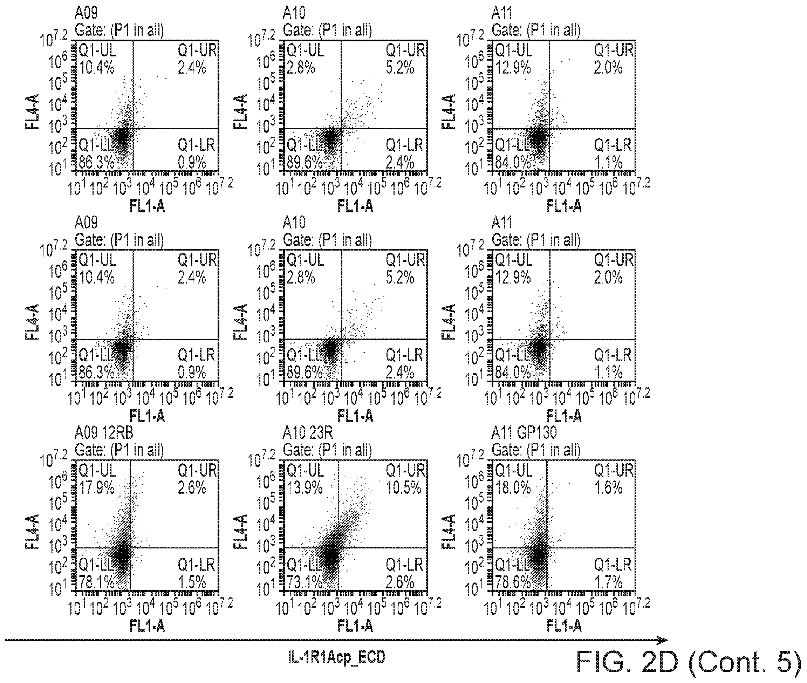

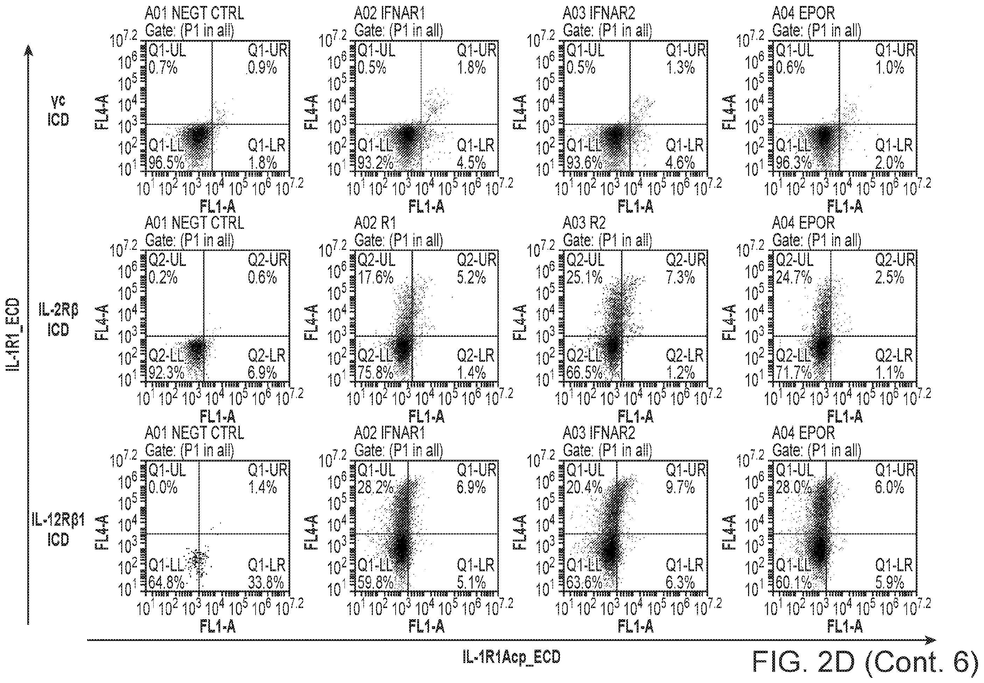

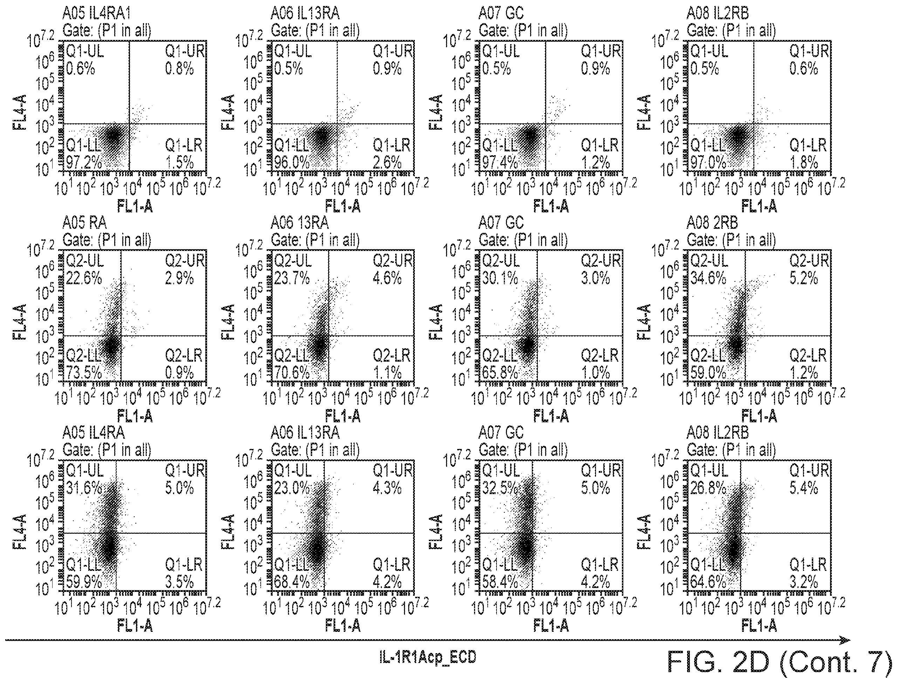

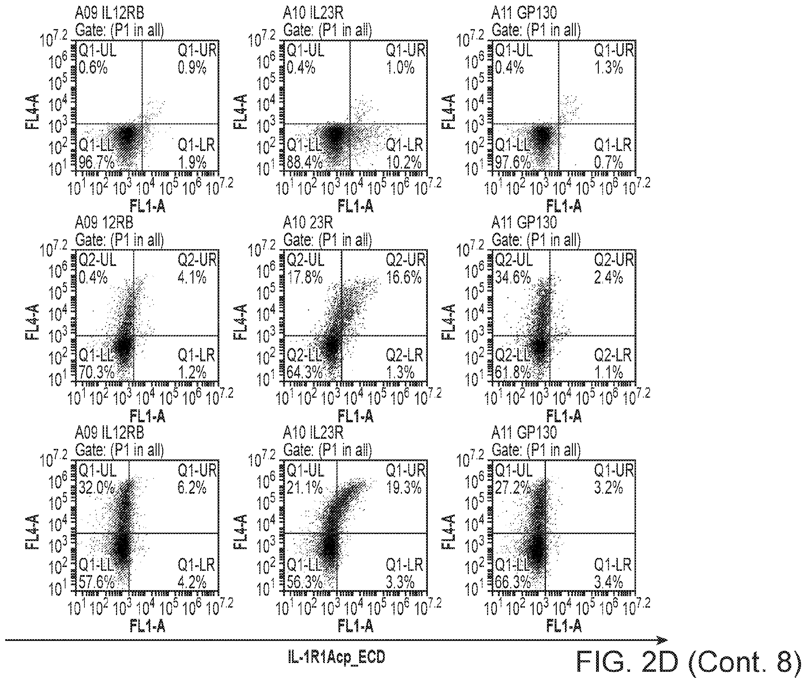

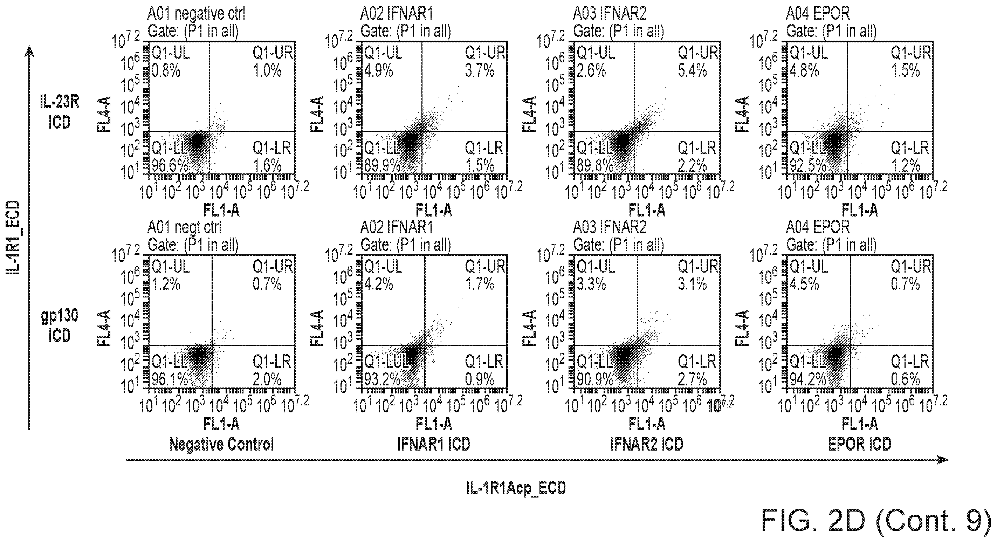

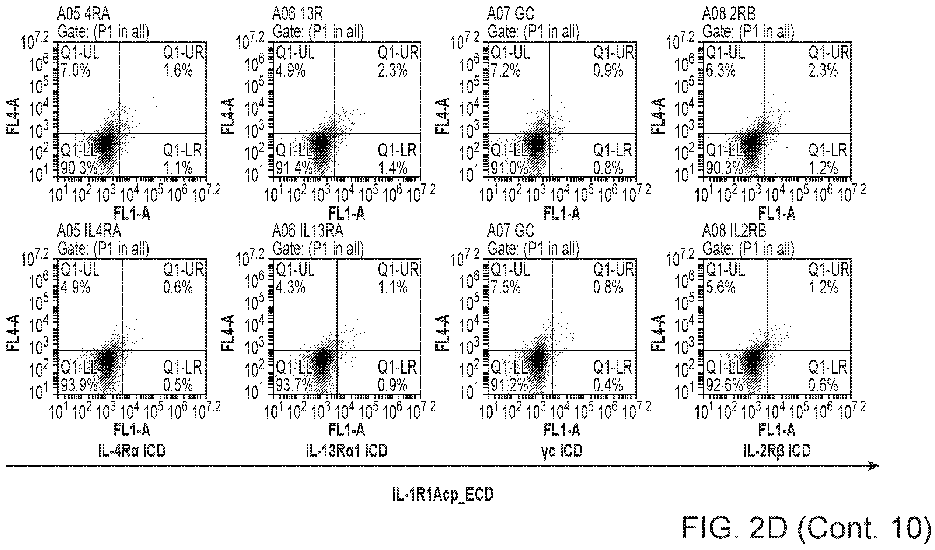

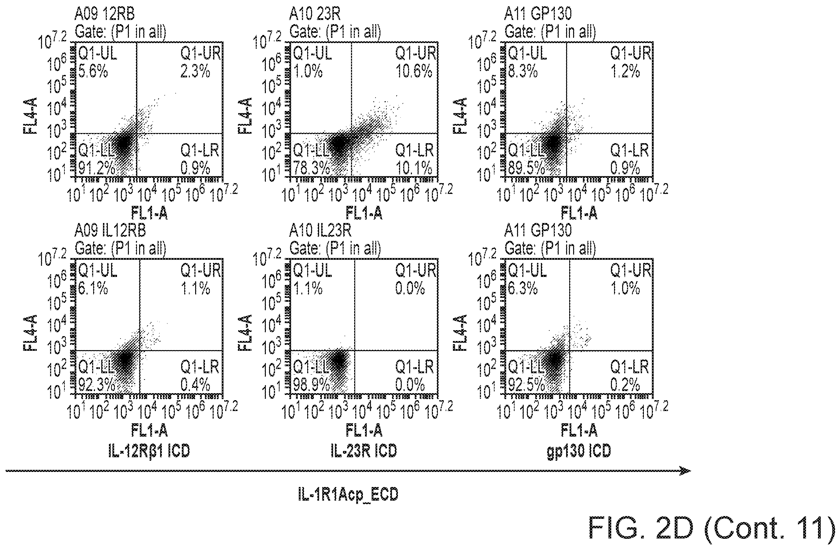

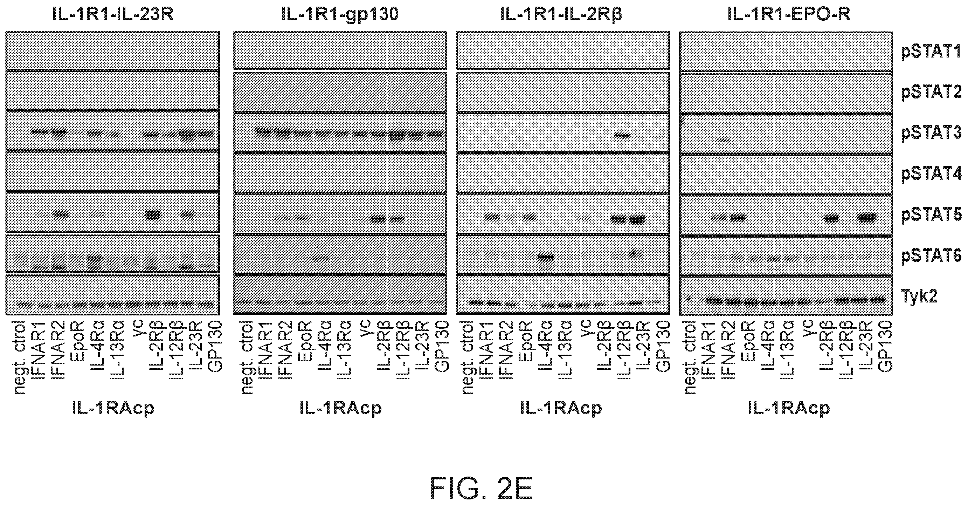

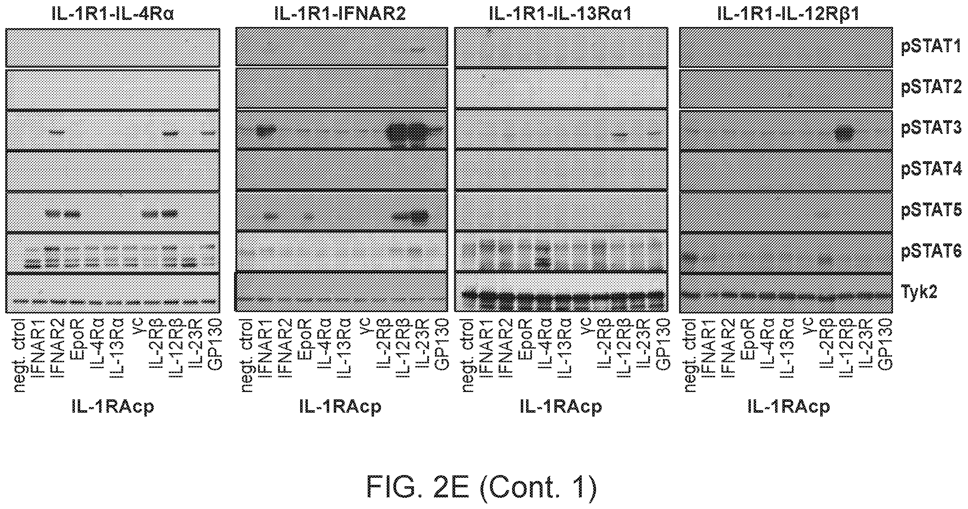

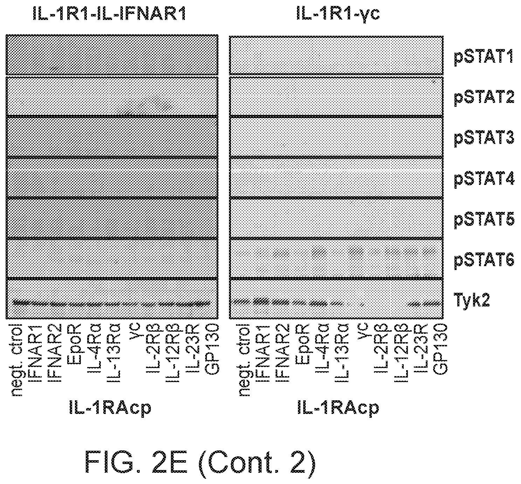

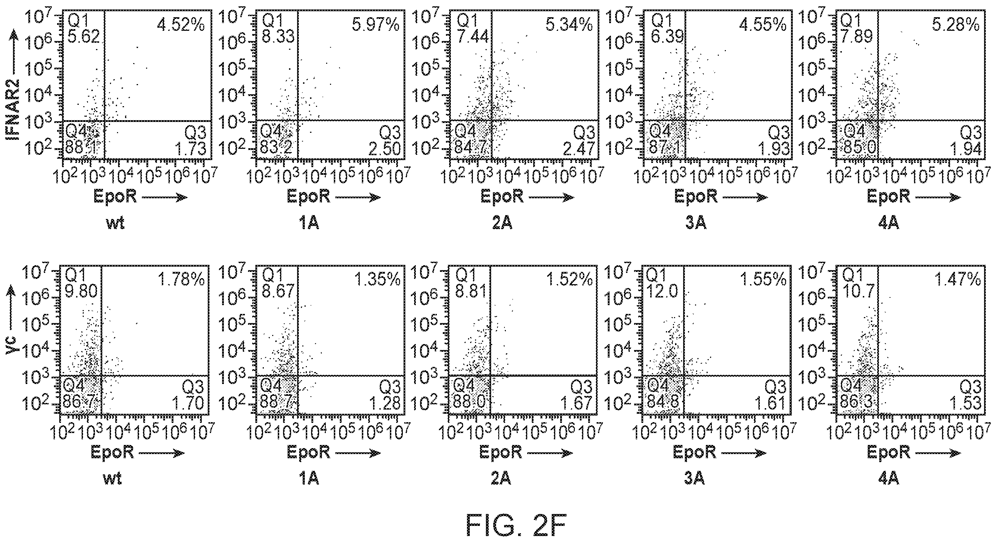

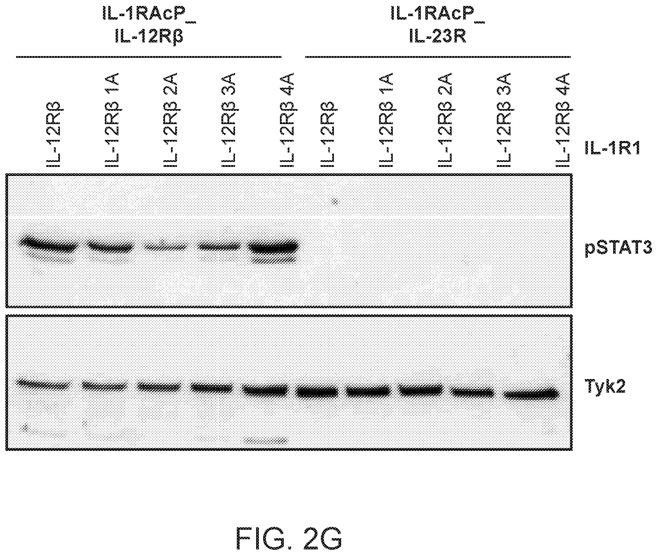

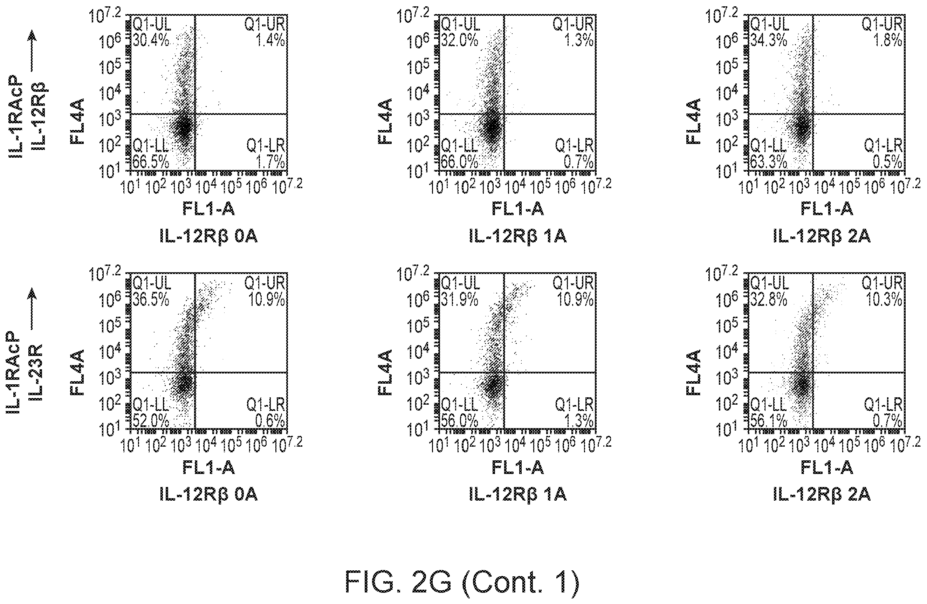

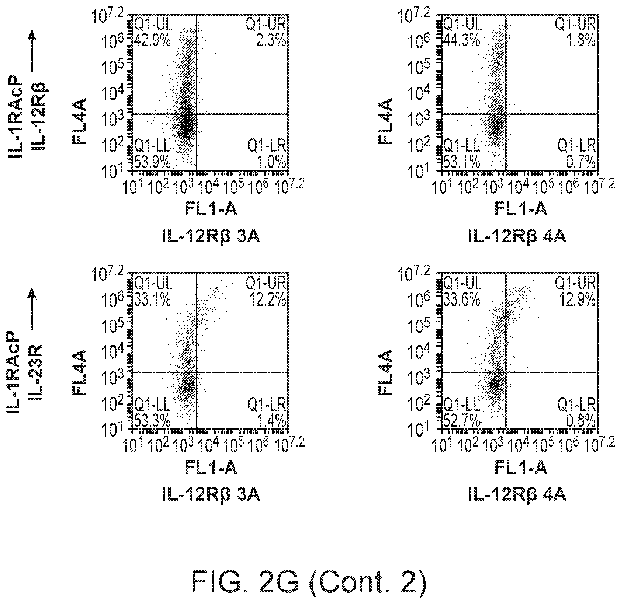

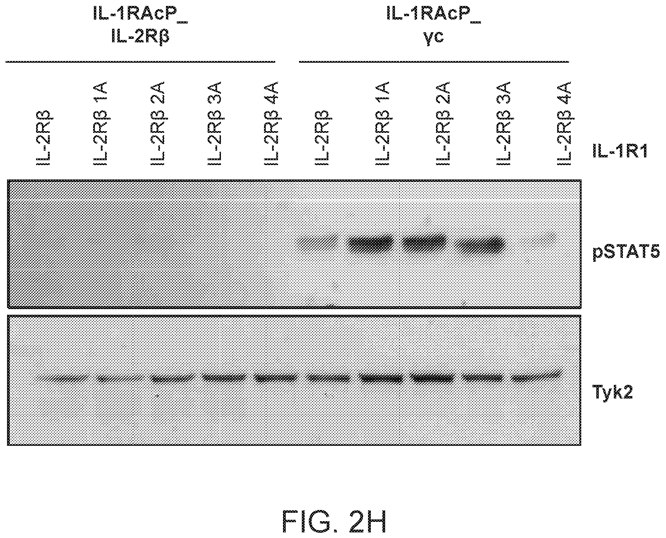

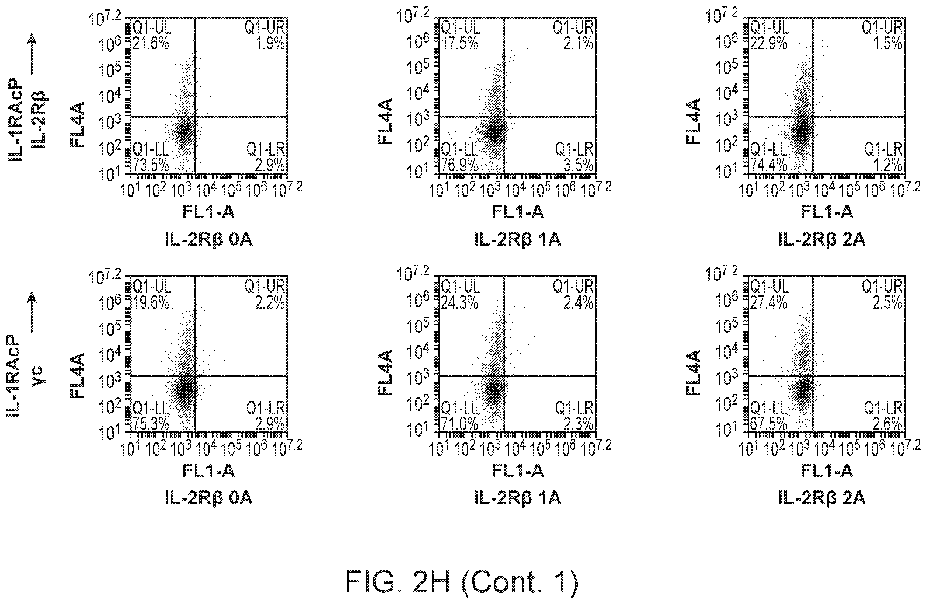

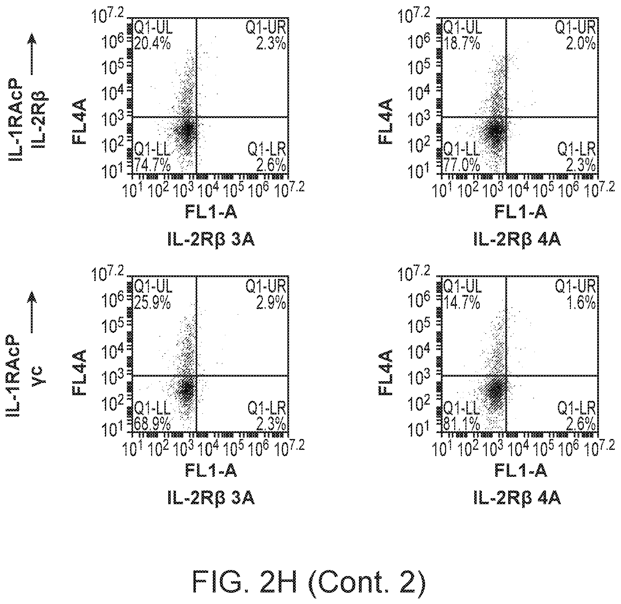

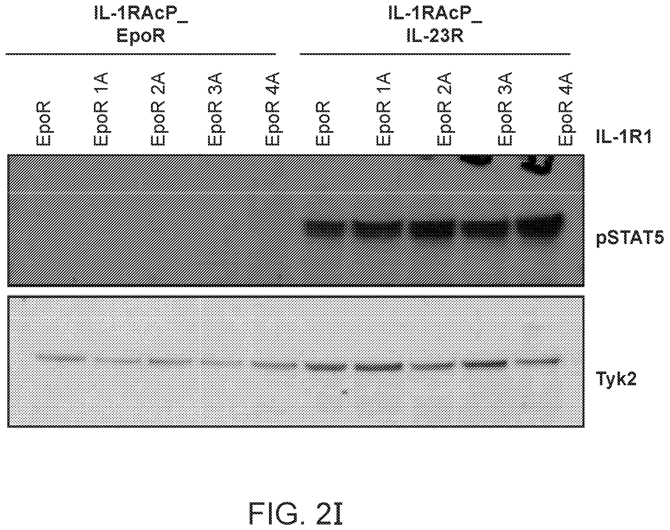

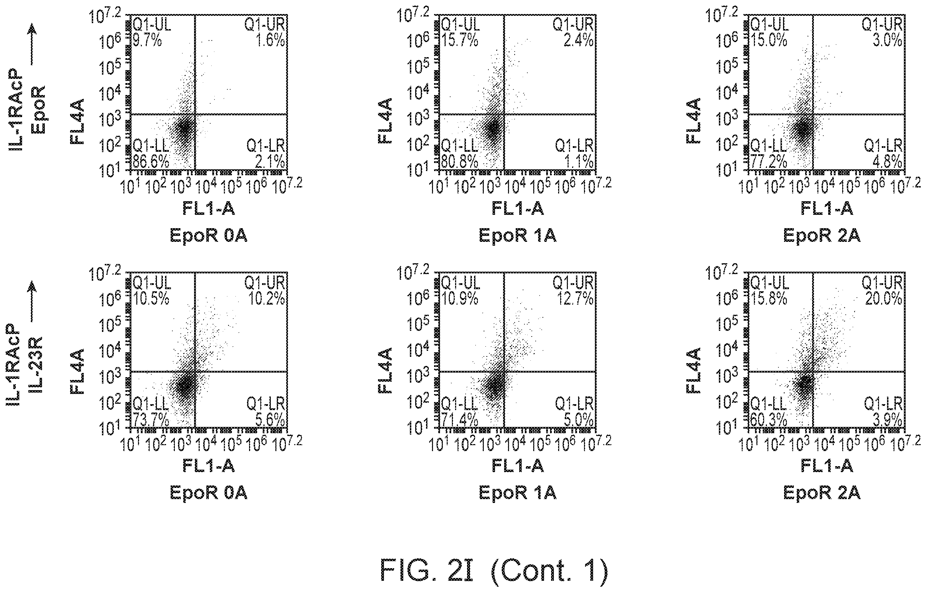

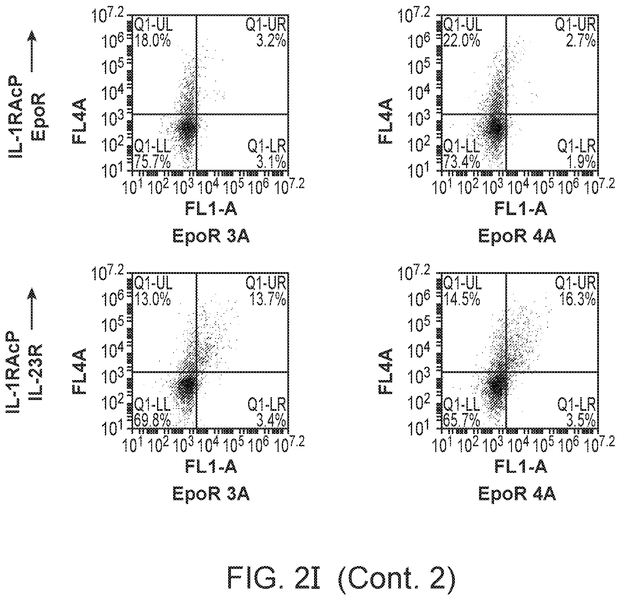

FIG. 2A-2I: Non-natural cytokine receptor pairs activate signaling. FIG. 2A Heatmap representation of STAT molecules activated by the 100 different cytokine receptor pair combinations generated from the chimeric receptor matrix described in Figure 1B. Results were binary coded to 1, presence of band, or 0, absence of band, in western blot analysis. FIG. 2B Schematic representation of the designed IL-1-inducible chimeric receptors (left). Alanine insertion mutagenesis of the EpoR juxtamembrane domain is detailed in the center. Alanine residues (1A, 2A, 3A, or 4A) were inserted after R₂₅₁. Alpha-helical wheel projections of the register twists introduced by alanine residue addition are presented at right bottom. Each residue adds a 109° rotation, with insertion of 3A residues bringing the register close to the original position. FIG. 2C Phospho-STAT3 (pSTAT3) and pSTAT5 levels measured by western blot in IL-1-activated Jurkat cells expressing the indicated chimeric receptor pairs. Insertion of two alanines recovers signaling by the IL-1R1-EpoR/IL-1R1AcP-γc receptor pair. Total levels of TYK2 are presented as a loading control. The western blot presented is a representative example of two independent experiments. FIG. 2D Cell surface expression of chimeric receptors in Jurkat cells. Flow cytometry dot plots of IL-1R1 and IL-1R1Acp chimeric receptors surface expression levels in Jurkat cells 24 hr after transfection. FIG. 2E Signaling profiles activated by chimeric receptors in Jurkat cells. pSTAT1, pSTAT2, pSTAT3, pSTAT4, pSTAT5 and pSTAT6 levels measured by western blot in IL-1-activated Jurkat cells expressing the indicated chimeric receptor pairs. Total levels of TYK2 are presented as a loading control. FIG. 2F Cell surface expression of EpoR chimeric receptors in Jurkat cells. Flow cytometry dot plot representation of the cell surface levels of the indicated chimeric receptors in Jurkat cells 24 hr post-transfection. FIG. 2G, FIG. 2H, FIG. 2I Alanine insertions do not recover signaling by the IL-23R-IL-12Rβ, IL-2Rβ-IL2Rβ and EpoR-EpoR chimeric receptors. (FIG. 2G, FIG. 2H, FIG. 2I ) STAT activation upon IL-1 stimulation measured by western blot (left panel) and cell surface expression in Jurkat cells measured by flow cytometry (right panel) for chimeric receptors with the indicated alanine insertions.

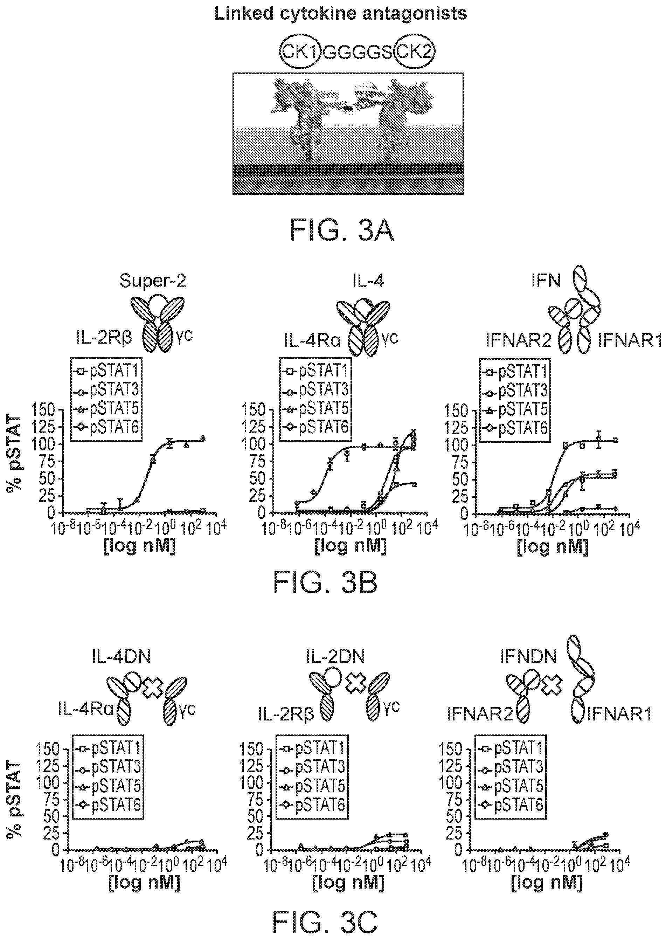

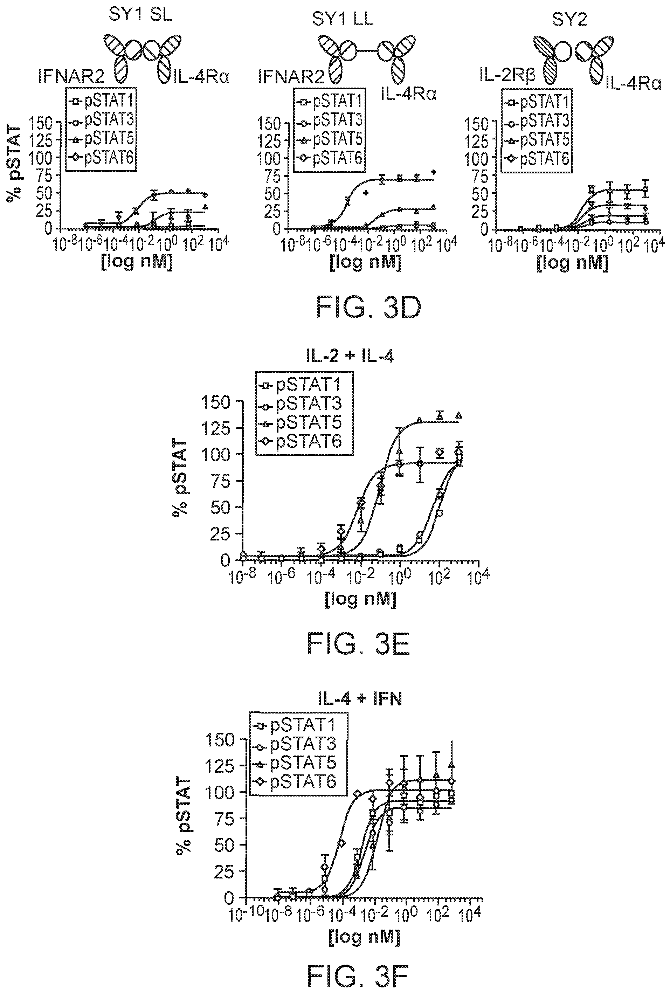

FIG. 3A-3F: Synthekines dimerizing non-natural cytokine receptor pairs activate signaling. FIG. 3A Layout and complex formation by a synthekine. Two dominant negative cytokine variants are genetically fused by a Gly₄/Ser linker, resulting in a new molecule that induces formation of a non-natural cytokine receptor heterodimer. FIG. 3B-3D pSTAT1, pSTAT3, pSTAT5 and pSTAT6 levels activated by the IL-4, Super-2 (affinity-matured variant of IL-2), and IFNω cytokines FIG. 3B, the dominant negative cytokine variants IL-4DN, IL-2DN, and IFNDN FIG. 3C, or the SY1 SL, SY1 LL, and SY2 synthekines FIG. 3D in the Hut78 T cell, as measured by flow cytometry. Data (mean +/- SD) are from two independent replicates. FIG. 3E, FIG. 3F. Signaling profiles activated by stimulation with Super-2/IL-4 and IL-4/IFN cytokine combinations. (FIG. 3A-3B) pSTAT1, pSTAT3, pSTAT5 and pSTAT6 activation levels induced by 15 min stimulation with the indicated concentrations of the Super-2/IL-4 FIG. 3A and IL-4/IFN cytokine combinations in Hut78 cells, as measured by flow cytometry. Data (mean +/- SD) are from three independent experiments.

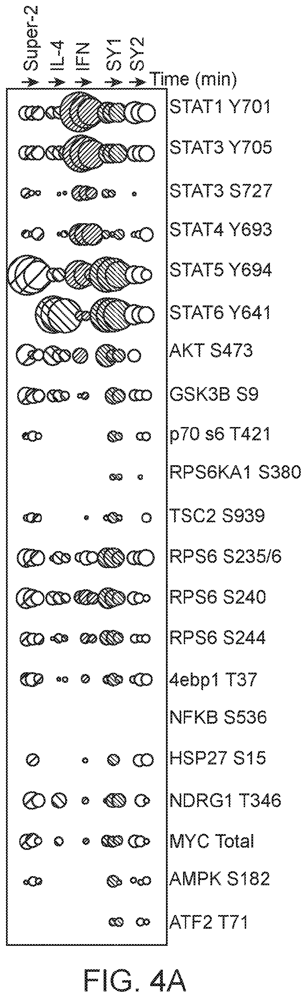

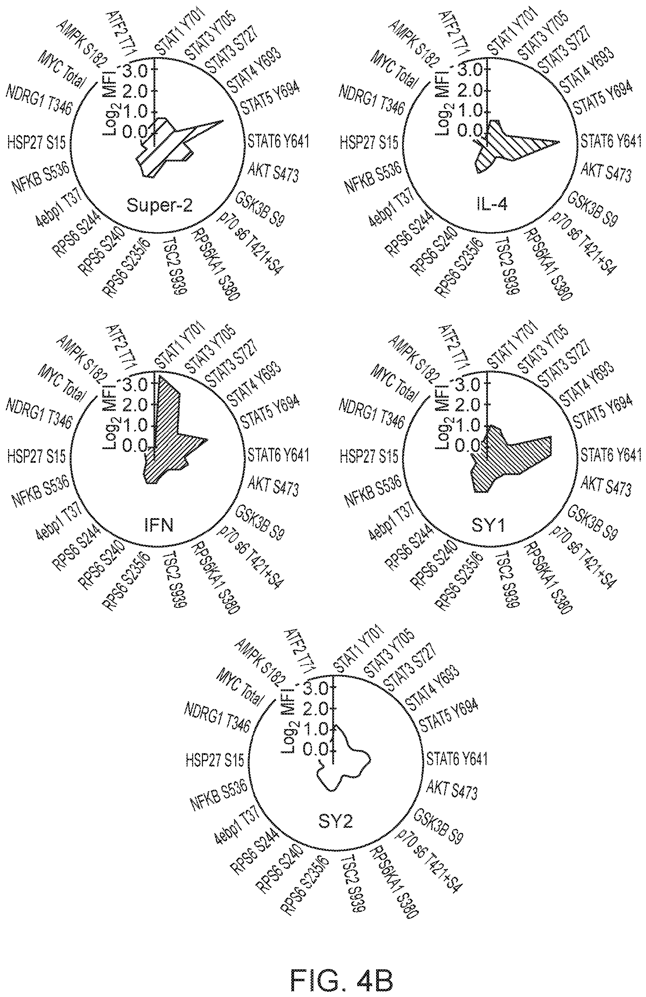

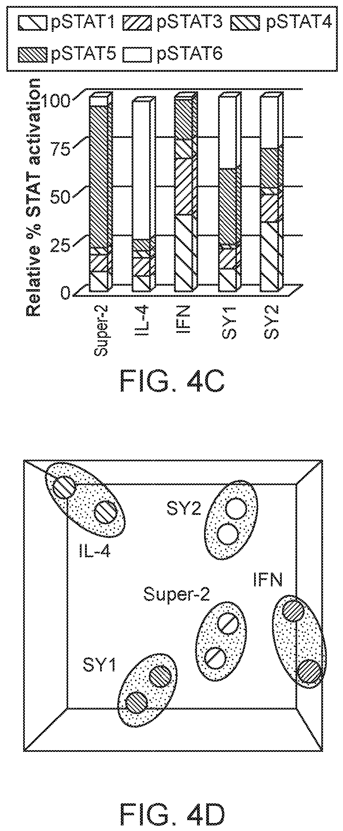

FIG. 4A-4D: Synthekines activate different signaling programs than genome-encoded cytokines. FIG. 4A Bubble plot representation of the signaling pathways activated by the indicated ligands after stimulation for 15, 60 or 120 min in Hut78 T cells. The size of the bubble represents the intensity of the signal activated. FIG. 4B Filled radar representation of the signaling molecules activated by the genome-encoded cytokines and synthekines following 15 min stimulation in Hut78 cells. The signaling molecules activated by the ligands are shown on the perimeter of the circle and their respective activation potencies are denoted by the radius of the circle. The different shapes of the filled radar exhibited by the different ligands define their distinct signaling signatures. FIG. 4C Ratio of STAT activation by cytokines and synthekines after 15 min stimulation on Hut78 cells. Each column represents the total STAT activation by each ligand normalized to 100%. The relative activation potency of each STAT is corrected accordingly. The different distribution of STAT activation by the various ligands suggest differential STAT usages between genome-encoded cytokines and synthekines. Data (mean) are from two independent replicates. FIG. 4D Unsupervised clustering of signaling programs engaged by cytokines and synthekines. Principal Component Analsysis (PCA) of signaling programs engaged by genome-encoded cytokines and synthekines after 15 and 60 min stimulation in Hut78 cells. Genome-encoded cytokines and synthekines signatures are separated in the space by equivalent distances, indicating that synthekines signaling programs are as different from the parental cytokines as they are from each other.

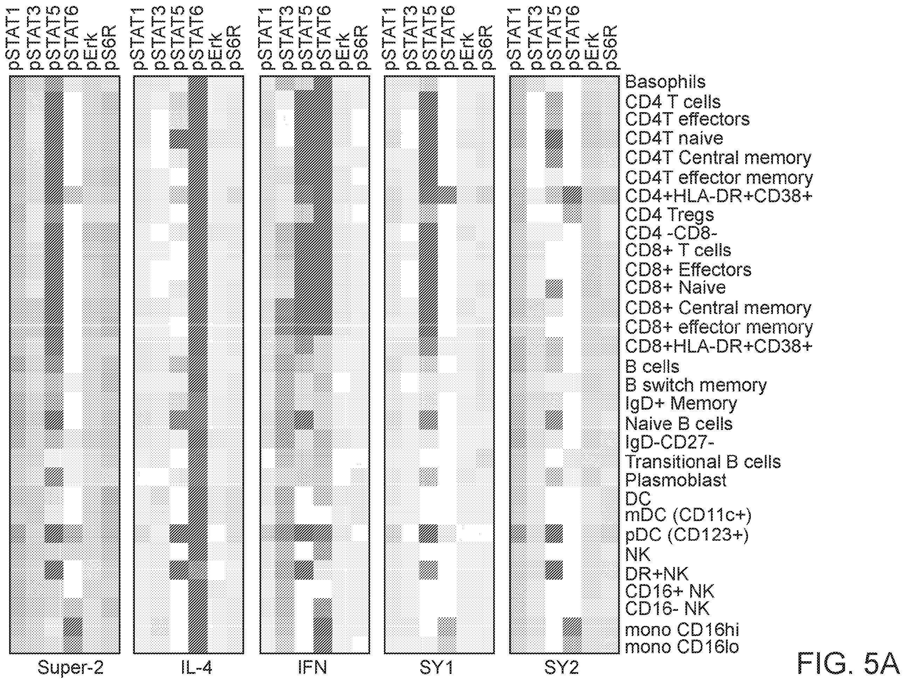

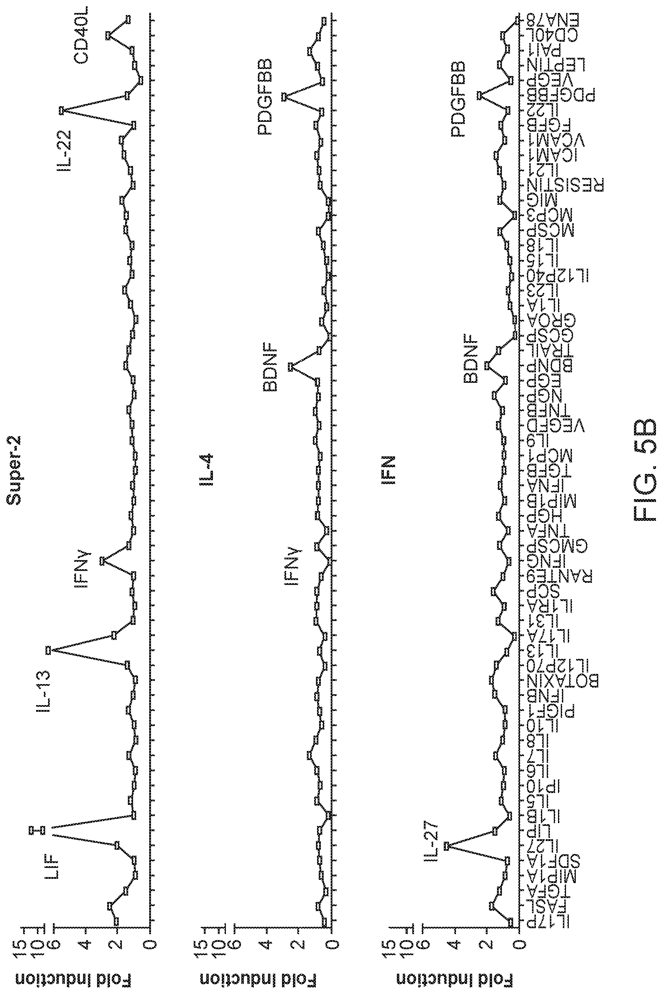

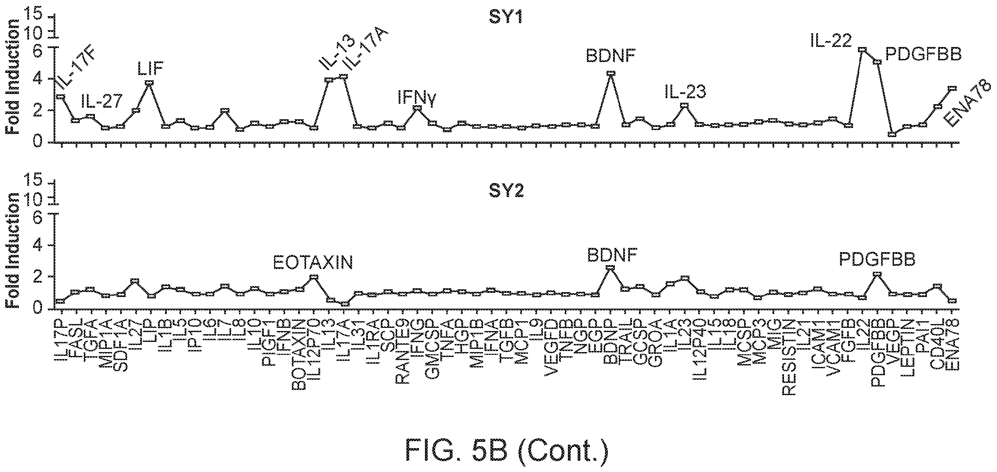

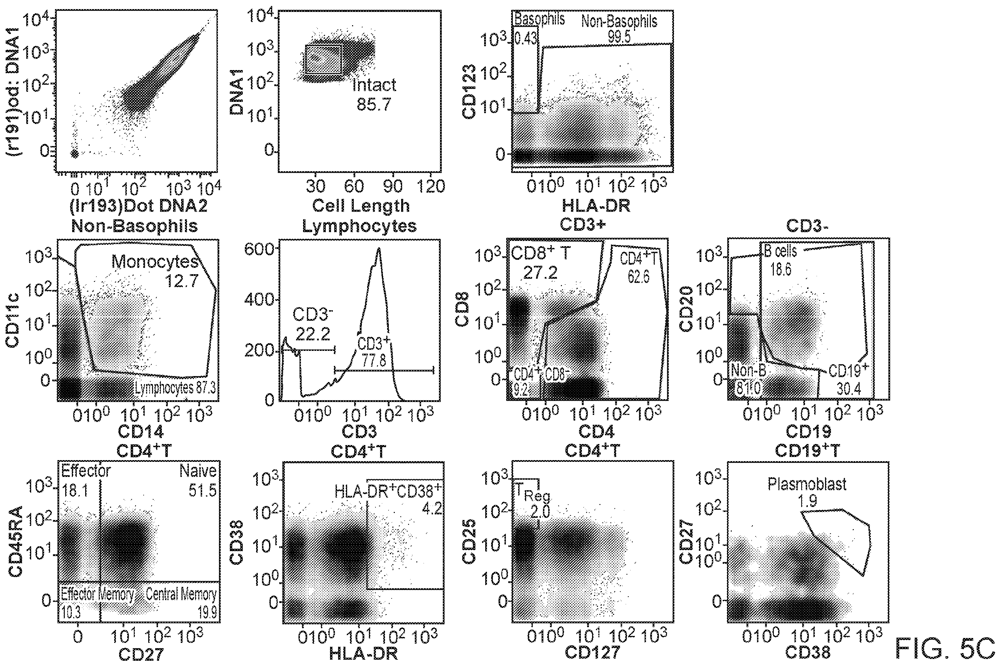

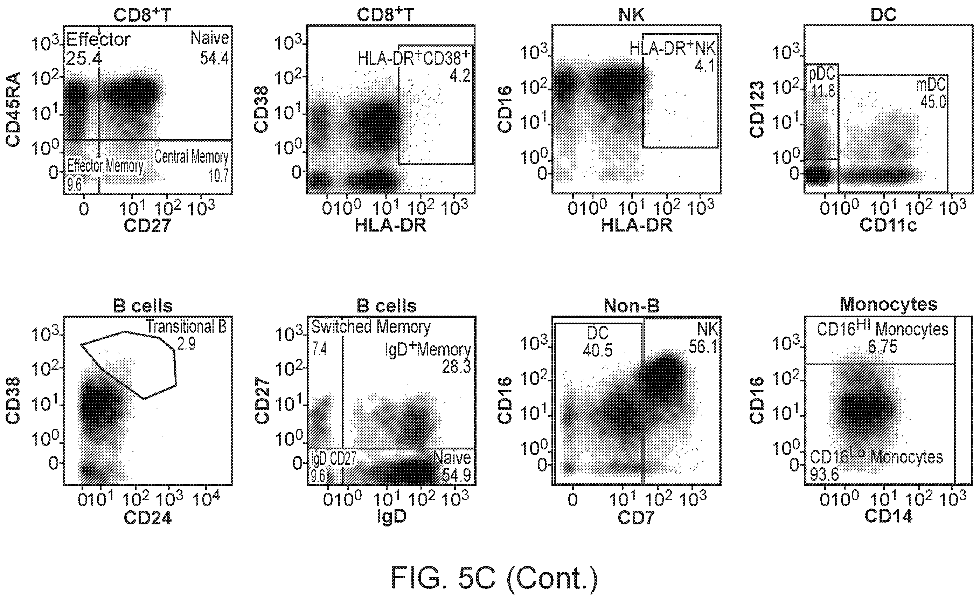

FIG. 5A-5C: Synthekines elicit different cellular signatures and immune activities than genome-encoded cytokines. FIG. 5A Heat map representations of the activation levels of six signal effectors induced by saturating doses of the indicated ligands in 29 immune cell types profiled from PBMCs, as measured by mass cytometry (CyTOF). Data (mean) are from two independent replicates. FIG. 5B Detailed analysis of the secretion profiles of 63 cytokines from PBMCs stimulated with the indicated ligands. Cytokines that were secreted more than 2 fold above background are labeled. Data (mean +/- SD) are from two independent replicates. FIG. 5C Immune cell profiling to identify signaling signatures activated by cytokines versus synthekines. Gating scheme for the mass cytometry-mediated identification of the 29 distinct immune cell subsets within peripheral blood mononuclear cells (PBMCs) isolated from whole blood that were used to assess signal effector activation responses to cytokine versus synthekine treatment.

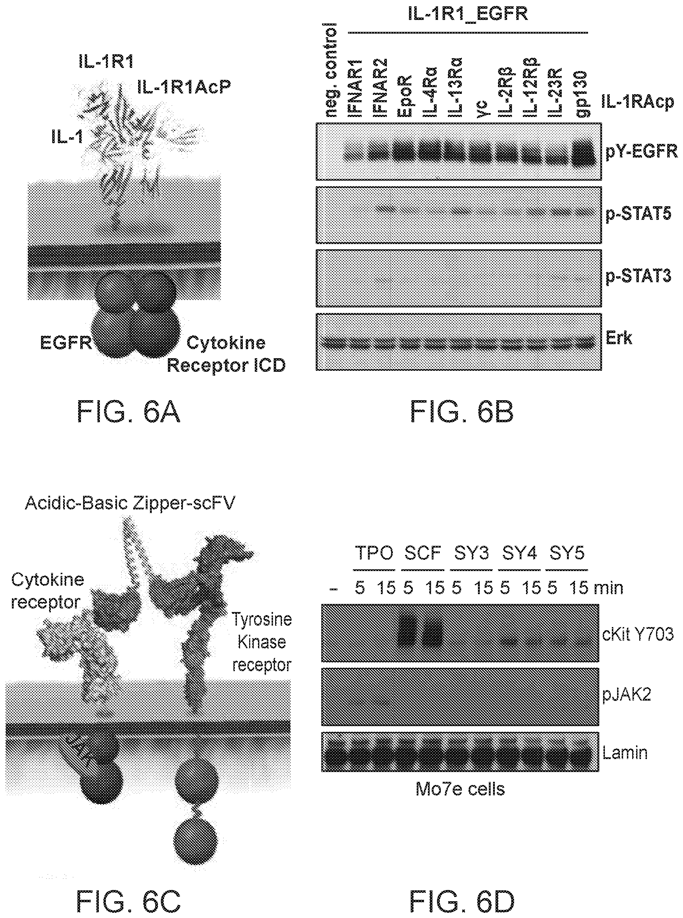

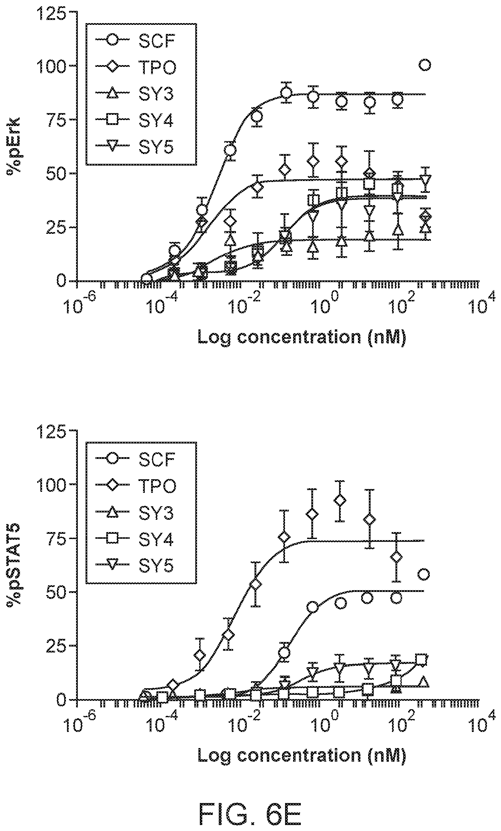

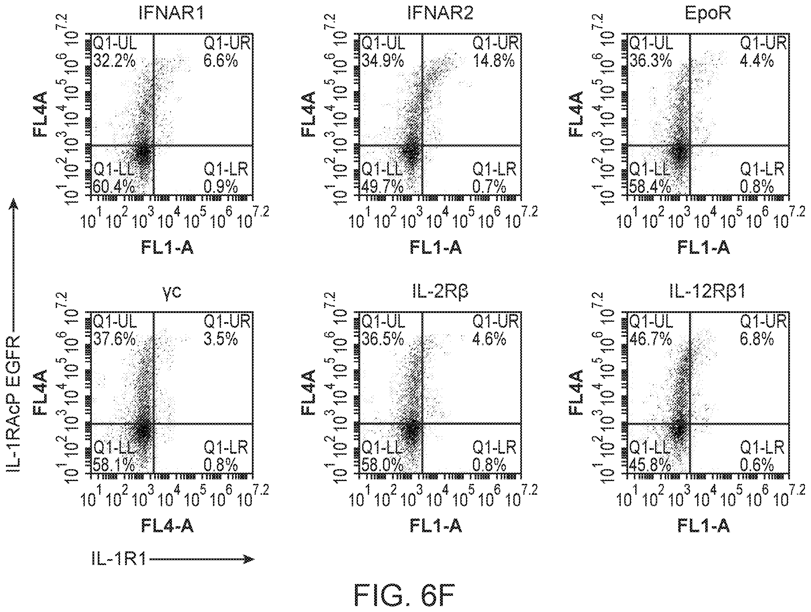

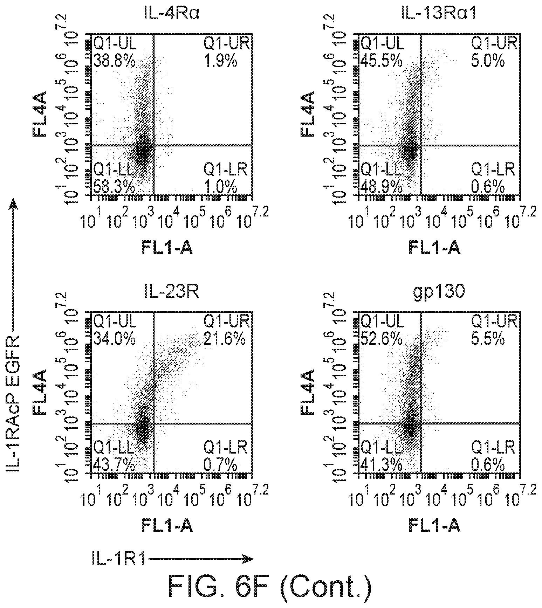

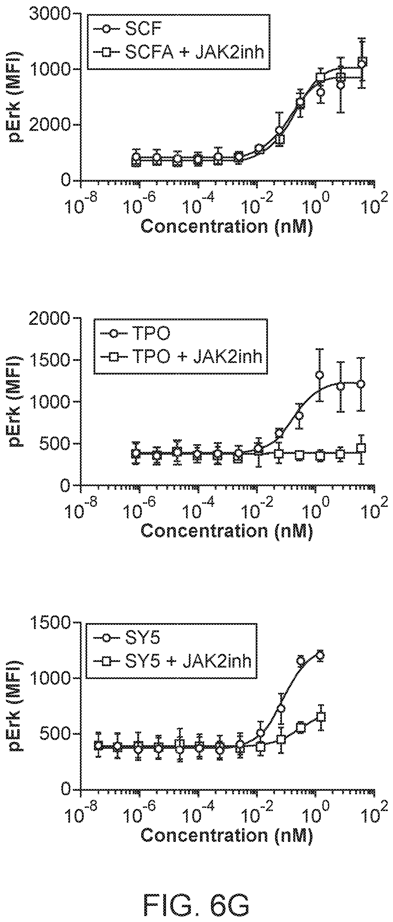

FIG. 6A-6G: Synthekines dimerizing a cytokine receptor and a tyrosine kinase receptor activate signaling. FIG. 6A Schematic representation of the IL-1-mediated complexation of IL1-R1-EGFR and IL-1R1AcP-cytokine receptor chimeras. FIG. 6B Phospho- EGFR (pY EGFR), pSTAT3 and pSTAT5 levels measured by western blot analysis in IL- 1-activated Jurkat cells expressing the indicated chimeric receptor pairs. Total levels of Erk are presented as a loading control. The western blot presented is a representative example of two independent experiments. FIG. 6C Layout and complex formation by a synthekine dimerizing a cytokine receptor and a tyrosine kinase receptor. Two scFvs binding a cytokine receptor and a tyrosine kinase receptor respectively are genetically fused to acidic or basic leucine zippers, resulting in a new molecule able to form a heterodimeric receptor complex that does not exist in nature. FIG. 6D Phospho cKit Y703 and pJAK2 levels measured by western blot in Mo7E cells after stimulation with synthekines that dimerize TpoR and cKit (SY3, SY4 and SY5) for the indicated time periods. Total levels of Lamin are presented as a loading control. The western blot presented is a representative example of two independent experiments. FIG. 6E Erk (left panel) and STAT5 (right panel) phosphorylation activated by 10 min stimulation with the indicated doses of SCF, TPO, or the indicated synthekines in Mo7e cells, as measured by flow cytometry. Data (mean +/- SD) are from three independent replicates. FIG. 6F, FIG. 6G Functional characterization of tyrosine kinase receptor/cytokine receptor dimerizing synthekines. FIG. 6F Flow cytometry plots representing the surface expression levels of the indicated chimeric receptors in Jurkat cells 24 hr post-transfection. FIG. 6G Erk activation response to 10 min stimulation with the indicated doses of SCF, TPO, and SY5 +/- JAK2 inhibitor in Mo7e cells, as measured by flow cytometry. Data (mean +/- SD) are from three independent experiments.

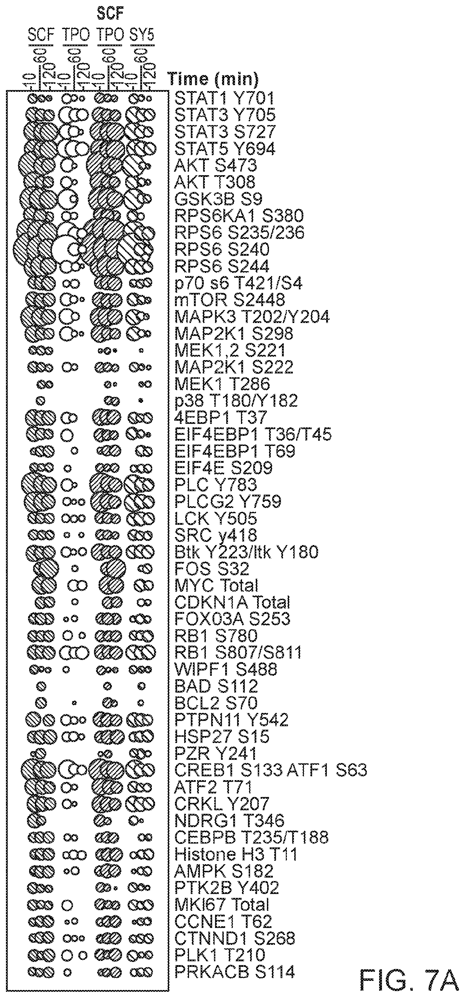

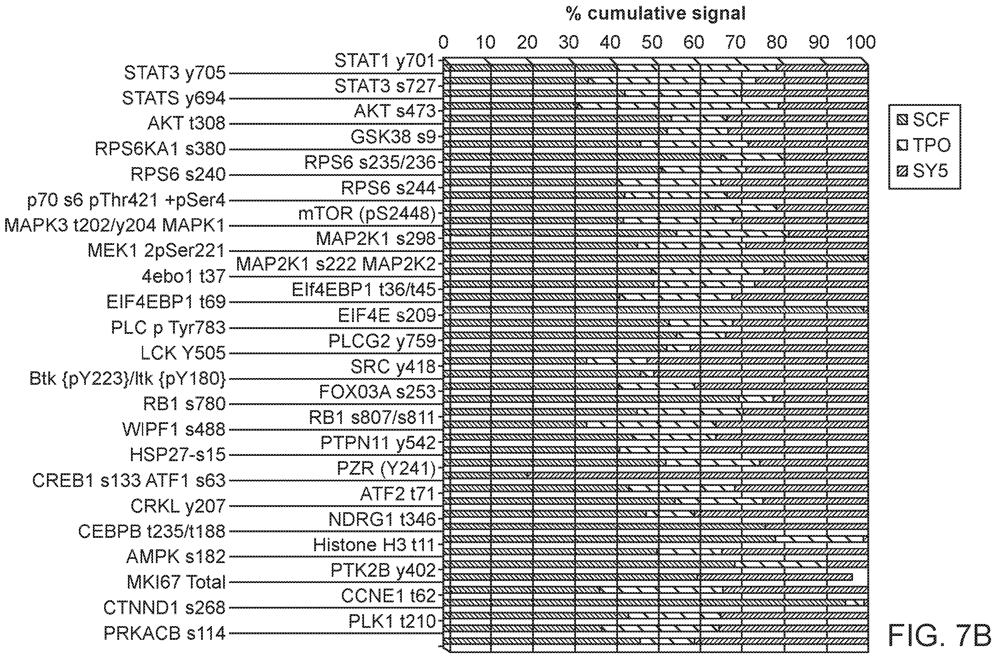

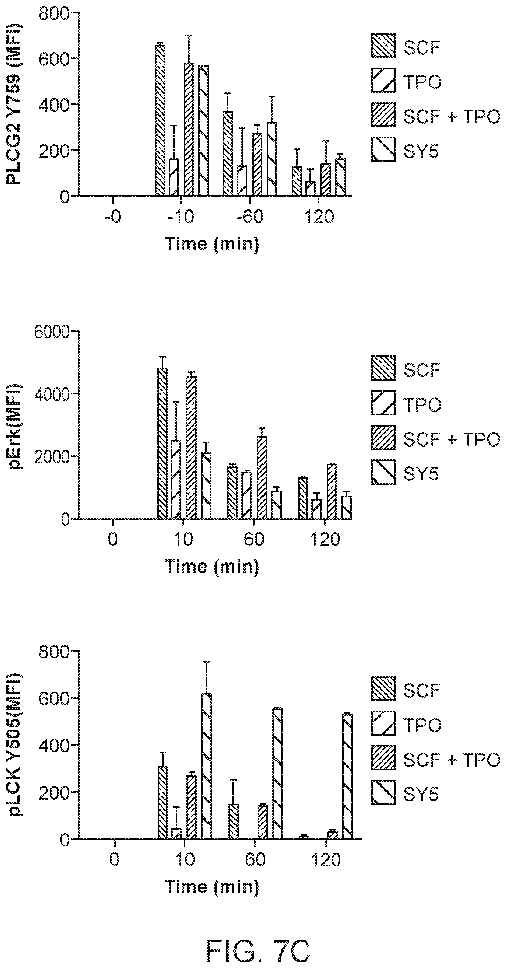

FIG. 7A-7C: Synthekines dimerizing a cytokine receptor and a tyrosine kinase receptor activate different signaling programs than their natural ligands. FIG. 7A Bubble plot representation of the signaling pathways activated by the indicated ligands after stimulation for 10, 60 and 120 min in Mo7e cells. The size of the bubble represents the intensity of the signal activated. FIG. 7B Stack column representation of the signaling molecules engaged by SCF, TPO and SY5 after 10 min stimulation in Mo7e cells. For each molecule, the combined activation of the three ligands was normalized to 100% and the relative contribution of each ligand was corrected accordingly. Some molecules were better activated by SCF, others were better activated by TPO and yet others were better activated by SY5. Data presented in panel A and B represents the mean value of two independent experiments performed in triplicate. FIG. 7C pPLCG2, pErk, and pLCK levels induced by the indicated ligands in Mo7e cells after 10, 60 and 120 min stimulation. Data (mean +/- SD) are from three independent replicates.

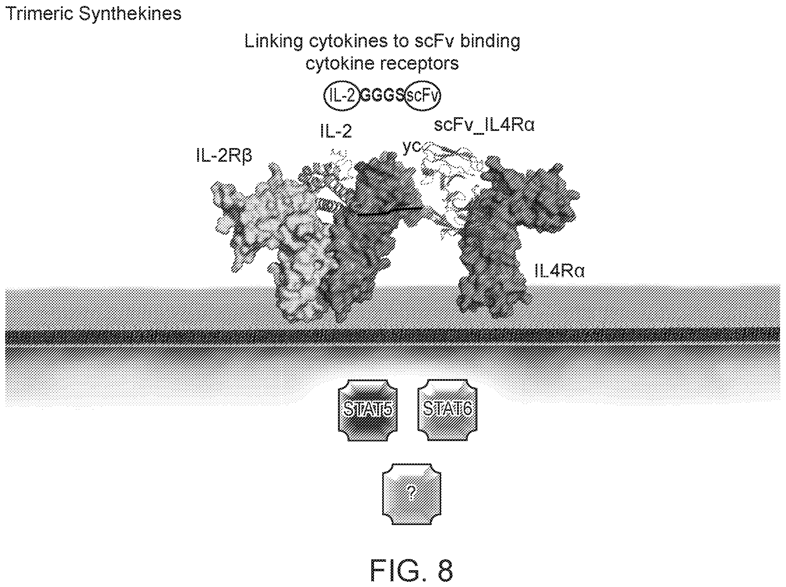

FIG. 8: A trimeric synthekine (SY3) was designed, joining through a gly₄ser linker IL-2 to a scFv that specifically binds to IL-4Rα. The trimeric synthekine therefore binds to 3 receptor polypeptides, γc, IL-2Rβ, and IL-4Rα.

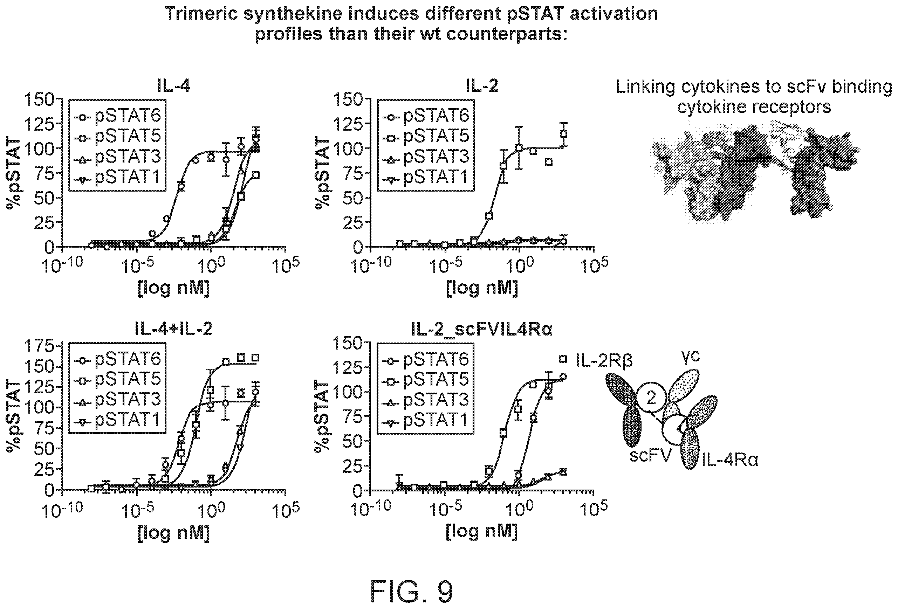

FIG. 9: Trimeric synthekine induces different pSTAT activation profiles than wild-type counterparts.

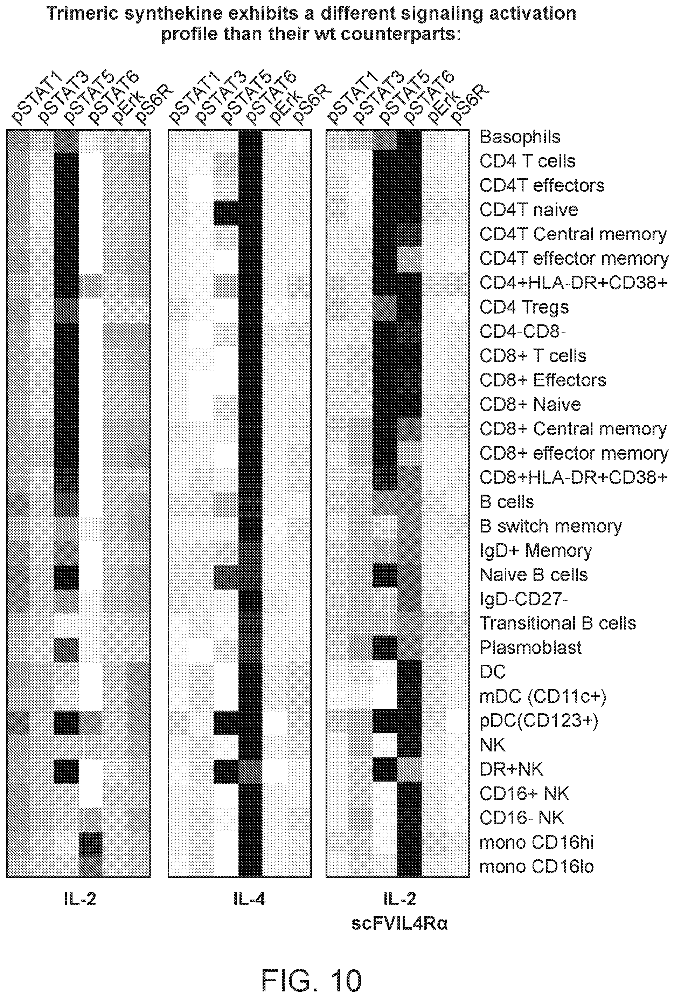

FIG. 10: Trimeric synthekine exhibits a different signaling activation profile than wild-type counterparts.

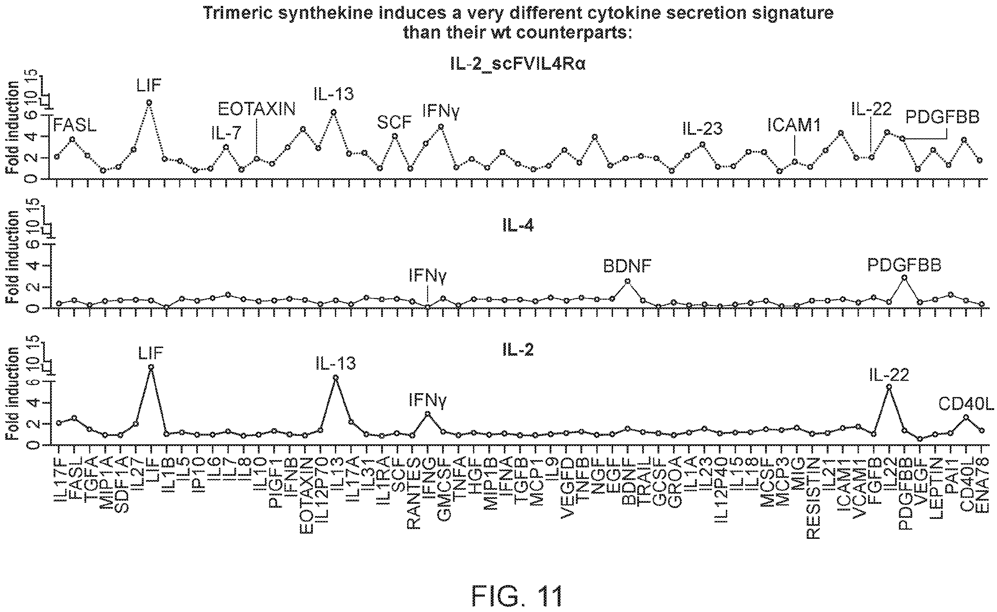

FIG. 11: Trimeric synthekine induces a very different cytokine secretion signature than wild-type counterparts.

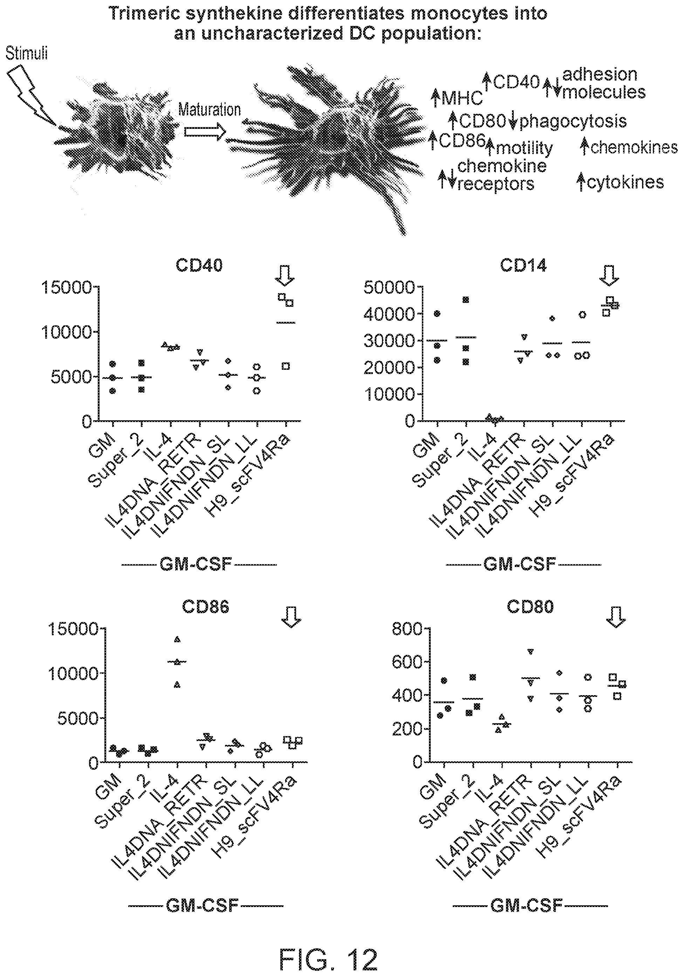

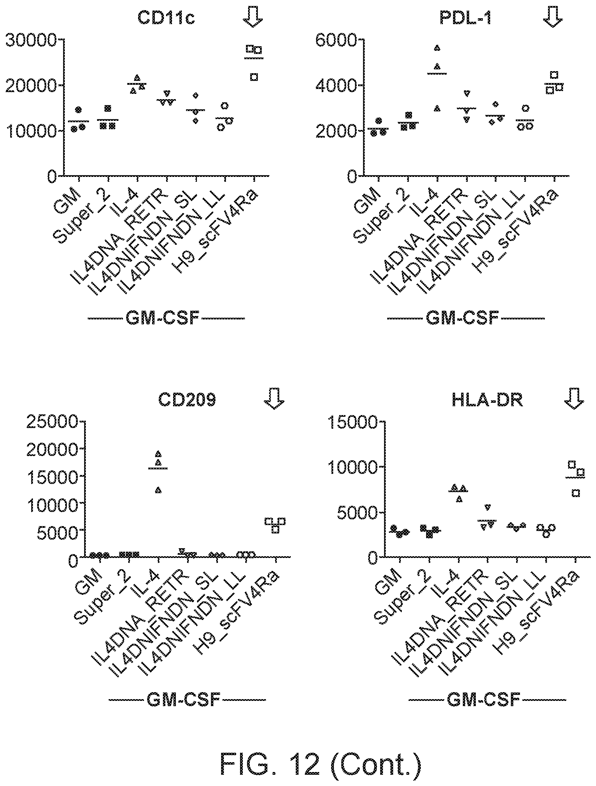

FIG. 12: Trimeric synthekine differentiates monocytes in a previously uncharacterized dendritic cell population.

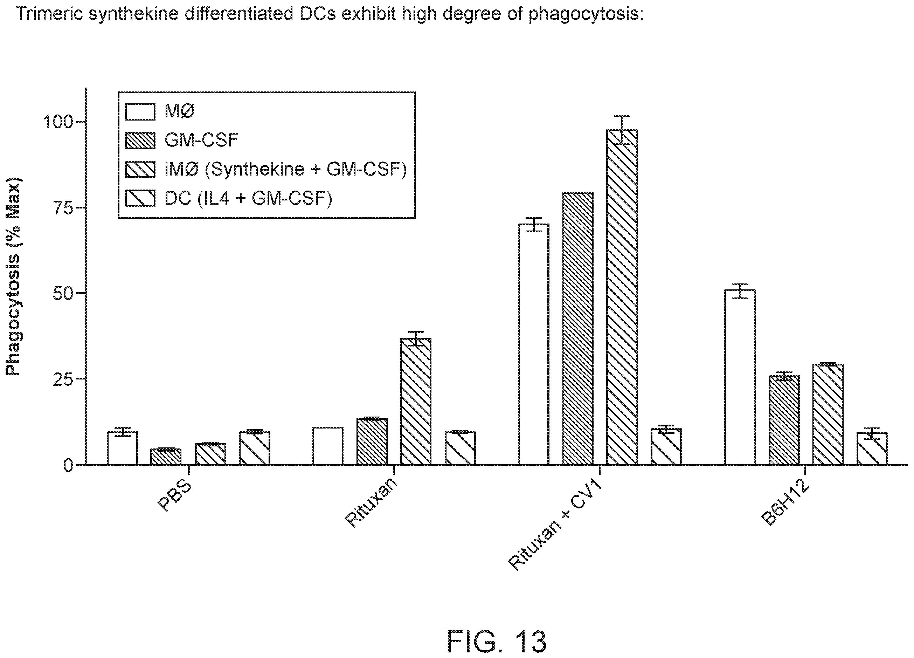

FIG. 13: Trimeric synthekine differentiated dendritic cells exhibit a high degree of phagocytosis.

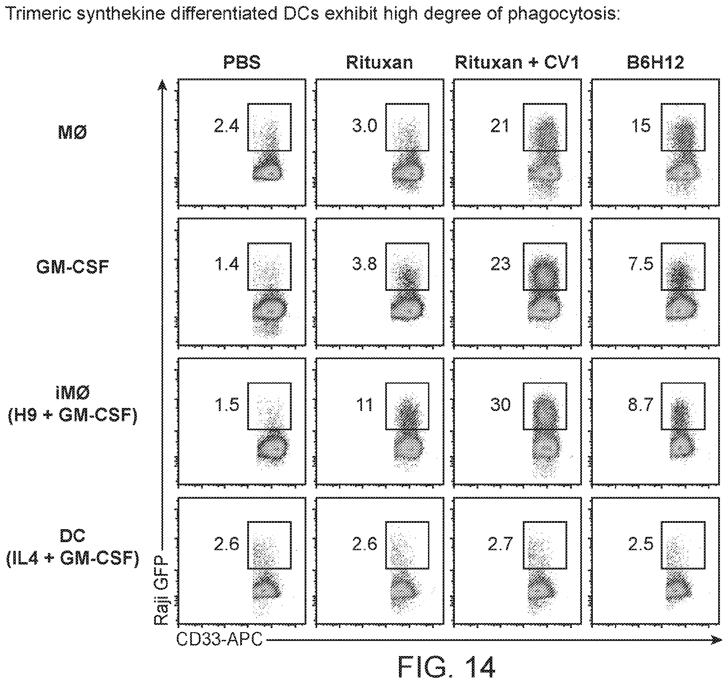

FIG. 14: Trimeric synthekine differentiated dendritic cells exhibit a high degree of phagocytosis.

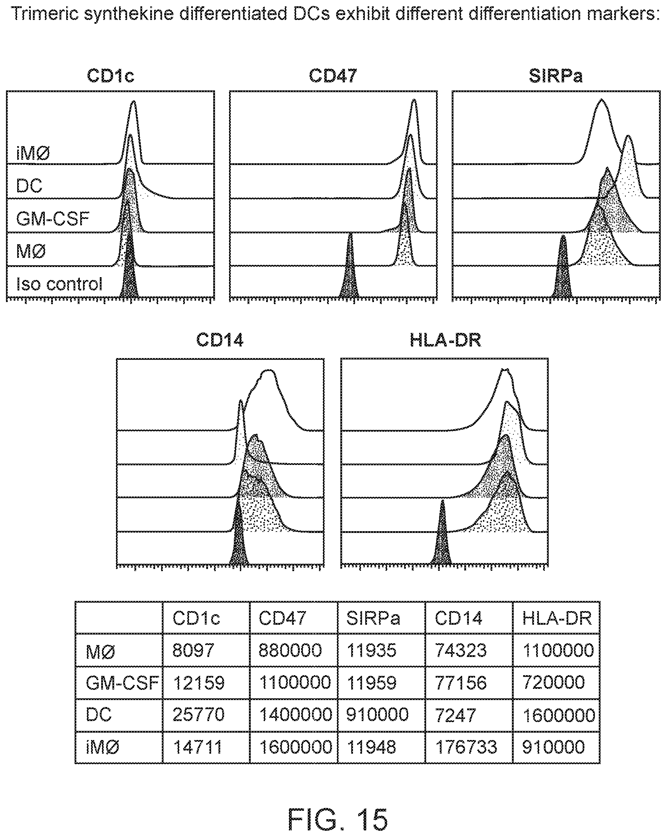

FIG. 15: Differentiation markers on trimeric synthekine differentiated dendritic cells differ from native cell populations.

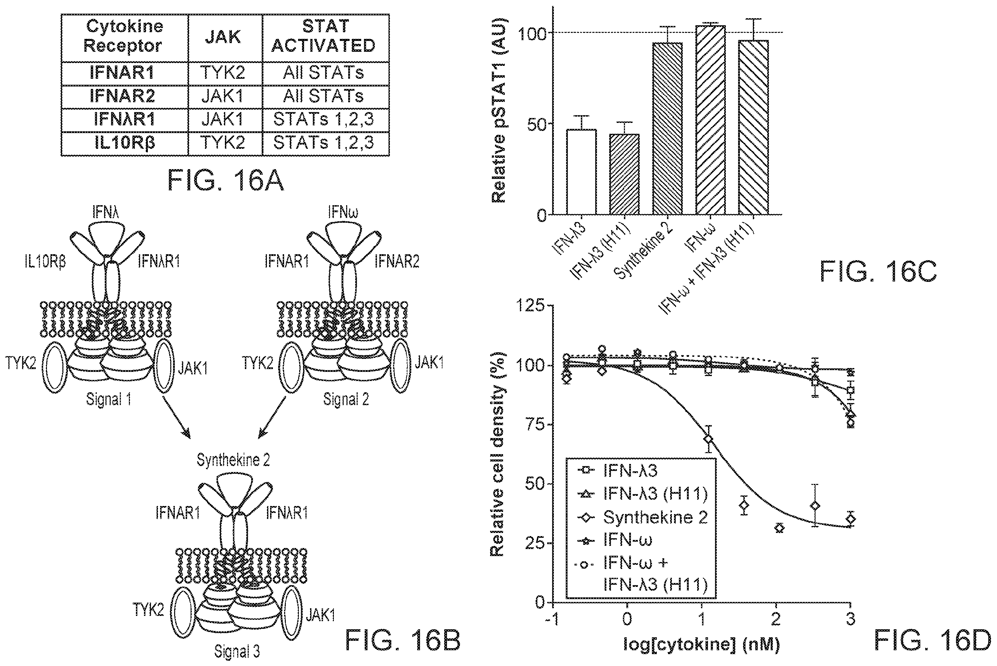

FIG. 16. IL-2λ synthekine. Synthekine (SY6) is a hybrid Interferon that dimerizes type I and type III IFN receptors. A) Table of IFN receptors, their associated JAKs, and STATs activated upon receptor dimerization. B) SY6 is a hybrid interferon that dimerizes IFNAR1 and IFNλR1 receptors and their respective JAKs. C) The Emax of phospho-STAT1 activation by SY6 is equal to that of type I IFN and twice the signal induced by type III IFNs. Error bars represent ± SEM (n = 3). D) SY6 potently induces the anti-proliferative effect whereas type I IFN, type III IFN or a combination type I and III IFN treatment is ineffective. Error bars represent ± SEM (n = 3). Phospho-STAT1 signaling and anti-proliferative assays were performed in Hap1 cells which are naturally responsive to both type I and type III IFNs.

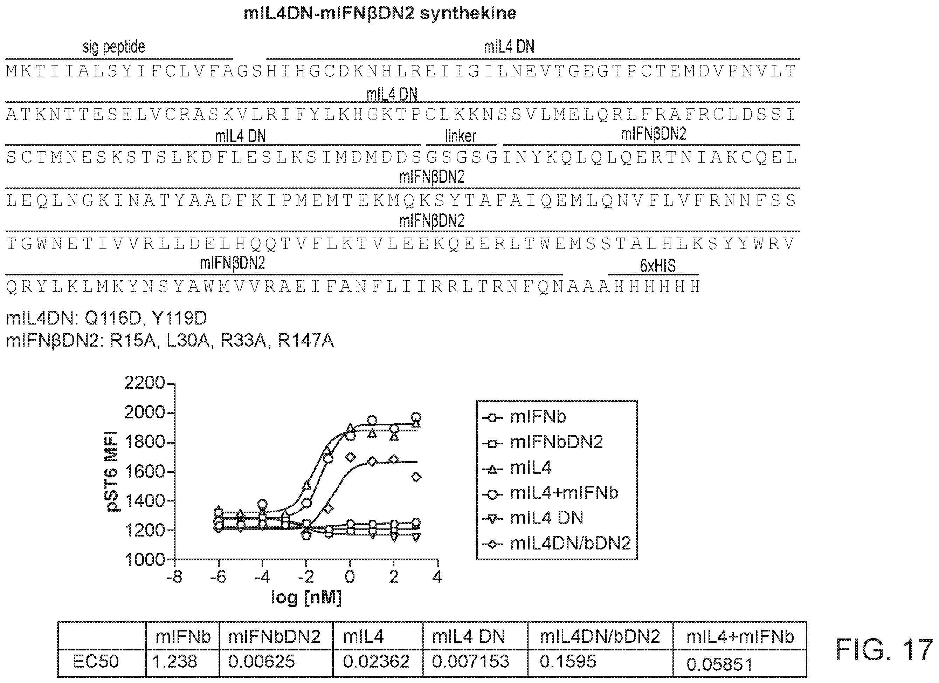

FIG. 17. IL-4DN-IFNβDN2 (SY7) joins IL-4DN with IFNβ2DN2 through a gly/ser linker. Lymphocytes were isolated from spleen/LNs of C57BL/6 mice, and activated with plate-bound anti-CD3 (2.5 µg/ml) + soluble anti-CD28 (5 µg/ml) for 48H. Cells were then rested overnight in 10 IU/ml mIL2, then serum-starved for 4H prior to stimulation with indicated cytokine/synthekine for 20'. Cell signaling terminated and cells fixed with PFA, permeabilized with Permlll buffer (BD) and stained with phosphoSTAT6(Y641) antibody (BD). Sequence is provided as SEQ ID NO:2.

### DETAILED DESCRIPTION

In order for the present disclosure to be more readily understood, certain terms and phrases are defined below as well as throughout the specification. The definitions provided herein are non-limiting and should be read in view of what one of skill in the art would know at the time of invention.

### Definitions

Before the present methods and compositions are described, it is to be understood that this disclosure is not limited to particular method or composition described, as such may, of course, vary. It is also to be understood that the terminology used herein is for the purpose of describing particular examples only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the disclosure. The upper and lower limits of these smaller ranges may independently be included or excluded in the range, and each range where either, neither or both limits are included in the smaller ranges is also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this invention belongs. Although any methods and materials similar or equivalent to those described herein can be used in the practice or testing, some potential and preferred methods and materials are now described.

It must be noted that as used herein and in the appended claims, the singular forms "a", "an", and "the" include plural referents unless the context clearly dictates otherwise. Thus, for example, reference to "a cell" includes a plurality of such cells and reference to "the peptide" includes reference to one or more peptides and equivalents thereof, e.g. polypeptides, known to those skilled in the art, and so forth.

The publications discussed herein are provided solely for their disclosure prior to the filing date of the present application.. Further, the dates of publication provided may be different from the actual publication dates which may need to be independently confirmed.

By "comprising" it is meant that the recited elements are required in the composition/method/kit, but other elements may be included to form the composition/method/kit etc. within the scope of the claim. For example, a composition comprising a wnt synthekine is a composition that may comprise other elements in addition to wnt synthekine(s), e.g. functional moieties such as polypeptides, small molecules, or nucleic acids bound, e.g. covalently bound, to the wnt synthekine; agents that promote the stability of the wnt synthekine composition, agents that promote the solubility of the wnt synthekine composition, adjuvants, etc. as will be readily understood in the art, with the exception of elements that are encompassed by any negative provisos.

By "consisting essentially of", it is meant a limitation of the scope of composition or method described to the specified materials or steps that do not materially affect the basic and novel characteristic(s) of the subject disclosed herein. For example, a wnt synthekine "consisting essentially of" a disclosed sequence has the amino acid sequence of the disclosed sequence plus or minus about 5 amino acid residues at the boundaries of the sequence based upon the sequence from which it was derived, e.g. about 5 residues, 4 residues, 3 residues, 2 residues or about 1 residue less than the recited bounding amino acid residue, or about 1 residue, 2 residues, 3 residues, 4 residues, or 5 residues more than the recited bounding amino acid residue.

By "consisting of", it is meant the exclusion from the composition, method, or kit of any element, step, or ingredient not specified in the claim. For example, a wnt synthekine "consisting of" a disclosed sequence consists only of the disclosed amino acid sequence.

By "functional moiety" or "FM" it is meant a polypeptide, small molecule or nucleic acid composition that confers a functional activity upon a composition. Examples of functional moieties include, without limitation, therapeutic moieties, binding moieties, and imaging moieties.

By "therapeutic moiety", or "TM", it is meant a polypeptide, small molecule or nucleic acid composition that confers a therapeutic activity upon a composition. Examples of therapeutic moieties include cytotoxins, e.g. small molecule compounds, protein toxins, and radiosensitizing moieties, i.e. radionuclides etc. that are intrinsically detrimental to a cell; agents that alter the activity of a cell, e.g. small molecules, peptide mimetics, cytokines, chemokines; and moieties that target a cell for ADCC or CDC-dependent death, e.g. the Fc component of immunoglobulin.

By an "imaging moiety", or "IM", it is meant a non-cytotoxic agent that can be used to locate and, optionally, visualize cells, e.g. cells that have been targeted by compositions of the subject application.

The terms "treatment", "treating" and the like are used herein to generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease in a mammal, and includes: (a) preventing the disease from occurring in a subject which may be predisposed to the disease but has not yet been diagnosed as having it; (b) inhibiting the disease, i.e., arresting its development; or (c) relieving the disease, i.e., causing regression of the disease. The therapeutic agent may be administered before, during or after the onset of disease or injury. The treatment of ongoing disease, where the treatment stabilizes or reduces the undesirable clinical symptoms of the patient, is of particular interest. Such treatment is desirably performed prior to complete loss of function in the affected tissues. The subject therapy may be administered during the symptomatic stage of the disease, and in some cases after the symptomatic stage of the disease.

The terms "individual," "subject," "host," and "patient," are used interchangeably herein and refer to any mammalian subject for whom diagnosis, treatment, or therapy is desired, particularly humans.

General methods in molecular and cellular biochemistry can be found in such standard textbooks as Molecular Cloning: A Laboratory Manual, 3rd Ed. (Sambrook et al., CSH Laboratory Press 2001); Short Protocols in Molecular Biology, 4th Ed. (Ausubel et al. eds., John Wiley & Sons 1999); Protein Methods (Bollag et al., John Wiley & Sons 1996); Nonviral Vectors for Gene Therapy (Wagner et al. eds., Academic Press 1999); Viral Vectors (Kaplift & Loewy eds., Academic Press 1995); Immunology Methods Manual (I. Lefkovits ed., Academic Press 1997); and Cell and Tissue Culture: Laboratory Procedures in Biotechnology (Doyle & Griffiths, John Wiley & Sons 1998). Reagents, cloning vectors, and kits for genetic manipulation referred to in this disclosure are available from commercial vendors such as BioRad, Stratagene, Invitrogen, Sigma-Aldrich, and ClonTech.

Native receptor and ligand pairs include, without limitation, the following receptors:

| **Native ligands** | **Receptor subunits** |
|---|---|
| IL-1-like | |
| IL-1α | CD121a, CDw121b |
| IL-1β | CD121a, CDw121b |
| IL-1RA | CD121a |
| IL-18 | IL-18Rα, β |
| IL-2 | CD25, 122,132 |
| IL-4 | CD124,213a13, 132 |
| IL-7 | CD127, 132 |
| IL-9 | IL-9R, CD132 |
| IL-13 | CD213a1, 213a2, |
| IL-4 | CD124, 132 |
| IL-15 | IL-15Ra, CD122, 132 |
| IL-3 | CD123, CDw131 |
| IL-5 | CDw125, 131 |
| GM-CSF | CD116, CDw131 |
| IL-6 | CD126, 130 |
| IL-11 | IL-11Ra, CD130 |
| G-CSF | CD114 |
| IL-12 | CD212 |
| LIF | LIFR, CD130 |
| OSM | OSMR, CD130 |
| IL-10 | CDw210 |
| IL-20 | IL-20Rα, β |
| IL-14 | IL-14R |
| IL-16 | CD4 |
| IL-17 | CDw217 |
| IFN-α | CD118 |
| IFN-β | CD118 |
| IFN-γ | CDw119 |
| LT-β | LTβR |
| TNF-α | CD120a, b |
| TNF-β | CD120a, b |
| 4-1BBL | CDw137 (4-1BB) |
| APRIL | BCMA, TACI |
| CD70 | CD27 |
| CD153 | CD30 |
| CD178 | CD95 (Fas) |
| TALL-1 | BCMA, TACI |
| TGF-β1 | TGF-βR1 |
| TGF-β2 | TGF-βR2 |
| TGF-β3 | TGF-βR3 |
| Epo | EpoR |
| Tpo | TpoR |
| Flt-3L | Flt-3 |
| SCF | CD117 |
| M-CSF | CD115 |
| MSP | CDw136 |
| TNF, LTα | TNFR1; TNFRSF1A |
| TNF, LTα | TNFR2; TNFRSF1B; TNFRSF2 |
| 41-BB ligand; CD137 | 41-BB; TNFRSF9 |
| GITR ligand | AITR; TNFRSF18 |
| BAFF | BCMA; TNFRSF17 |
| CD27 ligand | CD27; TNFRSF7 |
| CD153 | CD30; TNFRSF8 |
| CD40 ligand, CD154 | CD40; TNFRSF5 |
| TRAIL | Death Receptor 1; TNFRSF10C |
| Apo3 ligand, TWEAK | Death Receptor-3; TNFRSF25 |
| TRAIL | Death Receptor 4; TNFRSF10A |
| TRAIL | Death Receptor 5; TNFRSF10B |
| App | Death Receptor -6; TNFRSF21 |
| Fas ligand, LIGHT | Decoy Receptor-3; TNFRSF6B |
| TRAIL | Decoy Receptor 2; TNFRSF10D |
| EDA | EDAR |
| Fas ligand | Fas; TNFRSF6 |
| LIGHT, LTα | HVEM; TNFRSF14 |
| LTα, LTβ, LIGHT | LTβ-R; TNFRSF3 |
| OX40 ligand | OX40; TNFRSF4 |
| RANKL | RANK; TNFRSF11A |
| APRIL, THANK | TACI; TNFRSF13B |
| LTα | Troy; TNFRSF19 |
| EDA | XEDAR; TNFRSF27 |
| RANKL | Osteoprotegerin; TNFRSF11B |
| TWEAK | TWEAK receptor; TNFRSF12A |
| BAFF | BAFF Receptor; TNFRSF13C |
| NGF, BDNF, NT-3, NT-4 | NGF receptor; TNFRSF16 |

Synthekines can be engineered to bind to any combination of receptor polypeptides in the table above, but generally do not activate the same combination or receptor polypeptides as a native ligand listed above. For example, while LIF activates a heterodimer of LIFR and cd130, a synthekine might activate LIFR and γc, or LIFR and βc, and the like. The combination of receptor polypeptides activated by a synthekine may be naturally expressed in a cell of interest, or the cell may be engineered to expression the desired combination of receptor polypeptides.

*JAK*/*STAT pathways and receptors.* Receptor that activate JAK/STAT pathways when dimerized include, without limitation, βc, γc, IL-3Rα, βIL-3R, GM-CSFRα, IL-5Rα, CNTFα, CRLF1, LIFRα, gp130, IL-6Rα, IL-11Rα, OSMRβ, IL-2Rα, IL-2Rβ, IL-2Ry, IL-4Rα, IL-7Rα, IL-9Rα, IL-13Rα, IL-15Rα, IL-21Rα, IFNAR2, IL-23R, EpoR, IL-12Rβ, IFNAR1, G-CSFR, c-MPLR.

The JAK-STAT signaling pathway transmits information from extracellular chemical signals to the nucleus resulting in transcription and expression of genes involved in immunity, proliferation, differentiation, apoptosis and oncogenesis. The JAK-STAT signaling cascade consists of three main components: a cell surface receptor as disclosed above, a Janus kinase (JAK) and two Signal Transducer and Activator of Transcription (STAT) proteins. Disrupted or dysregulated JAK-STAT functionality can result in immune deficiency syndromes and cancers.

Cytokine binding to a receptor on the cell surface leads to the activation of receptor-associated tyrosine kinases, the JAKs. Once activated, JAKs trans-phosphorylate each other, thereby creating docking sites for signal transducer and activator of transcription (STAT) molecules. Subsequent to binding, STATs become activated by JAK-mediated tyrosine phosphorylation and form homo- or heterodimers, translocate to the nucleus where they regulate transcription. Four distinct JAK kinases (JAK1, 2, 3, and TYK2) as well as seven different STAT proteins exist (STAT1, 2, 3, 4, 5A, 5B, and 6). One cytokine may activate more than one JAK and each JAK targets more than one STAT protein. This multilayered and complex activation pattern creates sometimes elaborate phenotypes. Review of Jak-STAT signaling include, for example, Villarino et al. (2017) Nat. Immunol. 18(4):374-384; Majoros et al. (2017) Front Immunol. 8:29; Bannarjee et al. (2017) Drugs 77(5):521-546' Pencik et al. (2016) Cytokine 87:26-36.

*Receptor tyrosine kinase pathways.* RTK receptor polypeptides include, without limitation, EGFR, ErbB2, ErbB3, ErbB4, InsR, IGF1R, InsRR, PDGFRα, PDGFRβ, CSF1R/Fms, cKit, Flt-3/Flk2, VEGFR1, VEGFR2, VEGFR3, FGFR1, FGFR2, FGFR3, FGFR4, PTK7/CCK4, TrkA, TrkB, TrkC, Ror1, Ror2, MuSK, Met, Ron, Axl, Mer, Tyro3, Tie1, Tie2, EphA1-8, EphA10, EphB1-4, EphB6, Ret, Ryk, DDR1, DDR2, Ros, LMR1, LMR2, LMR3, ALK, LTK, and SuRTK106/STYK1.

Receptor tyrosine kinases (RTKs) are the high-affinity cell surface receptors for many polypeptide growth factors, cytokines, and hormones. See, for example, Robinson et al. (2000). Oncogene 19(49):5548-57. Humans have 58 known RTKs, which fall into twenty subfamilies. All RTKs have a similar molecular architecture, with a ligand-binding region in the extracellular domain, a single transmembrane helix, and a cytoplasmic region that contains the protein tyrosine kinase (TK) domain plus additional carboxy (C-) terminal and juxtamembrane regulatory regions.

In general, growth factor binding activates RTKs by inducing receptor dimerization, although a subset of RTKs forms oligomers even in the absence of activating ligand. Ligand binding activates the receptor by stabilizing the individual receptor molecules in an active multimeric configuration. Typically one of the polypeptide chains then phosphorylates one or more tyrosines, and the phosphorylated receptor is active in assembling and activating intracellular signaling proteins. Ligand-induced dimerization of the extracellular regions of RTKs leads to activation of the intracellular tyrosine kinase domain (TKD).

The first and primary substrates that RTKs phosphorylate are the receptors themselves. Autophosphorylation sites in the kinase domain itself play an important regulatory role in most RTKs. Additional tyrosines are then autophosphorylated in other parts of the cytoplasmic region of most RTKs. The resulting phosphotyrosines function as specific sites for the assembly of downstream signaling molecules that are recruited to the receptor and activated in response to growth factor stimulation. Autophosphorylation occurs in trans, and autophosphorylation sites are phosphorylated in a precise order. Each successive event has a significant effect on catalytic properties by destabilizing cis-autoinhibitory interactions.

The cellular response to autophosphorylation of RTKs is the recruitment and activation of a host of downstream signaling molecules. These molecules contain SH2 or PTB domains that specifically bind to phosphotyrosine. They may be directly recruited to phosphotyrosines in the receptor, or they may be recruited indirectly by binding to docking proteins that are phosphorylated by RTKs with which they associate. These docking proteins include FRS2, IRS1 (insulin receptor substrate-1), and Gab1 (the Grb2-associated binder). Docking proteins typically contain a membrane targeting site at their amino terminus, followed by an array of tyrosine phosphorylation sites that serve as binding sites for a distinct repertoire of downstream signaling proteins. Although a number of docking proteins (such as Gab1) are recruited by multiple RTKs, others are restricted to particular subsets of receptors. With multiple phosphotyrosines in most receptors and the involvement of numerous docking proteins, activated RTKs can recruit and influence a large number of different signaling molecules. Review of signaling pathways include, for example, Lemmon and Schlesinger (2010) Cell 141(7):1117-34.

*TNFRSF Pathways.* TNFRSF polypeptides include, without limitation, TNFR1 (TNFRSF1A), TNFR2 (TNFRSF1B; TNFRSF2), 41-BB (TNFRSF9); AITR (TNFRSF18); BCMA (TNFRSF17), CD27 (TNFRSF7), CD30 (TNFRSF8), CD40 (TNFRSF5), Death Receptor 1 (TNFRSF10C), Death Receptor-3 (TNFRSF25), Death Receptor 4 (TNFRSF10A), Death Receptor 5 (TNFRSF10B), Death Receptor -6 (TNFRSF21), Decoy Receptor-3 (TNFRSF6B), Decoy Receptor 2 (TNFRSF10D), EDAR, Fas (TNFRSF6), HVEM (TNFRSF14), LTβ-R (TNFRSF3), OX40 (TNFRSF4), RANK (TNFRSF11A), TACI (TNFRSF13B), Troy (TNFRSF19), XEDAR (TNFRSF27), Osteoprotegerin (TNFRSF11B), TWEAK receptor (TNFRSF12A), BAFF Receptor (TNFRSF13C), NGF receptor (TNFRSF16).

The tumor necrosis factor receptor (TNFR) superfamily consists of 29 transmembrane receptors with significant homology in their extracellular domain, characterized by the presence of up to six cysteine-rich domains (CRD), which defines their ligand specificity. The members of this family are type-I transmembrane proteins with a C-terminal intracellular tail, a membrane-spanning region, and an extracellular ligand-binding N-terminal domain. Members of TNFRs contain an extracellular domain responsible for ligand binding and an intracellular domain that mediates activation of signaling pathway. The TNF homology domain (THD) triggers formation of non-covalent homotrimers. TNFRs may be divided into two groups: activating receptors and death receptors (DRs).

DRs include eight members, such as TNFR1 and Fas, which have a protein interaction module called the death domain (DD) in the intracellular region that mediates extrinsic signal-induced cell death. Binding to the ligand results in receptor aggregation and recruitment of adaptor proteins, which, in turn, initiates a proteolytic cascade by recruiting and activating initiator caspases 8 and 10. Death receptors initiate multiple signaling pathways, including regulation of cell proliferation and differentiation, chemokine production, inflammatory responses, apoptosis, and tumor-promoting activities.

Death receptors are activated by their cognate ligands, a group of complementary cytokines that belong to the TNF protein family. Cytotoxic signal transduction by death receptors proceeds through 1) binding to the cognate ligand; 2) recruitment of adaptor/docking proteins, which, in turn, recruit the initiator caspases 8 and 10; and 3) discrete signaling pathways depending on the stoichiometry of the various adaptor proteins and caspases 8 and 10, and cellular internalization events. Numerous noncytotoxic signaling pathways, mainly mediated by the activation of nuclear factor-κB (NF-κB) and mitogen-activated protein kinase (MAPK), from the receptor/adaptor protein complexes may also be involved.

Most TNF receptors require specific adaptor protein such as TRADD, TRAF, RIP and FADD for downstream signaling, and may ultimately act to activate NF-κB. For example, on binding with TNFα, the intracellular DD of TNFR1 recruits TNF receptor-associated DD protein (TRADD), which in turn recruits receptor-interacting protein kinase 1 (RIP1), cellular inhibitor of apoptosis proteins 1 and 2 (clAP1 and 2), and TNF receptor-associated factor 2. TRADD is important for the TNF-induced NF-κB signaling pathway, as in TRADD-deficient MEFs, IκB phosphorylation and degradation are completely abolished.

*Expression construct:* In the present methods, a synthekine may be produced by recombinant methods. The synthekine may be introduced on an expression vector into the cell to be engineered. DNA encoding a synthekine may be obtained from various sources as designed during the engineering process.

Amino acid sequence variants are prepared by introducing appropriate nucleotide changes into the coding sequence, as described herein. Such variants represent insertions, substitutions, and/or specified deletions of, residues as noted. Any combination of insertion, substitution, and/or specified deletion is made to arrive at the final construct, provided that the final construct possesses the desired biological activity as defined herein.

The nucleic acid encoding a synthekine is inserted into a replicable vector for expression. Many such vectors are available. The vector components generally include, but are not limited to, one or more of the following: an origin of replication, one or more marker genes, an enhancer element, a promoter, and a transcription termination sequence. Vectors include viral vectors, plasmid vectors, integrating vectors, and the like.

A synthekine may be produced recombinantly not only directly, but also as a fusion polypeptide with a heterologous polypeptide, e.g. a signal sequence or other polypeptide having a specific cleavage site at the N-terminus of the mature protein or polypeptide. In general, the signal sequence may be a component of the vector, or it may be a part of the coding sequence that is inserted into the vector. The heterologous signal sequence selected preferably is one that is recognized and processed (i.e., cleaved by a signal peptidase) by the host cell. In mammalian cell expression the native signal sequence may be used, or other mammalian signal sequences may be suitable, such as signal sequences from secreted polypeptides of the same or related species, as well as viral secretory leaders, for example, the herpes simplex gD signal.

Expression vectors usually contain a selection gene, also termed a selectable marker. This gene encodes a protein necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media.

Expression vectors will contain a promoter that is recognized by the host organism and is operably linked to a synthekine coding sequence. Promoters are untranslated sequences located upstream (5') to the start codon of a structural gene (generally within about 100 to 1000 bp) that control the transcription and translation of particular nucleic acid sequence to which they are operably linked. Such promoters typically fall into two classes, inducible and constitutive. Inducible promoters are promoters that initiate increased levels of transcription from DNA under their control in response to some change in culture conditions, e.g., the presence or absence of a nutrient or a change in temperature. A large number of promoters recognized by a variety of potential host cells are well known.

Transcription from vectors in mammalian host cells may be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus (such as murine stem cell virus), hepatitis-B virus and most preferably Simian Virus 40 (SV40), from heterologous mammalian promoters, e.g., the actin promoter, PGK (phosphoglycerate kinase), or an immunoglobulin promoter, from heat-shock promoters, provided such promoters are compatible with the host cell systems. The early and late promoters of the SV40 virus are conveniently obtained as an SV40 restriction fragment that also contains the SV40 viral origin of replication.

Transcription by higher eukaryotes is often increased by inserting an enhancer sequence into the vector. Enhancers are cis-acting elements of DNA, usually about from 10 to 300 bp, which act on a promoter to increase its transcription. Enhancers are relatively orientation and position independent, having been found 5' and 3' to the transcription unit, within an intron, as well as within the coding sequence itself. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin, the cytomegalovirus early promoter enhancer, the polyoma enhancer on the late side of the replication origin, and adenovirus enhancers. The enhancer may be spliced into the expression vector at a position 5' or 3' to the coding sequence, but is preferably located at a site 5' from the promoter.

Expression vectors used in eukaryotic host cells will also contain sequences necessary for the termination of transcription and for stabilizing the mRNA. Such sequences are commonly available from the 5' and, occasionally 3', untranslated regions of eukaryotic or viral DNAs or cDNAs. Construction of suitable vectors containing one or more of the above-listed components employs standard techniques.

Nucleic acids are "operably linked" when placed into a functional relationship with another nucleic acid sequence. For example, DNA for a signal sequence is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous.

Recombinantly produced sythekines can be recovered from the culture medium as a secreted polypeptide, although it can also be recovered from host cell lysates. A protease inhibitor, such as phenyl methyl sulfonyl fluoride (PMSF) also may be useful to inhibit proteolytic degradation during purification, and antibiotics may be included to prevent the growth of adventitious contaminants. Various purification steps are known in the art and find use, e.g. affinity chromatography. Affinity chromatography makes use of the highly specific binding sites usually present in biological macromolecules, separating molecules on their ability to bind a particular ligand. Covalent bonds attach the ligand to an insoluble, porous support medium in a manner that overtly presents the ligand to the protein sample, thereby using natural biospecific binding of one molecular species to separate and purify a second species from a mixture. Antibodies are commonly used in affinity chromatography. Size selection steps may also be used, e.g. gel filtration chromatography (also known as size-exclusion chromatography or molecular sieve chromatography) is used to separate proteins according to their size. In gel filtration, a protein solution is passed through a column that is packed with semipermeable porous resin. The semipermeable resin has a range of pore sizes that determines the size of proteins that can be separated with the column. Also of interest is cation exchange chromatography.

The final synthekine composition may be concentrated, filtered, dialyzed, *etc.,* using methods known in the art. For therapeutic applications, the synthekines can be administered to a mammal comprising the appropriate combination of receptor polypeptides. Administration may be intravenous, as a bolus or by continuous infusion over a period of time. Alternative routes of administration include intramuscular, intraperitoneal, intra-cerobrospinal, subcutaneous, intra-articular, intrasynovial, intrathecal, oral, topical, or inhalation routes. The synthekines also are suitably administered by intratumoral, peritumoral, intralesional, or perilesional routes or to the lymph, to exert local as well as systemic therapeutic effects.

Such dosage forms encompass physiologically acceptable carriers that are inherently non-toxic and non-therapeutic. Examples of such carriers include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and PEG. Carriers for topical or gel-based forms of polypeptides include polysaccharides such as sodium carboxymethylcellulose or methylcellulose, polyvinylpyrrolidone, polyacrylates, polyoxyethylene-polyoxypropylene-block polymers, PEG, and wood wax alcohols. For all administrations, conventional depot forms are suitably used. Such forms include, for example, microcapsules, nano-capsules, liposomes, plasters, inhalation forms, nose sprays, sublingual tablets, and sustained-release preparations. The polypeptide will typically be formulated in such vehicles at a concentration of about 0.1 µg/ml to 100 µg/ml.

In the event the synthekine is "substantially pure," they can be at least about 60% by weight (dry weight) the polypeptide of interest, for example, a polypeptide containing the synthekine amino acid sequence. For example, the polypeptide can be at least about 75%, about 80%, about 85%, about 90%,about 95% or about 99%, by weight, the polypeptide of interest. Purity can be measured by any appropriate standard method, for example, column chromatography, polyacrylamide gel electrophoresis, or HPLC analysis.

An article of manufacture containing materials useful for the treatment of the conditions described above is also dsiclosed herein. The article of manufacture comprises a container and a label. Suitable containers include, for example, bottles, vials, syringes, and test tubes. The containers may be formed from a variety of materials such as glass or plastic. The container holds a composition that is effective for treating the condition and may have a sterile access port (for example the container may be an intravenous solution bag or a vial having a stopper pierceable by a hypodermic injection needle). The active agent in the composition is the synthekine. The label on, or associated with, the container indicates that the composition is used for treating the condition of choice. Further container(s) may be provided with the article of manufacture which may hold, for example, a pharmaceutically-acceptable buffer, such as phosphate-buffered saline, Ringer's solution or dextrose solution. The article of manufacture may further include other materials desirable from a commercial and user standpoint, including other buffers, diluents, filters, needles, syringes, and package inserts with instructions for use.

The term "identity," as used herein in reference to polypeptide or DNA sequences, refers to the subunit sequence identity between two molecules. When a subunit position in both of the molecules is occupied by the same monomeric subunit (e.g., the same amino acid residue or nucleotide), then the molecules are identical at that position. The similarity between two amino acid or two nucleotide sequences is a direct function of the number of identical positions. In general, the sequences are aligned so that the highest order match is obtained. If necessary, identity can be calculated using published techniques and widely available computer programs, such as the GCS program package (Devereux et al., Nucleic Acids Res. 12:387, 1984), BLASTP, BLASTN, FASTA (Atschul et al., J. Molecular Biol. 215:403, 1990). Sequence identity can be measured using sequence analysis software such as the Sequence Analysis Software Package of the Genetics Computer Group at the University of Wisconsin Biotechnology Center (1710 University Avenue, Madison, Wis. 53705), with the default parameters thereof.

The term "polypeptide," "protein" or "peptide" refer to any chain of amino acid residues, regardless of its length or post-translational modification (e.g., glycosylation or phosphorylation).

By "protein variant" or "variant protein" or "variant polypeptide" herein is meant a protein that differs from a wild-type protein by virtue of at least one amino acid modification. The parent polypeptide may be a naturally occurring or wild-type (WT) polypeptide, or may be a modified version of a WT polypeptide. Variant polypeptide may refer to the polypeptide itself, a composition comprising the polypeptide, or the amino sequence that encodes it. Preferably, the variant polypeptide has at least one amino acid modification compared to the parent polypeptide, e.g. from about one to about ten amino acid modifications, and preferably from about one to about five amino acid modifications compared to the parent.

By "parent polypeptide", "parent protein", "precursor polypeptide", or "precursor protein" as used herein is meant an unmodified polypeptide that is subsequently modified to generate a variant. A parent polypeptide may be a wild-type (or native) polypeptide, or a variant or engineered version of a wild-type polypeptide. Parent polypeptide may refer to the polypeptide itself, compositions that comprise the parent polypeptide, or the amino acid sequence that encodes it.

By "wild type" or "WT" or "native" herein is meant an amino acid sequence or a nucleotide sequence that is found in nature, including allelic variations. A WT protein, polypeptide, antibody, immunoglobulin, IgG, etc. has an amino acid sequence or a nucleotide sequence that has not been intentionally modified.

The terms "recipient", "individual", "subject", "host", and "patient", are used interchangeably herein and refer to any mammalian subject for whom diagnosis, treatment, or therapy is desired, particularly humans. "Mammal" for purposes of treatment refers to any animal classified as a mammal, including humans, domestic and farm animals, and zoo, sports, or pet animals, such as dogs, horses, cats, cows, sheep, goats, pigs, etc. Preferably, the mammal is human.

As used herein, a "therapeutically effective amount" refers to that amount of the therapeutic agent, e.g. adoptive T cell and orthogonal cytokine combinations, sufficient to treat or manage a disease or disorder. A therapeutically effective amount may refer to the amount of therapeutic agent sufficient to delay or minimize the onset of disease, e.g., delay or minimize the spread of cancer, or the amount effect to decrease or increase signaling from a receptor of interest. A therapeutically effective amount may also refer to the amount of the therapeutic agent that provides a therapeutic benefit in the treatment or management of a disease. Further, a therapeutically effective amount with respect to a therapeutic agent disclosed herein means the amount of therapeutic agent alone, or in combination with other therapies, that provides a therapeutic benefit in the treatment or management of a disease.

As used herein, the terms "prevent", "preventing" and "prevention" refer to the prevention of the recurrence or onset of one or more symptoms of a disorder in a subject as result of the administration of a prophylactic or therapeutic agent.

As used herein, the term "in combination" refers to the use of more than one prophylactic and/or therapeutic agents. The use of the term "in combination" does not restrict the order in which prophylactic and/or therapeutic agents are administered to a subject with a disorder. A first prophylactic or therapeutic agent can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second prophylactic or therapeutic agent to a subject with a disorder.

### Compositions

Synthekines and methods for their use are provided. Sythekines result in a measurable increase in the level of signaling by the targeted pathway, e.g. Jak/STAT, ERK, AKT, NF-κB, etc., with the proviso that a different profile of signals are activated relative to a native ligand. These and other objects, advantages, and features of the invention will become apparent to those persons skilled in the art upon reading the details of the compositions and methods as more fully described below.

A synthekine molecule is defined by its physical and biological properties. Key features are that the synthekine specifically binds to one or more, usually 2 or more distinct extracellular domains of cell surface receptors, which receptors are characterized by being activated through ligand-induced multimerization, often ligand-induced dimerization, in many instances resulting in activation by trans-phosphorylation. Synthekines activate non-natural combinations of receptors, and generally do not activate receptor combinations activated by native, i.e. genomically encoded, ligands. Receptors of interest include receptors that activate JAK-STAT signaling, exemplified by cytokine receptors described herein; receptor tyrosine kinases, exemplified by cytokine and growth factor receptors, and TNF receptors.

A synthekine can be any molecule, e.g. protein or pharmaceutical that has the desired binding properties. Small molecules, which may be less than about 15 Kd, are of interest and can be developed through compound screening as described herein. Polypeptides are also of interest. In addition, certain synthekines may comprise both a polypeptide region or domain and a non'-polypeptide region or domain.

A synthekine can be a polypeptide, where binding domains for two different receptor extracellular domains are linked. A polypeptide synthekine may be a single chain, dimer, or higher order multimer. The binding domains may be directly joined, or may be separated by a linker, e.g. a polypeptide linker, or a non-peptidic linker, *etc.*

One or all of the binding domain(s) may comprise the binding domain of a native ligand, i.e. IL-1α, IL-1β, IL-1RA, IL-18, IL-2, IL-4, IL-7, IL-9, IL-13, IL-4, IL-15, IL-3, IL-5, GM-CSF, IL-6, IL-11, G-CSF, IL-12, LIF, OSM, IL-10, IL-20, IL-14, IL-16, IL-17, IFN-α, IFN-β, IFN-γ, LT-β, TNF-α, TNF-β, 4-1BBL, CD70, CD153, CD178, TGF-β1, TGF-β2, TGF-β3, Epo, Tpo, Flt-3L, SCF, M-CSF, MSP; where the binding domain does not activate the native receptor for the ligand. For example, a binding domain may comprise targeted amino acid substitutions that result in a lack of binding to one of the native receptor polypeptides, but not the other. Many such modified binding domains are known in the art, and can, for example, result in dominant negative mutations with respect to the native receptor configuration.

The binding domain may be an antibody, or a binding portion derived therefrom, that specifically binds to one chain of a receptor.

The term "specific binding" refers to that binding which occurs between such paired species as enzyme/substrate, receptor/ligand, antibody/antigen, and lectin/carbohydrate which may be mediated by covalent or non-covalent interactions or a combination of covalent and non-covalent interactions. When the interaction of the two species produces a non-covalently bound complex, the binding which occurs is typically electrostatic, hydrogen-bonding, or the result of lipophilic interactions. Accordingly, "specific binding" occurs between a paired species where there is interaction between the two which produces a bound complex having the characteristics of an antibody/antigen or ligand/receptor interaction. One may determine the biological activity of a wnt synthekine in a composition by determining the level of activity in a functional assay after *in vivo* administration, *e.g.* accelerating bone regeneration, enhancing stem cell proliferation, *etc.,* nuclear localization of β-catenin, increased transcription of wnt-responsive genes; *etc.*

Each binding domain may be a small molecule or a polypeptide, and can be selected from any domain that binds the desired receptor extracellular domain at high affinity, e.g. a K_{D} of at least about 1 x 10⁻⁷ M, at least about 1 x 10⁻⁸ M, at least about 1 x 10⁻⁹ M, at least about 1 x 10⁻¹⁰ M. Suitable binding domains include, without limitation, de novo designed binding proteins, antibody derived binding proteins, *e.g.* scFv, Fab, *etc.* and other portions of antibodies that specifically bind to one or more proteins; nanobody derived binding domains; knottin-based engineered scaffolds; and the like.

A binding domain may be affinity selected to enhance binding to a desired extracellular domain. Methods of affinity selection for this purpose may optionally utilize one or more rounds of selection by introducing targeted amino acid changes and generating a library of candidate coding sequences, transforming a population of cells with the candidate coding sequence, e.g. into yeast cells, selecting (for example using paramagnetic microbeads) for the desired specificity. Typically multiple rounds of selection will be performed, and the resulting vectors sequenced and used as the basis for protein engineering. For example, the binding domain, including without limitation a modified cytokine, an antibody or nanobody derived domain, an engineered protein, etc. can be selected to bind selectively to an extracellular domain of interest.

*Variants.* Binding domains may also include derivatives, variants, and biologically active fragments of polypeptides described above, e.g. variants of native ligands. A "variant" polypeptide means a biologically active polypeptide as defined below having less than 100% sequence identity with a provided sequence. Such variants include polypeptides comprising one or more amino acid modifications, e.g., insertions, deletions or substitutions, as compared to the provided sequence, e.g., wherein one or more amino acid residues are added at the N- or C-terminus of, or within, the native sequence; from about one to forty amino acid residues are deleted, and optionally substituted by one or more amino acid residues; and derivatives of the above polypeptides, wherein an amino acid residue has been covalently modified so that the resulting product has a non-naturally occurring amino acid. Ordinarily, a biologically active variant will have an amino acid sequence having at least about 90% amino acid sequence identity with a native sequence polypeptide, preferably at least about 95%, more preferably at least about 99%.

A "functional derivative" of a sequence is a compound having a qualitative biological property in common with an initial sequence. "Functional derivatives" include, but are not limited to, fragments of a sequence and derivatives of a sequence, provided that they have a biological activity in common. The term "derivative" encompasses both amino acid sequence variants of polypeptide and covalent modifications thereof.

Binding domains for use in the subject compositions and methods may be modified using ordinary molecular biological techniques and synthetic chemistry so as to improve their resistance to proteolytic degradation or to optimize solubility properties or to render them more suitable as a therapeutic agent. Analogs of such polypeptides include those containing residues other than naturally occurring L-amino acids, e.g. D-amino acids or non-naturally occurring synthetic amino acids. D-amino acids may be substituted for some or all of the amino acid residues.

A synthekine may be fused or bonded to an additional polypeptide sequence. Examples include immunoadhesins, which combine a synthekine with an immunoglobulin sequence particularly an Fc sequence, and epitope tagged polypeptides, which comprise a native inhibitors polypeptide or portion thereof fused to a "tag polypeptide". The tag polypeptide has enough residues to provide an epitope against which an antibody can be made, yet is short enough such that it does not interfere with biological activity of the native inhibitors polypeptide. Suitable tag polypeptides generally have at least six amino acid residues and usually between about 6-60 amino acid residues. The synthekine may also be fused or combined in a formulation, or co-administered with an agent that enhances activity, *e.g.* cytokines, growth factors, chemotherapeutic agents, immunosuppressants, *etc.*

*Linker.* The binding domains may be separated by a linker, e.g. a polypeptide linker, or a non-peptidic linker, *etc.* The amino acid linkers that join domains can play an important role in the structure and function of multi-domain proteins. There are numerous examples of proteins whose catalytic activity requires proper linker composition. In general, altering the length of linkers connecting domains has been shown to affect protein stability, folding rates and domain-domain orientation (see George and Hering (2003) Prot. Eng. 15:871-879). The length of the linker in the synthekine, and therefore the spacing between the binding domains, can be used to modulate the signal strength of the synthekine, and can be selected depending on the desired use of the synthekine. The enforced distance between binding domains of a synthekine can vary, but in certain embodiments may be less than about 100 angstroms, less than about 90 angstroms, less than about 80 angstroms, less than about 70 angstroms, less than about 60 angstroms, less than about 50 angstroms.

The linker may be a rigid linker, or a flexible linker. The linker moiety may be a peptide linker. In some cases, the peptide linker comprises 2 to 100 amino acids. In some cases, the peptide linker comprises 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 but no greater than 100 amino acids. In some cases, the peptide linker is between 5 to 75, 5 to 50, 5 to 25, 5 to 20, 5 to 15, 5 to 10 or 5 to 9 amino acids in length. Exemplary linkers include linear peptides having at least two amino acid residues such as Gly-Gly, Gly-Ala-Gly, Gly-Pro-Ala, Gly-Gly-Gly-Gly-Ser. Suitable linear peptides include poly glycine, polyserine, polyproline, polyalanine and oligopeptides consisting of alanyl and/or serinyl and/or prolinyl and/or glycyl amino acid residues. In some cases, the peptide linker comprises the amino acid sequence selected from the group consisting of Gly₉, Glu₉, Ser₉, Gly₅-Cys-Pro₂-Cys, (Gly₄-Ser)₃, Ser-Cys-Val-Pro-Leu-Met-Arg-Cys-Gly-Gly-Cys-Cys-Asn, Pro-Ser-Cys-Val-Pro-Leu-Met-Arg-Cys-Gly-Gly-Cys-Cys-Asn, Gly-Asp-Leu-Ile-Tyr-Arg-Asn-Gln-Lys, and Gly₉-Pro-Ser-Cys-Val-Pro-Leu-Met-Arg-Cys-Gly-Gly-Cys-Cys-Asn. In one case a linker comprises the amino acid sequence GSTSGSGKSSEGKG, or (GGGGS)n, where n is 1, 2, 3, 4, 5, etc.; however many such linkers are known and used in the art and may serve this purpose.

Synthekines can be provided in single-chain form, which means that the binding domains are linked by peptide bonds through a linker peptide. The binding domains may be individual peptides and can be joined through a non-peptidic linker.

Chemical groups that find use in linking binding domains include carbamate; amide (amine plus carboxylic acid); ester (alcohol plus carboxylic acid), thioether (haloalkane plus sulfhydryl; maleimide plus sulfhydryl), Schiff's base (amine plus aldehyde), urea (amine plus isocyanate), thiourea (amine plus isothiocyanate), sulfonamide (amine plus sulfonyl chloride), disulfide; hyrodrazone, lipids, and the like, as known in the art.

The linkage between binding domains may comprise spacers, e.g. alkyl spacers, which may be linear or branched, usually linear, and may include one or more unsaturated bonds; usually having from one to about 300 carbon atoms; more usually from about one to 25 carbon atoms; and may be from about three to 12 carbon atoms. Spacers of this type may also comprise heteroatoms or functional groups, including amines, ethers, phosphodiesters, and the like. Specific structures of interest include: (CH₂CH₂O)n where n is from 1 to about 12; (CH₂CH₂NH)n, where n is from 1 to about 12; [(CH₂)n(C=O)NH(CH₂)ₘ]_{z}, where n and m are from 1 to about 6, and z is from 1 to about 10; [(CH₂)nOPO₃(CH₂)ₘ]_{z} where n and m are from 1 to about 6, and z is from 1 to about 10. Such linkers may include polyethylene glycol, which may be linear or branched.

The binding domains may be joined through a homo- or heterobifunctional linker having a group at one end capable of forming a stable linkage to the hydrophilic head group, and a group at the opposite end capable of forming a stable linkage to the targeting moiety. Illustrative entities include: azidobenzoyl hydrazide, N-[4-(p-azidosalicylamino)butyl]-3'-[2'-pyridyldithio]propionamide), bis-sulfosuccinimidyl suberate, dimethyladipimidate, disuccinimidyltartrate, N-γ-maleimidobutyryloxysuccinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, NHS-PEG-MAL; succinimidyl 4-[N-maleimidomethyl]cyclohexane-1-carboxylate; 3-(2-pyridyldithio)propionic acid N-hydroxysuccinimide ester (SPDP); N, N'-(1,3-phenylene) bismaleimide; N, N'-ethylene-bis-(iodoacetamide); or 4-(N-maleimidomethyl)-cyclohexane-1-carboxylic acid N-hydroxysuccinimide ester (SMCC); m-maleimidobenzoyl-N-hydroxysuccinimide ester (MBS), and succinimide 4-(p-maleimidophenyl)butyrate (SMPB), an extended chain analog of MBS. The succinimidyl group of these cross-linkers reacts with a primary amine, and the thiol-reactive maleimide forms a covalent bond with the thiol of a cysteine residue.

Other reagents useful for this purpose include: p,p'-difluoro-m,m'-dinitrodiphenylsulfone (which forms irreversible cross-linkages with amino and phenolic groups); dimethyl adipimidate (which is specific for amino groups); phenol-1,4-disulfonylchloride (which reacts principally with amino groups); hexamethylenediisocyanate or diisothiocyanate, or azophenyl-p-diisocyanate (which reacts principally with amino groups); disdiazobenzidine (which reacts primarily with tyrosine and histidine); O-benzotriazolyloxy tetramethuluronium hexafluorophosphate (HATU), dicyclohexyl carbodiimde, bromo-tris (pyrrolidino) phosphonium bromide (PyBroP); N,N-dimethylamino pyridine (DMAP); 4-pyrrolidino pyridine; N-hydroxy benzotriazole; and the like. Homobifunctional cross-linking reagents include bismaleimidohexane ("BMH").

*Antibody:* As used herein, the term "antibody" refers to a polypeptide that includes canonical immunoglobulin sequence elements sufficient to confer specific binding to a particular target antigen. As is known in the art, intact antibodies as produced in nature are approximately 150 kD tetrameric agents comprised of two identical heavy chain polypeptides (about 50 kD each) and two identical light chain polypeptides (about 25 kD each) that associate with each other into what is commonly referred to as a "Y-shaped" structure. Each heavy chain is comprised of at least four domains (each about 110 amino acids long)- an amino-terminal variable (VH) domain (located at the tips of the Y structure), followed by three constant domains: CH1, CH2, and the carboxy-terminal CH3 (located at the base of the Y's stem). A short region, known as the "switch", connects the heavy chain variable and constant regions. The "hinge" connects CH2 and CH3 domains to the rest of the antibody. Two disulfide bonds in this hinge region connect the two heavy chain polypeptides to one another in an intact antibody. Each light chain is comprised of two domains - an amino-terminal variable (VL) domain, followed by a carboxy-terminal constant (CL) domain, separated from one another by another "switch". Intact antibody tetramers are comprised of two heavy chain-light chain dimers in which the heavy and light chains are linked to one another by a single disulfide bond; two other disulfide bonds connect the heavy chain hinge regions to one another, so that the dimers are connected to one another and the tetramer is formed. Naturally-produced antibodies are also glycosylated, typically on the CH2 domain. Each domain in a natural antibody has a structure characterized by an "immunoglobulin fold" formed from two beta sheets (e.g., 3-, 4-, or 5-stranded sheets) packed against each other in a compressed antiparallel beta barrel. Each variable domain contains three hypervariable loops known as "complement determining regions" (CDR1, CDR2, and CDR3) and four somewhat invariant "framework" regions (FR1, FR2, FR3, and FR4). When natural antibodies fold, the FR regions form the beta sheets that provide the structural framework for the domains, and the CDR loop regions from both the heavy and light chains are brought together in three-dimensional space so that they create a single hypervariable antigen binding site located at the tip of the Y structure.

The Fc region of naturally-occurring antibodies binds to elements of the complement system, and also to receptors on effector cells, including for example effector cells that mediate cytotoxicity. As is known in the art, affinity and/or other binding attributes of Fc regions for Fc receptors can be modulated through glycosylation or other modification. Antibodies produced and/or utilized in accordance with the present disclosure include glycosylated Fc domains, including Fc domains with modified or engineered such glycosylation.

Any polypeptide or complex of polypeptides that includes sufficient immunoglobulin domain sequences as found in natural antibodies can be referred to and/or used as an "antibody", whether such polypeptide is naturally produced (e.g., generated by an organism reacting to an antigen), or produced by recombinant engineering, chemical synthesis, or other artificial system or methodology. Antibody sequence elements may be humanized, primatized, chimeric, etc, as is known in the art.

Moreover, the term "antibody" as used herein, can refer (unless otherwise stated or clear from context) to any of the art-known or developed constructs or formats for utilizing antibody structural and functional features in alternative presentation. For example, an antibody utilized in accordance with the present disclosure may be in a format selected from, but not limited to, intact IgG, IgE and IgM, bi- or multi- specific antibodies (e.g., Zybodies^{®}, etc), single chain Fvs, Fabs, Small Modular ImmunoPharmaceuticals ("SMIPs^{™}"), single chain or Tandem diabodies (TandAb^{®}), VHHs, Anticalins^{®}, Nanobodies^{®}, minibodies, BiTE^{®}s, ankyrin repeat proteins or DARPINs^{®}, Avimers^{®}, a DART, a TCR-like antibody, Adnectins^{®}, Affilins^{®}, Trans-bodies^{®}, Affibodies^{®}, a TrimerX^{®}, MicroProteins, Fynomers^{®}, Centyrins^{®}, and a KALBITOR^{®}. An antibody may lack a covalent modification (e.g., attachment of a glycan) that it would have if produced naturally. An antibody may contain a covalent modification (e.g., attachment of a glycan, a payload [e.g., a detectable moiety, a therapeutic moiety, a catalytic moiety, etc], or other pendant group [e.g., poly-ethylene glycol, etc.]

The antibody agent may comprise a polypeptide whose amino acid sequence includes one or more structural elements recognized by those skilled in the art as a complementarity determining region (CDR) and/or comprise a polypeptide whose amino acid sequence includes at least one CDR (e.g., at least one heavy chain CDR and/or at least one light chain CDR) that is substantially identical to one found in a reference antibody. In some cases an included CDR is substantially identical to a reference CDR in that it is either identical in sequence or contains between 1-5 amino acid substitutions as compared with the reference CDR. In some cases an included CDR is substantially identical to a reference CDR in that it shows at least 85%, 86%, 87%, 88%, 89%, 90%, 91%, 92%, 93%, 94%, 95%, 96%, 97%, 98%, 99%, or 100% sequence identity with the reference CDR. In some cases an included CDR is substantially identical to a reference CDR in that it shows at least 96%, 96%, 97%, 98%, 99%, or 100% sequence identity with the reference CDR. In some cases an included CDR is substantially identical to a reference CDR in that at least one amino acid within the included CDR is deleted, added, or substituted as compared with the reference CDR but the included CDR has an amino acid sequence that is otherwise identical with that of the reference CDR. In some cases an included CDR is substantially identical to a reference CDR in that 1-5 amino acids within the included CDR are deleted, added, or substituted as compared with the reference CDR but the included CDR has an amino acid sequence that is otherwise identical to the reference CDR. In some cases an included CDR is substantially identical to a reference CDR in that at least one amino acid within the included CDR is substituted as compared with the reference CDR but the included CDR has an amino acid sequence that is otherwise identical with that of the reference CDR. In some cases an included CDR is substantially identical to a reference CDR in that 1-5 amino acids within the included CDR are deleted, added, or substituted as compared with the reference CDR but the included CDR has an amino acid sequence that is otherwise identical to the reference CDR. In some cases, an antibody agent is or comprises a polypeptide whose amino acid sequence includes structural elements recognized by those skilled in the art as an immunoglobulin variable domain. In some cases, an antibody agent is a polypeptide protein having a binding domain which is homologous or largely homologous to an immunoglobulin-binding domain.

*Small Molecule Compositions.* synthekines also include organic molecules, preferably small organic compounds having a molecular weight of more than 50 and less than about 20,000 daltons. Useful synthekines are identified by, for example, a screening assay in which molecules are assayed for high affinity binding to one or both of ECD of interest. A molecule can provide for a binding moiety that will be joined to another binding moiety, or joined to a binding domain as described above for polypeptide agents.

Candidate synthekines comprise functional groups necessary for structural interaction with receptor ECD, particularly hydrogen bonding, and typically include at least an amine, carbonyl, hydroxyl or carboxyl group, preferably at least two of the functional chemical groups. The candidate synthekines often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof.

Candidate synthekines are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs. Test agents can be obtained from libraries, such as natural product libraries or combinatorial libraries, for example. A number of different types of combinatorial libraries and methods for preparing such libraries have been described, including for example, PCT publications WO 93/06121, WO 95/12608, WO 95/35503, WO 94/08051 and WO 95/30642.

Where the screening assay is a binding assay, one or more of the molecules may be joined to a label, where the label can directly or indirectly provide a detectable signal. Various labels include radioisotopes, fluorescers, chemiluminescers, enzymes, specific binding molecules, particles, e.g. magnetic particles, and the like. Specific binding molecules include pairs, such as biotin and streptavidin, digoxin and antidigoxin, etc. For the specific binding members, the complementary member would normally be labeled with a molecule that provides for detection, in accordance with known procedures.

A variety of other reagents may be included in the screening assay. These include reagents like salts, neutral proteins, *e.g.* albumin, detergents, *etc.* that are used to facilitate optimal protein-protein binding and/or reduce non-specific or background interactions. Reagents that improve the efficiency of the assay, such as protease inhibitors, nuclease inhibitors, anti-microbial agents, etc. may be used. The mixture of components are added in any order that provides for the requisite binding. Incubations are performed at any suitable temperature, typically between 4 and 40° C. Incubation periods are selected for optimum activity, but may also be optimized to facilitate rapid high-throughput screening. Typically between 0.1 and 1 hours will be sufficient.

Preliminary screens can be conducted by screening for compounds capable of binding to receptor polypeptide(s) of interest. The binding assays usually involve contacting a recveptor ECD with one or more test compounds and allowing sufficient time for the protein and test compounds to form a binding complex. Any binding complexes formed can be detected using any of a number of established analytical techniques. Protein binding assays include, but are not limited to, methods that measure co-precipitation, co-migration on non-denaturing SDS-polyacrylamide gels, and co-migration on Western blots (see, e.g., Bennet, J. P. and Yamamura, H. I. (1985) "Neurotransmitter, Hormone or Drug Receptor Binding Methods," in Neurotransmitter Receptor Binding (Yamamura, H. I., et al., eds.), pp. 61-89.

Certain screening methods involve screening for a compound that modulates signaling activity. Such methods may involve conducting cell-based assays in which test compounds are contacted with one or more cells expressing and then detecting and an increase in expression of responsive genes, detecting changes in various adapter proteins, Jak, STAT(s), and the like.

The level of expression or activity can be compared to a baseline value. As indicated above, the baseline value can be a value for a control sample or a statistical value that is representative of expression levels for a control population. Expression levels can also be determined for cells that do not express a receptor, as a negative control. Such cells generally are otherwise substantially genetically the same as the test cells. Various controls can be conducted to ensure that an observed activity is authentic including running parallel reactions with cells that lack the reporter construct or by not contacting a cell harboring the reporter construct with test compound. Compounds can also be further validated as described below.

Compounds that are initially identified by any of the foregoing screening methods can be further tested to validate the apparent activity. The basic format of such methods involves administering a lead compound identified during an initial screen to an animal or in a cell culture model, that serves as a model for humans. The animal models utilized in validation studies generally are mammals. Specific examples of suitable animals include, but are not limited to, primates, mice, and rats.

Active test agents identified by the screening methods described herein can serve as lead compounds for the synthesis of analog compounds. Typically, the analog compounds are synthesized to have an electronic configuration and a molecular conformation similar to that of the lead compound. Identification of analog compounds can be performed through use of techniques such as self-consistent field (SCF) analysis, configuration interaction (CI) analysis, and normal mode dynamics analysis. Computer programs for implementing these techniques are available. See, e.g., Rein et al., (1989) Computer-Assisted Modeling of Receptor-Ligand Interactions (Alan Liss, New York).

### Pharmaceutical Compositions

For therapeutic applications, the synthekine is administered to a mammal, preferably a human, in a physiologically acceptable dosage form, including those that may be administered to a human intravenously as a bolus or by continuous infusion over a period of time. Alternative routes of administration include topical, intramuscular, intraperitoneal, intra-cerobrospinal, subcutaneous, intra-articular, intrasynovial, intrathecal, oral, topical, or inhalation routes. The synthekines also are suitably administered by intratumoral, peritumoral, intralesional, or perilesional routes or to the lymph, to exert local as well as systemic therapeutic effects.

Pharmaceutical compositions may also comprise combinations of the molecules disclosed herein with cells, including stem cells, progenitor cells, immune effector cells, and the like. In such combinations, cells can be pre-treated with a molecule disclosed herein prior to use, e.g. ex vivo treatment of immune effector cells with the synthekine; cells can be administered concomitantly with a molecule disclosed herein in a separate or combined formulation; cells can be provided to an individual prior to treatment with a molecule disclosed herein, and the like.

Pharmaceutical compositions can include, depending on the formulation desired, pharmaceutically-acceptable, non-toxic carriers of diluents, which are defined as vehicles commonly used to formulate pharmaceutical compositions for animal or human administration. The diluent is selected so as not to affect the biological activity of the combination. Examples of such diluents are distilled water, buffered water, physiological saline, PBS, Ringer's solution, dextrose solution, and Hank's solution. In addition, the pharmaceutical composition or formulation can include other carriers, adjuvants, or non-toxic, nontherapeutic, nonimmunogenic stabilizers, excipients and the like. The compositions can also include additional substances to approximate physiological conditions, such as pH adjusting and buffering agents, toxicity adjusting agents, wetting agents and detergents.

The composition can also include any of a variety of stabilizing agents, such as an antioxidant for example. When the pharmaceutical composition includes a polypeptide, the polypeptide can be complexed with various well-known compounds that enhance the in vivo stability of the polypeptide, or otherwise enhance its pharmacological properties (e.g., increase the half-life of the polypeptide, reduce its toxicity, enhance solubility or uptake). Examples of such modifications or complexing agents include sulfate, gluconate, citrate and phosphate. The polypeptides of a composition can also be complexed with molecules that enhance their in vivo attributes. Such molecules include, for example, carbohydrates, polyamines, amino acids, other peptides, ions (e.g., sodium, potassium, calcium, magnesium, manganese), and lipids.

Further guidance regarding formulations that are suitable for various types of administration can be found in Remington's Pharmaceutical Sciences, Mace Publishing Company, Philadelphia, Pa., 17th ed. (1985). For a brief review of methods for drug delivery, see, Langer, Science 249:1527-1533 (1990).

The pharmaceutical compositions can be administered for prophylactic and/or therapeutic treatments. Toxicity and therapeutic efficacy of the active ingredient can be determined according to standard pharmaceutical procedures in cell cultures and/or experimental animals, including, for example, determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀ Compounds that exhibit large therapeutic indices are preferred.

The data obtained from cell culture and/or animal studies can be used in formulating a range of dosages for humans. The dosage of the active ingredient typically lines within a range of circulating concentrations that include the ED₅₀ with low toxicity. The dosage can vary within this range depending upon the dosage form employed and the route of administration utilized.

For oral administration, the active ingredient can be administered in solid dosage forms, such as capsules, tablets, and powders, or in liquid dosage forms, such as elixirs, syrups, and suspensions. The active component(s) can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate. Examples of additional inactive ingredients that may be added to provide desirable color, taste, stability, buffering capacity, dispersion or other known desirable features are red iron oxide, silica gel, sodium lauryl sulfate, titanium dioxide, and edible white ink. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain coloring and flavoring to increase patient acceptance.

The active ingredient, alone or in combination with other suitable components, can be made into aerosol formulations (i.e., they can be "nebulized") to be administered via inhalation. Aerosol formulations can be placed into pressurized acceptable propellants, such as dichlorodifluoromethane, propane, nitrogen.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives.

The components used to formulate the pharmaceutical compositions are preferably of high purity and are substantially free of potentially harmful contaminants (e.g., at least National Food (NF) grade, generally at least analytical grade, and more typically at least pharmaceutical grade). Moreover, compositions intended for in vivo use are usually sterile. To the extent that a given compound must be synthesized prior to use, the resulting product is typically substantially free of any potentially toxic agents, particularly any endotoxins, which may be present during the synthesis or purification process. Compositions for parental administration are also sterile, substantially isotonic and made under GMP conditions.

The effective amount of a therapeutic composition to be given to a particular patient will depend on a variety of factors, several of which will be different from patient to patient. A formulation may be provided, for example, in a unit dose. A competent clinician will be able to determine an effective amount of a therapeutic agent to administer to a patient. Dosage of the synthekine will depend on the treatment, route of administration, the nature of the therapeutics, sensitivity of the disease to the therapeutics, etc. Utilizing LD₅₀ animal data, and other information available, a clinician can determine the maximum safe dose for an individual, depending on the route of administration. Compositions which are rapidly cleared from the body may be administered at higher doses, or in repeated doses, in order to maintain a therapeutic concentration. Utilizing ordinary skill, the competent clinician will be able to optimize the dosage of a particular therapeutic or imaging composition in the course of routine clinical trials. Typically the dosage will be 0.001 to 100 milligrams of agent per kilogram subject body weight.

The compositions can be administered to the subject in a series of more than one administration. For therapeutic compositions, regular periodic administration (e.g., every 2-3 days) will sometimes be required, or may be desirable to reduce toxicity. For therapeutic compositions which will be utilized in repeated-dose regimens, moieties which do not provoke immune responses are preferred.

An article of manufacture containing materials useful for the treatment of the conditions described herein is further disclosed. The article of manufacture comprises a container and a label. Suitable containers include, for example, bottles, vials, syringes, and test tubes. The containers may be formed from a variety of materials such as glass or plastic. The container holds a composition that is effective for treating the condition and may have a sterile access port (for example the container may be an intravenous solution bag or a vial having a stopper pierceable by a hypodermic injection needle). The active agent in the composition is the synthekine. The label on, or associated with, the container indicates that the composition is used for treating the condition of choice. Further container(s) may be provided with the article of manufacture which may hold, for example, a pharmaceutically-acceptable buffer, such as phosphate-buffered saline, Ringer's solution or dextrose solution. The article of manufacture may further include other materials desirable from a commercial and user standpoint, including other buffers, diluents, filters, needles, syringes, and package inserts with instructions for use.

As used herein, the term "therapeutically effective amount" means an amount that is sufficient, when administered to a population suffering from or susceptible to a disease, disorder, and/or condition in accordance with a therapeutic dosing regimen, to treat the disease, disorder, and/or condition. A therapeutically effective amount may be one that reduces the incidence and/or severity of, stabilizes one or more characteristics of, and/or delays onset of, one or more symptoms of the disease, disorder, and/or condition. Those of ordinary skill in the art will appreciate that the term "therapeutically effective amount" does not in fact require successful treatment be achieved in a particular individual. Rather, a therapeutically effective amount may be that amount that provides a particular desired pharmacological response in a significant number of subjects when administered to patients in need of such treatment.

For example,the term "therapeutically effective amount", may refer to an amount which, when administered to an individual in need thereof in the context of inventive therapy, will block, stabilize, attenuate, or reverse a disease process occurring in said individual.

### Methods of Use

The synthekines are useful for both prophylactic and therapeutic purposes. Thus, as used herein, the term "treating" is used to refer to both prevention of disease, and treatment of a pre-existing condition. In certain instances, prevention indicates inhibiting or delaying the onset of a disease or condition, in a patient identified as being at risk of developing the disease or condition. The treatment of ongoing disease, to stabilize or improve the clinical symptoms of the patient, is a particularly important benefit provided by the present disclosure. Such treatment is desirably performed prior to loss of function in the affected tissues; consequently, prophylactic therapeutic benefits are also important. Evidence of therapeutic effect may be any diminution in the severity of disease. The therapeutic effect can be measured in terms of clinical outcome or can be determined by immunological or biochemical tests. Patients for treatment may be mammals, e.g. primates, including humans, may be laboratory animals, e.g. rabbits, rats, mice, etc., particularly for evaluation of therapies, horses, dogs, cats, farm animals, etc.

The dosage of the therapeutic formulation, e.g., pharmaceutical composition, will vary widely, depending upon the nature of the condition, the frequency of administration, the manner of administration, the clearance of the agent from the host, and the like. In particular, the initial dose can be larger, followed by smaller maintenance doses. In certain cases, the dose can be administered as infrequently as weekly or biweekly, or more often fractionated into smaller doses and administered daily, semi-weekly, or otherwise as needed to maintain an effective dosage level.

In some cases, administration of the composition or formulation comprising the synthekine is performed by local administration. Local administration, as used herein, may refer to topical administration, but also refers to injection or other introduction into the body at a site of treatment. Examples of such administration include intramuscular injection, subcutaneous injection, intraperitoneal injection, and the like. In other cases, the composition or formulation comprising the synthekine is administered systemically, e.g., orally or intravenously. In one case, the composition of formulation comprising the synthekine is administered by infusion, e.g., continuous infusion over a period of time, e.g., 10 min, 20 min, 3 min, one hour, two hours, three hours, four hours, or greater.

In some cases, the compositions or formulations are administered on a short term basis, for example a single administration, or a series of administrations performed over, e.g. 1, 2, 3 or more days, up to 1 or 2 weeks, in order to obtain a rapid, significant increase in activity. The size of the dose administered must be determined by a physician and will depend on a number of factors, such as the nature and gravity of the disease, the age and state of health of the patient and the patient's tolerance to the drug itself.

In certain methods disclosed herein, an effective amount of a composition comprising a synthekine is provided to cells, e.g. by contacting the cell with an effective amount of that composition to achieve a desired effect, e.g. to enhance signaling, proliferation, *etc.* In particular, the contacting may occur in vitro, ex vivo or in vivo. In particular, the cells may be derived from or present within a subject in need or increased signaling.

In some methods disclosed herein, an effective amount of the subject composition is provided to enhance signaling in a cell. Biochemically speaking, an effective amount or effective dose of a synthekine is an amount to increase signaling in a cell by at least 30%, at least 40%, at least 50%, at least 60%, at least 70%, at least 80%, at least 90%, at least 95%, or by 100% relative to the signaling in the absence of the synthekine. The amount of modulation of a cell's activity can be determined by a number of ways known to one of ordinary skill in the art of biology.

In a clinical sense, an effective dose of a synthekine composition is the dose that, when administered to a subject for a suitable period of time, e.g., at least about one week, and maybe about two weeks, or more, up to a period of about 4 weeks, 8 weeks, or longer, will evidence an alteration in the symptoms associated with lack of signaling. In some cases, an effective dose may not only slow or halt the progression of the disease condition but may also induce the reversal of the condition. It will be understood by those of skill in the art that an initial dose may be administered for such periods of time, followed by maintenance doses, which, in some cases, will be at a reduced dosage.

The calculation of the effective amount or effective dose of synthekine composition to be administered is within the skill of one of ordinary skill in the art, and will be routine to those persons skilled in the art. Needless to say, the final amount to be administered will be dependent upon the route of administration and upon the nature of the disorder or condition that is to be treated.

Cells suitable for use in the subject methods are cells that comprise one or more receptors. The cells to be contacted may be *in vitro,* that is, in culture, or they may be *in vivo,* that is, in a subject. Cells may be from/in any organism, but are preferably from a mammal, including humans, domestic and farm animals, and zoo, laboratory or pet animals, such as dogs, cats, cattle, horses, sheep, pigs, goats, rabbits, rats, mice, frogs, zebrafish, fruit fly, worm, etc. Preferably, the mammal is human. Cells may be from any tissue. Cells may be frozen, or they may be fresh. They may be primary cells, or they may be cell lines. Often cells are primary cells used in vivo, or treated ex vivo prior to introduction into a recipient.

Cells *in vitro* may be contacted with a composition comprising a synthekine by any of a number of well-known methods in the art. For example, the composition may be provided to the cells in the media in which the subject cells are being cultured. Nucleic acids encoding the synthekine may be provided to the subject cells or to cells co-cultured with the subject cells on vectors under conditions that are well known in the art for promoting their uptake, for example electroporation, calcium chloride transfection, and lipofection. Alternatively, nucleic acids encoding the synthekine may be provided to the subject cells or to cells cocultured with the subject cells via a virus, i.e. the cells are contacted with viral particles comprising nucleic acids encoding the peptide synthekine polypeptide. Retroviruses, for example, lentiviruses, are particularly suitable to the methods disclosed herein, as they can be used to transfect non-dividing cells (see, for example, Uchida et al. (1998) P.N.A.S. 95(20):11939-44). Commonly used retroviral vectors are "defective", *i.e.* unable to produce viral proteins required for productive infection. Rather, replication of the vector requires growth in a packaging cell line.

Likewise, cells *in vivo* may be contacted with the subject synthekine compositions by any of a number of well-known methods in the art for the administration of peptides, small molecules, or nucleic acids to a subject. The synthekine composition can be incorporated into a variety of formulations or pharmaceutical compositions, which in some cases will be formulated in the absence of detergents, liposomes, *etc.,* as have been described for the formulation of full-length proteins.

In some cases, the compounds disclosed herein are administered for use in treating diseased or damaged tissue, for use in tissue regeneration and for use in cell growth and proliferation, and/or for use in tissue engineering. In particular, the present disclosure provides a wnt synthekine, or a composition comprising one or more synthekines for use in treating tissue loss or damage due to aging, trauma, infection, or other pathological conditions.

In Synthekines act on immune effector cells, and modulate immune responsiveness. For example, pathways involved in inflammatory disease may be targeted. Inflammation is a process whereby the immune system responds to infection or tissue damage. Inflammatory disease results from an activation of the immune system that causes illness, in the absence of infection or tissue damage, or at a response level that causes illness. Inflammatory disease includes autoimmune disease, which are any disease caused by immunity that becomes misdirected at healthy cells and/or tissues of the body. Autoimmune diseases are characterized by T and B lymphocytes that aberrantly target self-proteins, -polypeptides, -peptides, and/or other self-molecules causing injury and or malfunction of an organ, tissue, or cell-type within the body (for example, pancreas, brain, thyroid or gastrointestinal tract) to cause the clinical manifestations of the disease. Autoimmune diseases include diseases that affect specific tissues as well as diseases that can affect multiple tissues, which can depend, in part on whether the responses are directed to an antigen confined to a particular tissue or to an antigen that is widely distributed in the body.

The immune system employs a highly complex mechanism designed to generate responses to protect mammals against a variety of foreign pathogens while at the same time preventing responses against self-antigens. In addition to deciding whether to respond (antigen specificity), the immune system must also choose appropriate effector functions to deal with each pathogen (effector specificity). Inflammatory diseases of interest include, without limitation Secondary Progressive Multiple Sclerosis (SPMS); Primary Progressive Multiple Sclerosis (PPMS); Neuromyelitis Optica (NMO); Psoriasis; Systemic Lupus Erythematosis (SLE); Ulcerative Colitis; Crohn's Disease; Ankylosing Spondylitis (see, for example, Mei et al. (2011) Clin. Rheumatol. 30:269-273; type 1 (IDDM); Asthma; Chronic Obstructive Pulmonary Disorder (COPD); Chronic Hepatitis; Amyotrophic Lateral Sclerosis (ALS); Alzheimer's Disease (AD); Parkinson's Disease; Frontotemporal Lobar Degeneration (FTLD), atherosclerosis/cardiovascular disease, and obesity/metabolic syndrome.

In some cases, a synthekine activates an immune effector cell for the treatment of cancer, or activates a pathway for inducing death or reducing growth of cancer cells. The term "cancer", as used herein, refers to a variety of conditions caused by the abnormal, uncontrolled growth of cells. Cells capable of causing cancer, referred to as "cancer cells", possess characteristic properties such as uncontrolled proliferation, immortality, metastatic potential, rapid growth and proliferation rate, and/or certain typical morphological features. A cancer can be detected in any of a number of ways, including, but not limited to, detecting the presence of a tumor or tumors (e.g., by clinical or radiological means), examining cells within a tumor or from another biological sample (e.g., from a tissue biopsy), measuring blood markers indicative of cancer, and detecting a genotype indicative of a cancer. However, a negative result in one or more of the above detection methods does not necessarily indicate the absence of cancer, e.g., a patient who has exhibited a complete response to a cancer treatment may still have a cancer, as evidenced by a subsequent relapse.

The term "cancer" as used herein includes carcinomas, (e.g., carcinoma in situ, invasive carcinoma, metastatic carcinoma) and pre-malignant conditions, i.e. neomorphic changes independent of their histological origin. The term "cancer" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer and primary carcinomas are included.

Cancers and cancer cells that can be treated include, but are not limited to, hematological cancers, including leukemia, lymphoma and myeloma, and solid cancers, including for example tumors of the brain (glioblastomas, medulloblastoma, astrocytoma, oligodendroglioma, ependymomas), carcinomas, e.g. carcinoma of the lung, liver, thyroid, bone, adrenal, spleen, kidney, lymph node, small intestine, pancreas, colon, stomach, breast, endometrium, prostate, testicle, ovary, skin, head and neck, and esophagus.

Synthekines also have widespread applications in non-therapeutic methods, for example in vitro research methods. The synthekine may be administered directly to cells in vivo, administered to the patient orally, intravenously, or by other methods known in the art, or administered to ex vivo cells. In some cases where the synthekine of is administered to ex vivo cells, these cells may be transplanted into a patient before, after or during administration of the synthekine.

The invention now being fully described, it will be apparent to one of ordinary skill in the art that various changes and modifications can be made without departing from the spirit or scope of the invention.

### EXPERIMENTAL

### Synthekines: synthetic cytokine and growth factor agonists that compel signaling through non-natural receptor dimers

Cytokine and growth factor ligands typically signal through homo- or hetero- dimeric cell surface receptors via JAK/TYK, or RTK-mediated trans-phosphorylation. However, the number of such receptor pairings occurring in nature is limited to those driven by endogenous ligands encoded within our genome. We have engineered synthetic cytokines (synthekines) that drive formation of cytokine receptor pairings not formed by endogenous cytokines, and that activate distinct signaling programs. We show that a wide range of non-natural cytokine receptor hetero-dimers are competent to signal. We engineered synthekines that assembled IL-2Rβ/IL-4Rα or IL-4Rα/IFNAR2 receptor heterodimers that do not occur naturally, triggering signaling and functional responses distinct from those activated by the native IL-2, IL-4, and IFN cytokines. Furthermore, hybrid synthekine ligands that dimerized a JAK/STAT cytokine receptor with a receptor tyrosine kinase (RTK) also elicited a signaling output. Synthekines represent a new family of synthetic "orphan" ligands to exploit the full combinatorial scope of dimeric signaling receptors encoded within the human genome.

For JAK/STAT cytokine receptors, it has been shown previously in several systems, that genetically modified chimeric receptors in which the extracellular domain (ECD) of a cytokine receptor have been fused onto the intracellular domain (ICD) of an unrelated receptor activated signaling in a ligand-dependent manner. However, for this concept to be practically useful, soluble ligands that co-opt endogenous receptors and assemble non-natural dimers on unmodified cells and tissues are required. This could be accomplished by synthetic cytokines, or *synthekines* that drive formation of cytokine receptor pairs not formed by natural endogenous cytokines.

Synthekines can activate new signaling programs and elicit unique immunomodulatory activities compared to genome-encoded cytokines, providing an almost unlimited supply of ligands with new functions. Previous studies have reported the engineering of cytokine variants that promote new activities by genetically fusing two cytokines via a polypeptide linker, resulting in dimers of natural receptor signaling dimers. An important caveat to this approach is that the two connected cytokines are fully active on their own and lead to additive combinations of two natural cytokine signaling dimers. They can therefore activate signaling either in cells expressing only one pair of cognate receptors as well as in cells expressing the four receptor subunits engaged by the two cytokines, resulting in a mixed phenotype. Thus, the molecular basis for the differential activities exhibited by these linked ligands are unclear.

Engineering cytokine ligands, (synthekines) that dimerize two different cytokine receptors in a typical molecularly defined 1:1 stoichiometry on the surface of responsive cells presents an alternative approach that will lead to unique, rather than additive, signaling outputs. Forming a defined dimeric complex, like the one formed by genome encoded cytokines, allows precise mechanistic insight into the nature of the signaling complex eliciting the new signaling programs and activities engaged by these ligands. The signaling elicited by synthekines would be more uniform and targeted than that of linked cytokines, thus decreasing pleiotropy and potential toxicity resulting from off-target effects. The synthekine approach allows one to explore non-natural cytokine receptor pairs and to determine whether they activate signaling.

To explore the generality of this idea, we expressed and characterized a 10×10 matrix of chimeric cytokine receptor pairs using an orthogonal extracellular domain as a common dimerizing unit, fused to cytokine receptor intracellular domains, allowing for the evaluation of the signaling of 100 different cytokine receptor dimers. Most cytokine receptor pairs sampled in this chimeric receptor matrix activated signaling. In a second step, we genetically fused two antagonist versions of IL-2, IL-4, and interferon (IFN) with a polypeptide linker to engineer synthekines that dimerize non-natural cytokine receptor pairs on the cell surface. Stimulation of cell lines and peripheral blood mononuclear cells (PBMCs) with engineered synthekines revealed signaling and cellular signatures distinct from the parent cytokines.

We extended the synthekine concept to dimerize c-kit, a tyrosine kinase receptor, and thrombopoietin receptor (TpoR), a JAK/STAT cytokine receptor, which resulted in a measurable signaling output that qualitatively differed from that induced by their respective endogenous ligands. Our results serve as proof of concept that dimerization of non-natural JAK/STAT and RTK receptor pairs is a viable approach to generate new signaling programs and activities whose functional consequences can be explored as if they are newly discovered orphan cytokine ligands.

*Signal activation induced by chimeric cytokine receptors.* We first wished to determine the potential for JAK/STAT cross-talk between a large number of enforced non-natural cytokine receptor dimers (Figure 1A). We generated an array of chimeric receptors, in which the extracellular domains (ECDs) of the IL-1 receptors (IL-1R1 and IL-1R1Acp) were fused to the transmembrane (TM) and intracellular domains (ICDs) of ten different cytokine receptors, generating a 10·10 matrix of possible receptor pair combinations (Figure 1B). We used the IL-1 system for two reasons: 1) IL-1 binds with very high affinity to its receptors, which allows for signal activation even at low receptor expression levels; and 2) IL-1 does not signal via the canonical JAK/STAT pathway, eliminating background activity resulting from dimerization of endogenous IL-1 receptors. Jurkat cells, which express all JAKs and STATs except STAT4, were electroporated with the indicated combinations of chimeric receptors and analyzed for IL- 1R1 and IL-1R1Acp surface expression by flow cytometry (Figure 2D) and for IL-1-dependent signal activation by Western blot (Figure 2A and Figure 2E).

Although most IL-1 receptor combinations exhibited robust cell surface expression, very low levels of expression were detected for some receptor pairs, although this did not significantly affect the detection of signaling by IL-1 stimulation (Figure 2E). Binary heat maps depicting phosphorylation of six STATs are presented in Figure 2A. Red squares indicate signaling, black squares indicate no signaling. As expected, STAT2 and STAT4 were not activated by any receptor combination due to low STAT2 expression and lack of STAT4 expression in Jurkat cells (Figure 2A). STAT1 and STAT6 proteins were activated only by chimeric receptor pairs containing IFNAR2 and IL-4Rα respectively, consistent with the specific activation of these two STATs by IFNs and the IL-4 and IL-13 cytokines (Figure 2A).

In contrast, STAT3 and STAT5 proteins were activated by many chimeric receptor pair combinations, consonant with the more pleiotropic use of these two STATs by cytokines (Figure 2A). Although the majority of receptor pair combinations activated signaling, we also found receptor pairs that did not induce productive signaling despite robust surface expression (Figure 2D), such as IL-2Rβ homodimers and IL-13Rα1 homodimers (Figure 2A and Figure 2E). Overall our data show that most receptor dimers combinations tested activated STAT proteins, revealing the high plasticity of the cytokine-cytokine receptor system.

*Signal activation effected by JAK2*/*JAK3 cytokine receptor pairs.* One striking observation from our chimeric receptor study was that chimeric receptor combinations pairing JAK2 and JAK3, *(i.e.* erythropoietin receptor (EpoR)/yc and IL-23R/yc), were unable to activate signaling. Interestingly, the JAK2/JAK3 pairing is not found in nature, raising the question of whether lack of signal activation by this pair could result from steric clashes or incompatible geometries between these two kinase molecules that would prevent cross-activation.

To test this hypothesis we inserted alanine residues in the juxtamembrane domain of EpoR, which has been shown to modulate signaling by altering the register of the juxtamembrane region of the receptor. Specifically, between one and four alanines were inserted after R₂₅₁ on the EpoR ICD (Figure 2B). These EpoR mutants were fused to the IL-1R1 ECD and co-transfected with either IL-1R1Acp-IFNAR2 (positive control) or IL-1R1Acp-yc in Jurkat cells (Figure 2F). Insertion of one, three or four alanines in the juxtamembrane domain of EpoR did not affect its ability to signal when paired with IFNAR2, but insertion of two alanines, prevented signaling by this receptor pair (Figure 2C). Insertion of one or three alanines in the juxtamembrane domain of EpoR did not recover signaling by the EpoR/yc (JAK2/JAK3) receptor pair, insertion of four alanines marginally recovered signaling, and insertion of two alanines fully recovered signal activity by this receptor pair (Figure 2C).

These results suggest the existence of structural constraints between JAK2 and JAK3 that prevent these two kinases from triggering signaling in our chimeric receptor system. However, this experiment makes clear that varying the ligand-receptor geometry in non-natural receptors pairs using JAK2 and JAK3 is a viable option to recover signaling.

Previously we have shown that altering the dimer geometry of EpoR with synthekine ligands results in differential signaling outputs, so synthekines could also exploit this parameter. In addition to JAK2/JAK3 pairs, we observed other chimeric receptor pairs that were unable to activate signaling. We asked whether insertion of alanines in the juxtamembrane domain would recover signaling by these receptors as well. Insertion of alanines in the juxtamembrane domains of IL-2Rβ, IL-12Rβ, and EpoR did not recover signaling by the IL-2Rβ-IL-2Rβ, IL-12Rβ-IL-23R and EpoR-EpoR pairs (Figure 2G).

Strikingly, the IL-12Rβ-IL-23R and EpoR-EpoR pairs represent the receptor dimers engaged by IL-23 and EPO respectively. We think it is most likely that the IL-1 receptor extracellular orientation and proximity is not favorable for some natural and non-natural cytokine receptor pairs. This is a technical limitation of the chimeric receptor strategy we used and the lack of signaling for some of the pairs is not due to intrinsic inability for particular JAK/TYK/STAT combinations to function. The collective results from the chimeric IL-1 receptor experiments is that many, if not most, non-natural cytokine receptor pairs can signal through one or more STATs, but that certain pairs will have distinct dimer orientation and proximities necessary that will depend on the synthekine.

*Signal activation profiles induced by synthekines.* We wished to explore whether dimerization of non-natural cytokine receptor pairs by synthekines would activate signaling in unmodified cells (Figure 3A). We used a bi-specific strategy where we fused two cytokines together, each of which could only bind to one of its two receptors, thus creating a defined 1:1 receptor dimer. To implement this approach, we engineered antagonist, or "dominant negative (DN)" versions of IL-4, IL-2, and IFN that preserve binding to their high affinity receptor subunits (IL-4Rα for IL-4, IL- 2Rβ for IL-2, and IFNAR2 for IFN) but for which binding to their low affinity receptor subunits has been disrupted (IL-13Rα1 and yc for IL-4, yc for IL-2, and IFNAR1 for IFN). These "DN" cytokines function as high affinity binding modules devoid of signaling activity on their own. As anticipated, the wild type cytokines activated signaling in the Hut78 T cell line (Figure 3B), but the dominant negative mutants were unable to promote denoted Super-2 that has 200-fold enhanced affinity for the IL-2Rβ receptor subunit (Figure 3B).

We then genetically fused pairs of dominant negative cytokine mutants with a Gly₄/Ser linker to generate new ligands that induce formation of IL-2Rβ-IL-4Rα and IL-4Rα-IFNAR2 non-natural receptor dimers (Figure 3A). When added to unmodified cell lines, these bi-specific-DN synthekines activated signaling profiles that were qualitatively and quantitatively distinct from those induced by the parent cytokines (Figure 3D).

Stimulation of Hut78 cells with SY1 SL, which promotes dimerization of IFNAR2 and IL-4Rα, resulted in STAT5 and STAT6 activation (Figure 3D). Notably, lengthening the polypeptide linker connecting these two dominant negative proteins by 10 residues (SY1 LL) increased the signaling potency exhibited by this synthekine (Figure 3D). Stimulation of Hut78 cells with SY2 synthekine, which dimerizes IL-2Rβ and IL-4Rα, resulted in STAT1 activation and weaker STAT3, STAT5 and STAT6 activation (Figure 3D). In all instances, the synthekines elicited maximum responses (Emax) significantly lower than those activated by genome-encoded cytokines.

Importantly, the different signaling programs activated by the synthekines are not merely the result of additive effects from the two parental cytokines, as the signaling programs induced by adding pairs of parental cytokines simultaneously were dissimilar from those induced by the corresponding synthekines (Figure 3E). In order to determine if the signaling programs activated by the synthekines differed from those activated by IL-2, IL-4 and IFN, we studied the activation of 120 different signaling molecules by phospho-flow cytometry in the CD4⁺ T cell line Hut78 (Figure 4). Of the 120 molecules studied, twenty were activated by the ligands. The natural cytokine profiles were as expected: Super-2 strongly activated STAT5 and the PI3K pathways (Figure 4A and 4B); IL-4 stimulation robustly induced STAT6 and the PI3K pathway activation (Figure 4A and 4B); and IFN led to a strong activation of all STATs molecules (Figure 4A and 4B).

When Hut78 cells were stimulated with the different synthekines, we could detect novel signaling programs engaged by these engineered ligands. Stimulation with SY1 LL (hereon referred to as SY1) strongly activated STAT5 and STAT6, and also stimulated STAT1 and STAT3 to a lower extent (Figure 4A and 4B). In addition, this synthekine induced strong activation of the PI3K pathway *(i.e.* GSK3B, Akt, RPS6) (Figure 4A). SY2 stimulation resulted in an overall weaker signal than the other ligands, with preferential activation of STAT1 (Figure 4A and 4B). Moreover, the STAT activation ratios elicited by the cytokines and synthekines differed significantly, with SY1 exhibiting a STAT5/STAT6 preference and SY2 exhibiting a STAT1 preference (Figure 4C). Principal component analysis of the signaling programs elicited by genome-encoded cytokines and synthekines further confirm that synthekines activate distinct signaling programs and not only a subset of the original programs engaged by the parental cytokines (Figure 4D).

*Cellular and signaling signatures induced by synthekines.* We analyzed responses to synthekine treatment in 31 cell populations profiled from human PBMCs via mass cytometry (CyTOF) (Figure 5A and Figure 5C). The native cytokines behaved as anticipated: Super-2 strongly activated STAT5, and also, to a lesser extent, activated STAT1, STAT3, Erk, and S6R, exhibiting a clear T cell preference (Figure 5A); IL-4 stimulation resulted in potent activation of STAT6 and a homogenous signaling footprint for T cells and monocytes, in agreement with the ubiquitous expression of the IL-4Rα and yc receptor subunits (Figure 5A). Stimulation with IFN promoted strong activation of STAT5 and STAT6 and weaker activation of STAT1 and STAT3, in agreement with previous observations (Figure 5A). The IFN-induced STAT activation profile mapped into two different cell clusters, with T cells inducing stronger STAT5 and STAT6 activation and B cells and monocytes exhibiting strong STAT6 activation but weak STAT5 activation (Figure 5A). Cell signaling patterns elicited by synthekines diverged from those elicited by endogenous cytokines (Figure 5A). The SY1 synthekine induced strong STAT5 activation in T cells, but failed to activate signaling in NK, B cells, and monocytes (Figure 5A). The SY2 synthekine elicited weak signal activation of each signal effector studied in all cell populations, with a small bias towards STAT1 activation (Figure 5A).

*Cytokine secretion profiles induced by synthekines.* After ligand stimulation, secreted cytokine levels in the extracellular milieu are often used to define the nature of the immune response generated by a given cytokine. We studied the cytokine secretion signatures induced by synthekines versus native cytokines. PBMCs were stimulated with IL-2, IL-4, IFN or the synthekines and the levels of 63 different analytes were measured after 24 hours of stimulation via bead based immunoassay (Figure 5B). Super-2 and the two synthekines increased cytokine secretion, IFN had a neutral effect, and IL-4 reduced the amount of cytokine secreted by PBMCs (Figure 5B).

More detailed analysis of the data revealed that, as expected, stimulation of PBMCs with Super-2 promoted secretion of high levels of LIF, IL-13 and IFNy (Figure 5B). In addition, Super-2 resulted in secretion of IL-22, and CD40L by PBMCs (Figure 5B). Also consistent with previous reports, IFN stimulation induced secretion of IL-27, while IL-4 stimulation led to down-regulation of cytokines secreted by resting PBMCs, with IFNy being the most potently down-regulated cytokine (Figure 5B). Stimulation profiles for the two synthekines differed from those induced by native cytokines. SY1 stimulation induced secretion of many cytokines: IL-17F, IL-27, IL-13, IL17A, IFNy, BDNF, IL-23, FGFβ, PDGFBB, and ENA78, and SY2 stimulation led to marginal levels of Eotaxin, BDNF and PDGFBB secretion (Figure 5B).

*Synthekines dimerizing an RTK with a JAK*/*STAT receptor activate signaling.* JAK/STAT cytokine receptors represent only a subset transmembrane receptors that signal via dimerization-induced kinase activation., Receptor Tyrosine Kinases (RTKs) (e.g. EGFR [epidermal growth factor receptor], c-Kit, etc), represent another large family of dimeric cell-surface receptors that signal through trans-phosphorylation of their intracellular kinase domains. We wondered if we could extend the scope of synthekines to include molecules that would compel heterodimerization and activatation between a JAK/STAT cytokine receptor and an RTK.

To assess the possibility of JAK/STAT receptor cross-talk with an RTK, we fused the TM and ICD of epidermal growth factor receptor (EGFR) to the ECD of IL-1R1 and transfected this construct together with our battery of IL-1R1AcP-cytokine receptors ICDs in Jurkat cells (Figure 6A and 6B). All ten cytokine-receptor/EGFR pairs expressed on the surface of Jurkat cells (Figure 6F). Stimulation with IL-1 resulted in variable degrees of phosphorylation of EGFR and, to a much lesser extent, STAT3 and STAT5 proteins (Figure 6B), demonstrating that these cytokine and tyrosine kinase receptors are capable of trans-phosphorylation when compelled through enforced proximity. This is consistent with prior studies showing that examples exist of such cross-talk can occur on natural cells. However, a caveat to these chimeric receptor studies is that overexpression of kinase-linked receptors can lead to aberrant, artefactual phosphorylation events. Therefore we sought to enforce heterodimerization of JAK/STAT and RTK-mediated receptors normally expressed on natural cells, in the absence of overexpression, using synthekine ligands.

We created a synthekine to compel dimerization of cKit, a tyrosine kinase receptor, and thrombopoietin receptor (TpoR), a cytokine receptor. To create the synthekine bi-specific ligand, we identified sequences of antibodies that bind with high affinity to either cKit or TpoR ECD. We reformatted them as single-chain variable fragments (scFvs), and enforced their heterodimerization by fusing each with complementary acidic and basic leucine zippers (Figure 6C) and applying them to the acute megakaryoblastic leukemia Mo7e cells, which are known to express cKit and TpoR. The SY4 and SY5 synthekines induced modest phosphorylation of cKit in Mo7e cells over background, but only SY5 induced detectable phosphorylation of TpoR associated JAK2 over background, albeit very weakly (Figure 6D). SY5 induced measurable Erk activation (50% Emax compared to the native ligand, stem cell factor [SCF]) but only weak STAT5 activation, which is consistent with the apparent asymmetric activation of cKit over TpoR (Figure 6E). Indeed, inhibition of JAK2 using a JAK2 small molecule inhibitor resulted in loss of Erk activation by SY5, suggesting that the signaling program engaged by these synthekines relies, at least in part, on JAK2 activity (Figure 6G). Thus, there appears to be asymmetry in the efficiency of trans-phosphorylation within the TpoR/cKit heterodimer. We performed a high throughput phospho-flow cytometry-based study to analyze the signaling response of 120 signaling molecules in stimulated Mo7e cells.

As shown in Figure 7A, 54 of the 120 different signaling molecules were activated above a significance threshold by the different ligands. Interestingly the signaling signature elicited by SY5 appeared to evoke qualitatively different outputs than SCF, TPO or the combination of the two ligands; depending on the pathway effector studied(Figure 7B and C). Collectively the chimeric receptor and synthekine studies show that, although inefficient compared to their natural ligands, JAK/TYK and RTK mediated signaling receptors are capable of cross-talk, which is consistent with prior studies suggesting that JAK and the RTK components are capable of phosphorylating one another's natural substrates.

In this study, we expanded the scope of kinase-linked dimeric receptor signaling on natural cells using synthetic ligands that can be loosely analogized to "orphan" or "synthekine" cytokines. This approach can exploit the full combinatorial potential of JAK/TYK/STAT, and RTK signaling through receptor dimers. A compelling rationale for our exploring this approach is that, despite their immunotherapeutic potential, relatively few cytokines are useful clinically, due in large part to their pleiotropy and off-target effects. In recent years, cytokine variants have been engineered with more defined activities and reduced toxicity. However, an intrinsic limitation to this approach is that engineered cytokines exhibit a subset of activities within the bioactivity space occupied by the parental cytokine.

We have performed a series of proof-of-concept experiments to show that activation of distinct signaling programs and by extension, immune activities, can be accomplished through engineering of synthetic cytokines (synthekines) that dimerize non-natural cytokine receptor pairs. The high plasticity of cytokine receptor pairing can be exploited by synthekines to elicit new signaling activities, paving the way for 'designer' ligands to specifically target biological processes relevant to health and disease. Our data show that synthekines activate distinct, and novel signaling programs and induce secretion of new cytokine signatures by stimulated PBMCs.

First, while most synthekines elicit signals that partially resemble those of the parent cytokines, the ratio of activated STATs differs significantly between cytokines and synthekines. For instance, SY1 elicits a STAT6>STAT5>STAT1>STAT3 pattern instead of the STAT6>STAT1>STAT3>STAT5 pattern seen with IL-4 plus IFN, while SY2 elicits a STAT1>STAT6>STAT5>STAT3 pattern instead of the STAT6>STAT5>STAT3>STAT1 pattern seen with IL-4 plus IL-2. Changes in STAT activation ratios can alter cytokine-induced biological responses.

Second, given that synthekines induce dimerization of non-naturally occurring cytokine receptor pairs, they may also change the abundance of STAT heterodimers and induce formation of novel STAT heterodimer pairings, resulting in the induction of completely novel gene expression programs and activities. Synthekine biology may be tested in mouse systems and disease models.

The physiological effects of synthekines will be no less complex than natural cytokines, but knowing the activities of the parent receptor chains used to form the non-natural dimer could predict activities by the synthekine. For example, we expect that in some cases the physiological effects and disease applications could be similar or related to those of one of the parent receptor chains, while in other cases entirely distinct.

The synthekine design paradigm encompasses several critical considerations: 1) Selection of two cytokine receptor subunits simultaneously expressed in the same cell. Cellular response to cytokines is tightly regulated by surface expression patterns of cytokine receptor subunits. Thus, there are many cytokine receptor pair combinations that, although compatible with signaling, would not have in vivo relevancy due to the lack of a naturally occurring cell subset that simultaneously expresses the two receptors subunits. 2) Selection of the cytokine receptor subunit types to be dimerized by synthekines. From our chimeric receptor study, we infer that most cytokine receptor pair combinations will activate signaling to some extent. However, other parameters such as structural properties may influence the degree and nature of signaling activation. For example, cytokine receptors can be subdivided into two classes based on ICD length. Receptors with long ICDs often bind their ligands with high affinity, pair with JAK1 or JAK2, encode for STAT binding sites, and drive signal activation. In contrast, receptors with short ICDs often bind their ligands with lower affinity, pair with TYK2 or JAK3, and minimally contribute to STAT recruitment and activation.

Interestingly, many receptor pairs that did not activate signaling in our chimeric receptor study comprised short ICD receptors, suggesting that synthekines dimerizing two short ICD receptor subunits would elicit weaker and less diverse signal activation programs than those dimerizing long ICDs receptor subunits. In addition, our results show that synthekines dimerizing receptors from two different cell surface receptor families (specifically the cytokine receptor and the tyrosine kinase receptor families) can be generated.

A very important aspect of the synthekines we have engineered is that they dimerize cytokine receptor pairs in a defined 1:1 molecular entity, which enables clear attribution of the signaling pathway to the receptor dimer. Formation of a molecularly defined surface complex by an engineered ligand is vital for characterizing the signaling and phenotypic programs activated by these ligands, as well as for predicting potential toxicities resulting from off-target effects and/or cellular interactions. Previous attempts at engineering synthetic ligands with novel activities by linking fully functional cytokines generated ligands that could form multiple independently functioning receptor complexes ranging from dimers to tetramers depending on the abundance and relative ratios of the receptor subunits expressed by a given cell type. Although these ligands elicited new bioactivity programs, the heterogeneous nature of the complexes they form makes very difficult to assign signaling or activities signatures to a particular complex or to predict toxic side effects that could arise from systemic administration.

By contrast, our targeted approach of dimerizing surface receptors using dominant negative cytokine mutants allows us to interrogate the activity of specific dimer pairs. A consistent finding from our study was that the engineered synthekines were relatively inefficient at activating signaling compared to the parent genome-encoded cytokines; at best we could detect 60% of the signaling amplitude induced by IL-2, IL-4, or IFN. The synthekines that evoked signaling from the cKit/TpoR heterodimer exhibited even weaker activation properties.

One explanation for this observation is that the architecture of the cytokine-receptor complex is a determinant of signal potency. It is possible that the receptor binding topology induced by the engineered synthekines is suboptimal and that signaling strength can be improved by altering the construction of these molecules.

### MATERIAL AND METHODS

Protein expression and purification. Human IL-4, Super-2, IFN, dominant negative cytokines, and synthekines were expressed and purified using a baculovirus expression system, as described in (Laporte et al., 2005). The sequence for the Super-2 variant of IL-2 is provided in (Levin et al., 2012). The SY1 SL and LL synthekines were generated by genetically fusing the IL-4DN and IFNDN proteins via a single (SY1 SL) or double (SY1 LL) Gly₄Ser linker. The SY2 synthekine was generated by genetically fusing the IL-2DN and IL-4DN proteins via a Gly₄Ser linker. IL-4DN was generated by introducing the previously described R121D/Y124D mutations on site II, which disrupt binding to common gamma chain (Wenzel S et al The Lancet, 2007). IFNDN was generating by disrupting the binding to IFNAR1 by introducing the mutations F63A and R120E on the IFN-IFNAR1 binding interface. IL-2DN (also known as IL-2 RETR) was previously described in Mitra et al, Immunity, 2015. Single-chain variable fragments (scFvs) used for engineering SY3, SY4 and SY5 were analogously expressed and purified in the baculovirus system via transfer of their variable regions into the pAcGP67A vector (BD Biosciences) with an N-terminal gp67 signal peptide and a C-terminal hexahistidine tag. scFvs were expressed with the variable heavy (V_{H}) and variable light (V_{L}) chains separated by a twelve-amino acid (Gly₄Ser)₃ linker fused either to acidic or basic leucine zippers for dimerization. All proteins contained C-terminal hexahistidine tags and were isolated by nickel chromatography and further purified to >98% homogeneity by size exclusion chromatography on a Superdex 200 column (GE Healthcare), equilibrated in 10 mM HEPES (pH 7.3) and 150 mM NaCl.

Chimeric Receptors generation. In order to generate the 10x10 signaling matrix, the ICDs of the 10 different parental cytokine receptors were fused with the IL-1R1 and IL-1R1Acp ECDs. In the IL-1R1 ECD format, the nucleotide sequence encoding the HA-tag was inserted between the end of the native signal sequence and the first residue of the IL-1R1 ECD. Each ICD was fused to the 3' end of IL-1R1 sequence. The IL-1R1Acp chimeras were cloned in the same manner except the V5-tag was used. The boundaries of the mature proteins and transmembrane spans were delineated using the SignalP and TMHMM webservers. The DNA sequence used for IL-1R1 was codon optimized for expression in Homo sapiens as the organism (jcat.de) and synthesized (Integrated DNA Technologies). The chimeric receptors were cloned into the pcDNA3.1 + vector (Invitrogen) using the Nhel and Kpnl restriction sites (NEB).

Tissue culture. Jurkat cells were cultured in DMEM complete medium (DMEM medium supplemented with 10% FBS, 2 mM L-glutamine, and penicillin-streptomycin (Gibco)). Hut78 cells were cultured in RPMI complete medium (RPMI 1640 medium supplemented with 10% FBS, 2 mM L-glutamine, and penicillin-streptomycin (Gibco)). Mo7e cells were cultured in IMEM complete media (IMEM medium supplemented with 10% FBS, 2 mM L-glutamine, 10 nM GM-SCF and penicillin-streptomycin (Gibco)). Prior to stimulation, Mo7e cells were starved overnight in modified growth medium lacking FBS and GM-CSF. All cell lines were maintained at 37°C in a humidified atmosphere with 5% CO₂.

Hut78 and Mo7e intracellular signaling studies. Approximately 3·10⁵ Hut78 or Mo7e cells per well were placed in a 96-well plate, washed with PBSA buffer (phosphate-buffered saline (PBS) pH 7.2, 1% BSA), and resuspended in PBSA containing serial dilutions of the indicated ligands. Cells were stimulated for the prescribed time at 37°C and immediately fixed by addition of formaldehyde to 1.5% followed by incubation for 10 min at room temperature. Cells were then permeabilized with 100% ice-cold methanol for 30 min at 4°C. The fixed and permeabilized cells were washed twice with PBSA and incubated with fluorescently22 labeled detection antibodies diluted in PBSA for 1 hr at room temperature. pSTAT3, pSTAT5 and pSTAT6 antibodies were purchased from BD Biosciences. pSTAT1, pErk, pcKit, pEGFR antibodies were purchased from Cell Signaling Technology. Cells were then washed twice in PBSA buffer and mean fluorescence intensity (MFI) was quantified on an Accuri C6 flow cytometer. Dose-response curves were fitted to a logistic model and EC₅₀ values were computed in the GraphPad Prism data analysis software after subtraction of the MFI of unstimulated cells and normalization to the maximum signal intensity induced by wild-type cytokine stimulation.

Peripheral blood mononuclear cell (PBMC) isolation from human whole blood. Peripheral blood mononuclear cells (PBMCs) were isolated from human whole blood (Stanford Blood Bank) using a gradient of Ficoll-Paque Plus (GE Healthcare) according to the manufacturer's protocol. Freshly isolated PBMCs were used for both mass cytometry studies and bead-based immunoassays. Prior to stimulation, PBMCs were rested at 37°C, 5% CO₂ for 1 hr in RPMI complete medium.

Mass cytometry immune cell signaling analysis. This assay was performed in the Human Immune Monitoring Center at Stanford University. Freshly isolated PBMC were seeded in 96-well plates at 5·10⁵ cells per well and stimulated with serial dilutions of the indicated ligands in RPMI complete for 20 min at 37°C, 5% CO₂. Cells were then fixed via 10 min incubation in paraformaldehyde (1.5% final concentration) at room temperature. Cells were washed and resuspended in CyFACS buffer (PBS supplemented with 2% BSA, 2 mM EDTA, and 0.1% sodium azide) containing the metal-chelating polymer-labeled anti-surface antigen antibodies for 30 min at room temperature. Antibodies were labeled from purified unconjugated, carrier protein-free stocks from BD Biosciences, Biolegend, or Cell Signaling Technology and the polymer and metal isotopes were from DVS Sciences. Cells were washed once in CyFACS buffer and then permeabilized overnight in methanol at -80°C. The following day, cells were washed once in CyFACS buffer and resuspended in CyFACS buffer containing the metal-chelating polymer-labeled anti-intracellular antigen antibodies for 30 min at room temperature. Cells were washed twice in PBS (phosphate-buffered saline pH 7.2), resuspended in iridium-containing DNA intercalator (1:200 dilution in PBS, DVS Sciences) and incubated on ice for 20 min. The cells were then washed three times in MilliQ water and then diluted in a total volume of 700 µL in MilliQ water before injection into the CyTOF instrument (DVS Sciences). Data analysis was performed using FlowJo (CyTOF settings) by gating on cells based on the iridium isotopes from the intercalator, then on intact singlets based on plots of one intercalator iridium isotope vs. cell length, followed by cell subset-specific gating. Signal intensity for each condition is reported as 90ₜₕ percentile intensity minus that of an unstimulated control sample. Heat maps of the response to the maximum concentration of each treatment were generated using the TM4 microarray software suite (Dana- Farber Cancer Institute) (Saeed et al., 2003), with signal intensities normalized to the maximum signal effector response in each cell type. Two independent replicates of mass cytometry experiments were performed with similar results obtained.

Bead-based immunoassay cytokine secretion studies. This assay was performed in the Human Immune Monitoring Center at Stanford University. Freshly isolated PBMCs (1.5·10₅ per well) were stimulated with the indicated ligands in the presence of 1 g/mL phytohaemagglutinin (PHA) in RPMI complete and incubated for 24 hr at 37°C, 5% CO₂. Cells were then pelleted via centrifugation and supernatants were harvested for bead-based immunoassay analysis using the LUMINEX^{®} platform (Luminex Corporation). Human 63-plex kits were purchased from eBiosciences/Affymetrix and used according to the manufacturer's recommendations with modifications as described below. Briefly: Beads were added to a 96 well plate and washed in a Biotek ELx405 washer. Harvested supernatants were added to the plate containing the mixed antibody-linked beads and incubated at room temperature for 1 hour followed by overnight incubation at 4°C with shaking. Cold and room temperature incubation steps were performed on an orbital shaker at 500-600 rpm. Following the overnight incubation, plates were washed in a Biotek ELx405 washer and biotinylated detection antibody was added for 75 minutes at room temperature with shaking. Plates were washed again as above and streptavidin-PE (Invitrogen) was added. After incubation for 30 minutes at room temperature, a final wash was performed as above and reading buffer was added to the wells. Plates were analyzed on a LUMINEX^{®} 200 instrument with a lower bound of 50 beads per sample per cytokine. Custom assay Control beads (Radix Biosolutions) were added to all wells. Each sample was measured in duplicate and raw MFI was averaged from the two replicates. Results are presented as fold change in MFI of treated cells relative to control cells stimulated with PHA only.

Primity Bio Pathway Phenotyping. Hut78 or Mo7e cells were stimulated with saturating concentrations of the indicated ligands for 15, 60 and 120 min and fixed with 1% PFA for 10 min at room temp. The fixed cells were prepared for antibody staining according to standard protocols (Krutzik and Nolan, 2003). Briefly, the fixed cells were permeabilized in 90% methanol for 15 minutes. The cells were then stained with a panel of antibodies specific to the markers indicated (Primity Bio Pathway Phenotyping service) and analyzed on an LSRII flow cytometer (Becton Dickinson, San Jose, CA). The log₂ ratio of the MFI of the stimulated samples divided by the unstimulated control samples were calculated as a measure of response.

Western blot analysis. Cells were lysed in 1% NP-40 lysis buffer and 30 µg protein was analyzed as described in (Marijanovic et al., 2007). The following polyclonal antibodies were used: anti-phospho Tyk2, anti-phospho STAT1; anti-phospho STAT2; anti-phospho STAT3; anti-phospho STAT4; anti-phospho STAT5; anti-phospho STAT6; anti-phospho EGFR; anti-phospho cKit pY703 (Cell Signaling Technology, Beverly, MA). Signal was revealed with the ECL enhanced chemiluminescence Western blotting reagent Western Lightning Chemiluminescence Reagent Plus (PerkinElmer).

Electroporation. 15-20x10⁶ Jurkat cells maintained at densities between 0.5-1.0·10⁶ cells/ml were washed twice with RPMI medium (sterile) and resuspended in 0.25 ml of Ingenio electroporation solution (Mirusbio). 5-30 µg DNA (not exceeding 15% of the total volume) was added to the resuspended Jurkat cells and the mixture was transferred to a 4 mm gap cuvette (Invitrogen) and incubated for 15-20 min room temperature. Cells were electroporated in a Biorad electroporator set at 0.28 kV and 960 µF. After electroporation, cells were transferred to prewarmed media (without Pen/Strep) and cultured normally. Protein expression was monitored after 24 hr.

Cell surface receptor staining. Surface receptor levels were monitored as described in (Marijanovic et al., 2007), using fluorescently-labeled monoclonal antibodies specific for the HA and V5 tags (Cell Signaling Technology). Electroporated Jurkat cells were resuspended in cold PBS containing 3% fetal calf serum and incubated with the indicated antibodies for 1hr. Samples were analyzed on an Accuri C6 flow cytometer (BD Biosciences).

### Example 2

### Trimeric Synthekines

Using a Gly/Ser polypeptide linker, a cytokine (IL-2) was linked with a scFv (monovalent antibody) that bound IL-4Rα. This new molecular entity bound to 3 cytokine receptor polypeptides: γc, IL-2Rβ, and IL-4Rα. The trimeric synthekine elicited signaling signatures different from those activated by either IL-2, IL-4 or a combination of IL-2 and IL-4 treatment. Additionally, this new cytokine induced the differentiation of monocytes into an uncharacterised subset of dendritic cells with high phagocytic activity, shown in Figure 8-15. In some embodiments, a composition of the novel synthekine is provided. In some embodiments a population of monocytes is contacted in vitro or in vivo with an effective dose of the trimeric synthekine. In some embodiments a phagocytic cell population differentiated from monocytes with the trimeric synthekine is provided.

### Example 3

### Synthekine that dimerizes type I and type III IFN receptors.

A synthekine (SY6) comprising an IFNλR1 binding sequence (H11DN), and an IFNAR1 binding sequence (IFNWDN2) was generated. The complete synthekine sequence is provided in SEQ ID NO:1. The synthekine thus generated is a hybrid Interferon that dimerizes IFNAR1 and IFNλR1 receptors and their respective JAKs. Shown in Figure 15, the Emax of phospho-STAT1 activation by SY6 is equal to that of type I IFNs, and twice the signal induced by type III IFNs. Error bars represent ± SEM (n = 3). Importantly, as shown in Figure 15D, SY6 potently induces the anti-proliferative effect, whereas type I IFN, type III IFN or a combination type I and III IFN treatment is ineffective. Error bars represent ± SEM (n = 3). Phospho-STAT1 signaling and anti-proliferative assays were performed in Hap1 cells which are naturally responsive to both type I and type III IFNs.

It is also important to note that the combination of type I and type III interferons does not provide this activity unless linked in a hybrid polypeptide such as SY6. The activity and specificity of the synthekine provides a potent agent for anti-proliferative and anti-viral activity, which provides selectivity of action and thus avoids undesirable side effects of Type I interferons.

### Example 4

### mIL4DN-mIFNβDN2 synthekine

This synthekine (SY7) was generated by genetically fusing mouse IL-4DN and mIFNβDN2 proteins via a Gly₄Ser linker. The sequence is as shown in Figure 16. Lymphocytes were isolated from spleen/LNs of C57BL/6 mice, and activated with plate-bound anti-CD3 (2.5 µg/ml) + soluble anti-CD28 (5 µg/ml) for 48H. Cells were then rested O/N in 10 IU/ml mIL2, then serum-starved for 4H prior to stimulation with indicated cytokine/synthekine for 20'. Cell signaling terminated and cells fixed with PFA, permeabilized with Permlll buffer (BD) and stained with phosphoSTAT6(Y641) antibody (BD).

**Table 1**

| Experimentally Generated Synthekines | | | |
|---|---|---|---|
| Name | Ligand 1 | Linker | Ligand 2 |
| SY1 SL | hIL-4DN | Gly/Ser | hIFNDN |
| SY1 LL | hIL-4DN | (Gly/Ser)2 | hIFNDN |
| SY2 | hIL-2DN | Gly/Ser | hIL-4DN |
| SY3 | anti-hIL4Rα ScFv | Gly/Ser | hIL-2 |
| SY4 | anti-cKit ScFv | | anti-TpoR ScFv |
| SY5 | anti-cKit ScFv | | anti-TpoR ScFv |
| SY6 | IFNλR1 binding sequence (H11DN) | Gly/Ser | IFNAR1 binding sequence (IFNWDN2) |
| SY7 | mIL-4DN | Gly/Ser | mIFNβDN2 |

Antagonist, or "dominant negative (DN)" versions of IL-4, IL-2, and IFN were engineered that preserve binding to their high affinity receptor subunits (IL-4Rα for IL-4, IL- 2Rβ for IL-2, and IFNAR2 for IFN) but for which binding to their low affinity receptor subunits has been disrupted (IL-13Rα1 and yc for IL-4, yc for IL-2, and IFNAR1 for IFN). These "DN" cytokines function as high affinity binding modules devoid of signaling activity on their own. Pairs of dominant negative cytokine mutants were fused with a Gly₄/Ser linker to generate new ligands that induce formation of IL-2Rβ-IL-4Rα and IL-4Rα-IFNAR2 non-natural receptor dimers, as indicated in the table above for SY1 and SY2. IL-4DN was generated by introducing the previously described R121D/Y124D mutations on site II, which disrupt binding to common gamma chain (Wenzel S et al The Lancet, 2007). IFNDN was generating by disrupting the binding to IFNAR1 by introducing the mutations F63A and R120E on the IFN-IFNAR1 binding interface. IL-2DN (also known as IL-2 RETR) was previously described in Mitra et al, Immunity, 2015.

Single-chain variable fragments (scFvs) used for engineering SY3, SY4 and SY5 were expressed with the variable heavy (V_{H}) and variable light (V_{L}) chains separated by a twelve-amino acid (Gly₄Ser)₃ linker fused either to acidic or basic leucine zippers for dimerization. The SY4 and SY5 constructs utilized antibodies that bind with high affinity to either cKit or TpoR ECD. We reformatted them as single-chain variable fragments (scFvs), and enforced their heterodimerization by fusing each with complementary acidic and basic leucine zippers.

The SY3 protein uses a Gly/Ser polypeptide linker to link a cytokine (IL-2) with a scFv (monovalent antibody) that bound IL-4Rα. This new molecular entity bound to 3 cytokine receptor polypeptides: yc, IL-2Rβ, and IL-4Rα.

The SY6 sequence is provided as SEQ ID NO:1, comprises from residues 1-163 a variant form of human IFNλ, with amino acid substitutions at the residues corresponding to Q26A, Q99A, H102A, H131R, T161A, and V174E of a reference human IFNλ3 sequence (Genbank reference XP_005258822.1) where the variant sequence is truncated truncated by deletion of residues 1-11 of the reference IFNλ3 protein. Residues 164-168 are a gly/ser linkers, and residues 169-342 comprise a variant form of human IFNω.

The SY7 sequence is provided as SEQ ID NO:2, where residues 1-17 are a signal peptide, residues 18-138 are mIL-4DN, with amino acid substitutions at the residues corresponding to Q116D and Y119D, as shown in Figure 17; residues 139-143 are a gly/ser linker; and residues 144-304 correspond to mIFNβDN2, with amino acid substitutions at the residues corresponding to R15A, L30A, R33A, R147A.

## Claims

1. A synthetic ligand comprising:
at least a first and a second binding domain, wherein each binding domain of the synthetic ligand specifically binds to the extracellular domain of a cytokine receptor polypeptide subunit expressed on the surface of a cell, wherein:
the first binding domain specifically binds to the extracellular domain of a first cytokine receptor subunit, wherein the first cytokine receptor subunit has an intracellular domain comprising at least one JAK/STAT binding sequence; and
the second binding domain specifically binds to the extracellular domain of a second cytokine receptor subunit, wherein the second cytokine receptor subunit has an intracellular domain comprising at least one JAK/STAT binding sequence,
wherein the first and second cytokine receptor subunits do not naturally multimerize in response to contacting the cell with a naturally occurring cytokine ligand;
wherein contacting the cell expressing the first and second cytokine receptor subunits with the synthetic ligand results in multimerization of the cytokine receptor subunits and activation of a JAK/STAT-mediated signal in the cell; and
wherein each binding domain is an antibody derived binding protein.

2. The synthetic ligand of claim 1, where the cytokine receptors are selected from interleukin 3 receptor alpha (IL-3Rα), beta interleukin 3 receptor (βIL-3R), granulocyte macrophage colony stimulating factor receptor (GM-CSFRα), interleukin 5 receptor alpha (IL-5Rα), ciliary neurotrophic receptor alpha (CNTFRα), cytokine receptor-like factor-1 (CRLF1), leukemia inhibitory factor receptor alpha (LIFRα), glycoprotein 130 (gp130), interleukin 6 receptor alpha (IL-6Rα), interleukin 11 receptor alpha (IL-11Rα), oncostatin M receptor beta subunit (OSMRβ), interleukin 2 receptor alpha (IL-2Rα), interleukin 2 receptor beta (IL-2Rβ), interleukin 2 receptor gamma (IL-2Rγ), interleukin 4 receptor alpha (IL-4Rα), interleukin 7 receptor alpha (IL-7Rα), interleukin 9 receptor alpha (IL-9Rα), interleukin 13 receptor alpha (IL-13Rα), interleukin 15 receptor alpha (IL-15Rα), interleukin 21 receptor alpha (IL-21Rα), interferon alpha receptor 2 (IFNAR2), interleukin 23 receptor (IL-23R), erythropoietin receptor (EpoR), interleukin 12 receptor beta (IL-12Rβ), interferon-alpha/beta receptor alpha chain (IFNAR1), interferon-alpha/beta receptor beta chain (IFNAR2), granulocyte colony stimulating factor receptor (G-CSFR), cluster of differentiation w210 (CDw210), interleukin 10 receptor beta (IL10Rβ), cluster of differentiation 212 (CD212), cluster of differentiation w119 (CDw119), and myeloproliferative leukemia protein receptor (c-MPLR).

3. The synthetic ligand of claim 1 or claim 2 wherein an antibody derived binding protein comprises a scFv.

4. The synthetic ligand of claim 3 wherein each antibody derived binding protein comprises a scFv.

5. The synthetic ligand of claim 1 or claim 2 wherein an antibody derived binding protein comprises a VHH.

6. The synthetic ligand of claim 5 wherein each antibody derived binding protein comprises a VHH.

7. The synthetic ligand of any one of claims 1 to 6 wherein the binding affinity of each antibody derived binding protein of the synthetic ligand for the extracellular domain of each receptor subunit is at least 1 x 10⁻⁷ M, optionally at least 1 x 10⁻⁸ M, optionally at least 1 x 10⁻⁹ M, optionally at least 1 x 10⁻¹⁰ M.

8. The synthetic ligand of any one of the preceding claims wherein the first and second binding domains are joined by a polypeptide linker of 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids in length.

9. The synthetic ligand of claim 1, wherein
(a) the first binding domain specifically binds to the extracellular domain of IL-4Rα;
(b) the second binding domain specifically binds to the extracellular domain of IFNAR2 or IL-2Rβ;
(c) binding of the synthetic ligand to the first and the second receptor subunits results in multimerization of the receptor subunits and activation of JAK/STAT intracellular signaling.

10. The synthetic ligand of any one of claims 1 to 7 wherein the first and the second binding domains are joined by a homo-bifunctional linker or a hetero-bifunctional linker.

11. The synthetic ligand of claim 10 wherein the linker comprises a succinimidyl group which reacts with a primary amine, and a thiol-reactive maleimide which forms a covalent bond with the thiol of a cysteine residue.

12. A synthetic ligand of any one of claims 1 to 11 for use in a method of treatment.

13. A synthetic ligand of any one of claims 1 to 11 for use in a method of treating an inflammatory disease, an autoimmune disease, or cancer.

14. A pharmaceutical formulation comprising a synthetic ligand of any one of claims 1 to 11.

15. An in vitro method for selective activation of a non-native combination of cytokine receptor subunits in a cell, the method comprising contacting the cell with a synthetic ligand according to any one of claims 1 to 11, thereby causing multimerization of the cytokine receptor subunits and activation of signalling.

16. The in vitro method of claim 15, wherein the cell is an immune effector cell.

## Patentansprüche

1. Synthetischer Ligand, umfassend:
zumindest eine erste und eine zweite Bindungsdomäne, wobei jede Bindungsdomäne des synthetischen Liganden spezifisch an die extrazelluläre Domäne einer Zytokinrezeptor-Polypeptiduntereinheit, die auf der Oberfläche einer Zelle exprimiert wird, bindet, wobei:
die erste Bindungsdomäne spezifisch an die extrazelluläre Domäne einer ersten Zytokinrezeptor-Untereinheit bindet, wobei die erste Zytokinrezeptor-Untereinheit eine intrazelluläre Domäne aufweist, die zumindest eine JAK/STAT-Bindungssequenz umfasst; und
die zweite Bindungsdomäne spezifisch an die extrazelluläre Domäne einer zweiten Zytokinrezeptor-Untereinheit bindet, wobei die zweite Zytokinrezeptor-Untereinheit eine intrazelluläre Domäne aufweist, die zumindest eine JAK/STAT-Bindungssequenz umfasst,
wobei die erste und die zweite Zytokinrezeptor-Untereinheit nicht natürlich als Reaktion auf das Kontaktieren der Zelle mit einem natürlich vorkommenden Zytokinliganden multimerisieren;
wobei das Kontaktieren der Zelle, die die erste und die zweite Zytokinrezeptor-Untereinheit exprimiert, mit dem synthetischen Liganden zur Multimerisierung der Zytokinrezeptor-Untereinheiten und zur Aktivierung eines JAK/STAT-vermittelten Signals in der Zelle führt; und
wobei jede Bindungsdomäne ein Antikörper-abgeleitetes Bindungsprotein ist.

2. Synthetischer Ligand nach Anspruch 1, wobei die Zytokinrezeptoren aus Interleukin-3-Rezeptor alpha (IL-3Rα), beta-Interleukin-3-Rezeptor (βIL-3R), Granulozyten-Makrophagen-Kolonie-stimulierender-Faktor-Rezeptor (GM-CSFRα), Interleukin-5-Rezeptor alpha (IL-5Rα), ziliärem neurotrophem Rezeptor alpha (CNTFRα), Zytokinrezeptor-ähnlichem Faktor-1 (CRLF-1), Leukämie-Inhibitionsfaktor-Rezeptor alpha (LIFRα), Glykoprotein 130 (gp130), Interleukin-6-Rezeptor alpha (IL-6Rα), Interleukin-11-Rezeptor alpha (IL-11Rα), Oncostatin-M-Rezeptor-beta-Untereinheit (OSMRβ), Interleukin-2-Rezeptor alpha (IL-2Rα), Interleukin-2-Rezeptor beta (IL-2Rβ), Interleukin-2-Rezeptor gamma (IL-2Rγ), Interleukin-4-Rezeptor alpha (IL-4Rα), Interleukin-7-Rezeptor alpha (IL-7Rα), Interleukin-9-Rezeptor alpha (IL-9Rα), Interleukin-13-Rezeptor alpha (IL-13Rα), Interleukin-15-Rezeptor alpha (IL-15Rα), Interleukin-21-Rezeptor alpha (IL-21Rα), Interferon-alpha-Rezeptor 2 (IFNAR2), Interleukin-23-Rezeptor (IL-23R), Erythropoietin-Rezeptor (EpoR), Interleukin-12-Rezeptor beta (IL-12Rβ), Interferon-alpha/beta-Rezeptor-alpha-Kette (IFNAR1), Interferon-alpha/beta-Rezeptor-beta-Kette (IFNAR2), Granulozyten-Kolonie-stimulierender-Faktor-Rezeptor (G-CSFR), Differenzierungscluster w210 (CDw210), Interleukin-10-Rezeptor beta (IL10Rβ), Differenzierungscluster 212 (CD212), Differenzierungscluster w119 (CDw119) und myloproliferative-Leukämie-Proteinrezeptor (c-MPLR) ausgewählt sind.

3. Synthetischer Ligand nach Anspruch 1 oder Anspruch 2, wobei ein Antikörper-abgeleitetes Bindungsprotein ein scFv umfasst.

4. Synthetischer Ligand nach Anspruch 3, wobei jedes Antikörper-abgeleitete Bindungsprotein ein scFv umfasst.

5. Synthetischer Ligand nach Anspruch 1 oder Anspruch 2, wobei ein Antikörper-abgeleitetes Bindungsprotein eine VHH umfasst.

6. Synthetischer Ligand nach Anspruch 5, wobei jedes Antikörper-abgeleitete Bindungsprotein eine VHH umfasst.

7. Synthetischer Ligand nach einem der Ansprüche 1 bis 6, wobei die Bindungsaffinität jedes Antikörper-abgeleiteten Bindungsproteins des synthetischen Liganden für die extrazelluläre Domäne jeder Rezeptoruntereinheit zumindest 1 x 10⁻⁷ M, gegebenenfalls zumindest 1 x 10⁻⁸ M, gegebenenfalls zumindest 1 x 10⁻⁹ M, gegebenenfalls zumindest 1 x 10⁻¹⁰ M, beträgt.

8. Synthetischer Ligand nach einem der vorangegangenen Ansprüche, wobei die erste und die zweite Bindungsdomäne durch einen Polypeptidlinker mit einer Länge von 1, 2, 3, 4, 5, 6, 7, 8, 9, 10, 11, 12, 13, 14, 15, 16, 17, 18, 19, 20, 21, 22, 23, 24, 25, 26, 27, 28, 29 oder 30 Aminosäuren verbunden sind.

9. Synthetischer Ligand nach Anspruch 1, wobei:
(a) die erste Bindungsdomäne spezifisch an die extrazelluläre Domäne von IL-4Rα bindet;
(b) die zweite Bindungsdomäne spezifisch an die extrazelluläre Domäne von INFAR2 oder IL-2Rβ bindet;
(c) die Bindung des synthetischen Liganden an die erste und die zweite Rezeptoruntereinheit zur Multimerisierung der Rezeptoruntereinheiten und zur Aktivierung der intrazellulären JAK/STAT-Signalgebung führt.

10. Synthetischer Ligand nach einem der Ansprüche 1 bis 7, wobei die erste und die zweite Bindungsdomäne durch einen homobifunktionellen Linker oder einen heterobifunktionellen Linker verbunden sind.

11. Synthetischer Ligand nach Anspruch 10, wobei der Linker eine Succinimidylgruppe umfasst, die mit einem primären Amin reagiert, und ein Thiol-reaktives Maleinimid umfasst, das eine kovalente Bindung mit dem Thiol eines Cysteinrests bildet.

12. Synthetischer Ligand nach einem der Ansprüche 1 bis 11 zur Verwendung in einem Verfahren zur Behandlung.

13. Synthetischer Ligand nach einem der Ansprüche 1 bis 11 zur Verwendung in einem Verfahren zur Behandlung einer Entzündungskrankheit, einer Autoimmunerkrankung oder einer Krebserkrankung.

14. Pharmazeutische Formulierung, umfassend einen synthetischen Liganden nach einem der Ansprüche 1 bis 11.

15. In-vitro-Verfahren zur selektiven Aktivierung einer nichtnativen Kombination von Zytokinrezeptor-Untereinheiten in einer Zelle, wobei das Verfahren das Kontaktieren der Zelle mit einem synthetischen Liganden nach einem der Ansprüche 1 bis 11 umfasst, wodurch die Multimerisierung der Zytokinrezeptor-Untereinheiten und die Aktivierung der Signalgebung bewirkt werden.

16. In-vitro-Verfahren nach Anspruch 15, wobei die Zelle eine Immuneffektorzelle ist.

## Revendications

1. Ligand synthétique, comprenant :
au moins un premier et un second domaine de liaison, dans lequel chaque domaine de liaison du ligand synthétique se lie spécifiquement au domaine extracellulaire d'une sous-unité polypeptidique de récepteur de cytokine exprimée sur la surface d'une cellule, dans lequel :
le premier domaine de liaison se lie spécifiquement au domaine extracellulaire d'une première sous-unité de récepteur de cytokine, dans lequel la première sous-unité de récepteur de cytokine présente un domaine intracellulaire comprenant au moins une séquence de liaison JAK/STAT ; et
le second domaine de liaison se lie spécifiquement au domaine extracellulaire d'une seconde sous-unité de récepteur de cytokine, dans lequel la seconde sous-unité de récepteur de cytokine présente un domaine intracellulaire comprenant au moins une séquence de liaison JAK/STAT,
dans lequel les première et seconde sous-unités de récepteur de cytokine ne multimérisent pas naturellement en réponse à une mise en contact de la cellule avec un ligand de cytokine d'origine naturelle ;
dans lequel la mise en contact de la cellule exprimant les première et seconde sous-unités de récepteur de cytokine avec le ligand synthétique entraîne une multimérisation des sous-unités de récepteur de cytokine et une activation d'un signal médié par JAK/STAT dans la cellule ; et
dans lequel chaque domaine de liaison est une protéine de liaison dérivée d'un anticorps.

2. Ligand synthétique selon la revendication 1, dans lequel les récepteurs de cytokine sont choisis parmi le récepteur alpha de l'interleukine 3 (IL-3Rα), le récepteur bêta de l'interleukine 3 (βIL-3R), le récepteur du facteur stimulant les colonies de macrophages granulocytaires (GM-CSFRα), le récepteur alpha de l'interleukine 5 (IL-5Rα), le récepteur alpha neurotrophique ciliaire (CNTFRα), le facteur 1 de type récepteur de cytokine (CRLF1), le récepteur alpha du facteur inhibiteur de la leucémie (LIFRα), la glycoprotéine 130 (gp130), le récepteur alpha de l'interleukine 6 (IL-6Rα), le récepteur alpha de l'interleukine 11 (IL-11Rα), la sous-unité bêta du récepteur de l'oncostatine M (OSMRβ), le récepteur alpha de l'interleukine 2 (IL-2Rα), le récepteur bêta de l'interleukine 2 (IL-2Rβ), le récepteur gamma de l'interleukine 2 (IL-2R*γ*), le récepteur alpha de l'interleukine 4 (IL-4Rα), le récepteur de l'interleukine 7 alpha (IL-7Rα), le récepteur alpha de l'interleukine 9 (IL-9Rα), le récepteur alpha de l'interleukine 13 (IL-13Rα), le récepteur alpha de l'interleukine 15 (IL-15Rα), le récepteur alpha de l'interleukine 21 (IL-21Rα), le récepteur alpha de l'interféron 2 (IFNAR2), le récepteur de l'interleukine 23 (IL-23R), le récepteur de l'érythropoïétine (EpoR), le récepteur bêta de l'interleukine 12 (IL-12Rβ), la chaîne alpha du récepteur de l'interféron alpha/bêta (IFNAR1), la chaîne bêta du récepteur de l'interféron alpha/bêta (IFNAR2), le récepteur du facteur de stimulation des colonies de granulocytes (G-CSFR), le groupe de différenciation w210 (CDw210), le récepteur de l'interleukine 10 bêta (IL10Rβ), le groupe de différenciation 212 (CD212), le groupe de différenciation w119 (CDw119) et le récepteur de la protéine de leucémie myéloproliférative (c-MPLR).

3. Ligand synthétique selon la revendication 1 ou la revendication 2, dans lequel une protéine de liaison dérivée d'un anticorps comprend un scFv.

4. Ligand synthétique selon la revendication 3, dans lequel chaque protéine de liaison dérivée d'un anticorps comprend un scFv.

5. Ligand synthétique selon la revendication 1 ou la revendication 2, dans lequel une protéine de liaison dérivée d'un anticorps comprend une VHH.

6. Ligand synthétique selon la revendication 5, dans lequel chaque protéine de liaison dérivée d'un anticorps comprend une VHH.

7. Ligand synthétique selon l'une quelconque des revendications 1 à 6, dans lequel l'affinité de liaison de chaque protéine de liaison dérivée d'un anticorps du ligand synthétique pour le domaine extracellulaire de chaque sous-unité de récepteur est d'au moins 1 x 10⁻⁷ M, facultativement d'au moins 1 x 10⁻⁸ M, facultativement d'au moins 1 x 10⁻⁹ M, facultativement d'au moins 1 x 10⁻¹⁰ M.

8. Ligand synthétique selon l'une quelconque des revendications précédentes, dans lequel les premier et second domaines de liaison sont joints par un lieur polypeptidique d'une longueur de 1, 2, 3, 4, 5, 6, 7, 8, 9, 10, 11, 12, 13, 14, 15, 16, 17, 18, 19, 20, 21, 22, 23, 24, 25, 26, 27, 28, 29 ou 30 acides aminés.

9. Ligand synthétique selon la revendication 1, dans lequel
(a) le premier domaine de liaison se lie spécifiquement au domaine extracellulaire d'IL-4Rα ;
(b) le second domaine de liaison se lie spécifiquement au domaine extracellulaire d'IFNAR2 ou d'IL-2Rβ ;
(c) la liaison du ligand synthétique aux première et seconde sous-unités de récepteur entraîne une multimérisation des sous-unités de récepteur et une activation de la signalisation intracellulaire JAK/STAT.

10. Ligand synthétique selon l'une quelconque des revendications 1 à 7, dans lequel les premier et second domaines de liaison sont joints par un lieur homo-bifonctionnel ou un lieur hétéro-bifonctionnel.

11. Ligand synthétique selon la revendication 10, dans lequel le lieur comprend un groupe succinimidyle qui réagit avec une amine primaire, et un maléimide réactif au thiol qui forme une liaison covalente avec le thiol d'un résidu de cystéine.

12. Ligand synthétique selon l'une quelconque des revendications 1 à 11, pour utilisation dans un procédé de traitement.

13. Ligand synthétique selon l'une quelconque des revendications 1 à 11 pour utilisation dans un procédé de traitement d'une maladie inflammatoire, d'une maladie autoimmune ou d'un cancer.

14. Formulation pharmaceutique comprenant un ligand synthétique selon l'une quelconque des revendications 1 à 11.

15. Procédé *in vitro* d'activation sélective d'une combinaison non native de sous-unités de récepteur de cytokine dans une cellule, le procédé comprenant une mise en contact de la cellule avec un ligand synthétique selon l'une quelconque des revendications 1 à 11, en provoquant ainsi une multimérisation des sous-unités de récepteur de cytokine et une activation de signalisation.

16. Procédé *in vitro* selon la revendication 15, dans lequel la cellule est une cellule effectrice immunitaire.
